# EUROPEAN PATENT SPECIFICATION

(11) **EP 2 352 520 B1**
(45) Date of publication and mention of the grant of the patent: **05.08.2015**
(21) Application number: 08877836.0
(22) Date of filing: 31.10.2008
(51) Int. Cl.: A61K 39/275, A61P 35/00, A61P 33/02, A61P 37/00, A61K 39/00

(54) **VACCINATION WITH POXVIRUS VECTORS VIA MECHANICAL EPIDERMAL DISRUPTION**
IMPFUNG MIT POXVIRUS-VEKTOREN ÜBER MECHANISCHES AUFBRECHEN DER EPIDERMIS
VACCINATION AVEC DES VECTEURS POXVIRAUX VIA RUPTURE ÉPIDERMIQUE MÉCANIQUE

(43) Date of publication of application: 10.08.2011
(73) Proprietor: TremRx, Inc., Boston, MA 02210 (US)
(72) Inventor: KUPPER, Thomas, S., Weston, MA 02493 (US); LIU, Luzheng, Lisa, Vernon Hills, IL 60061 (US)
(74) Representative: Potter Clarkson LLP
(86) International application number: PCT/US2008/012345
(87) International publication number: WO 2010/050913

(56) References cited:
- WO-A1-00/34494
- WO-A1-00/34494
- WO-A1-2004/058278
- WO-A2-01/95919
- WO-A2-01/95919
- WO-A2-2005/103303
- US-A- 6 045 802
- ATHALE S K ET AL: "Skin scarification with Modified Vaccinia Ankara virus provides superior and safer protective immunization strategy against poxvirus challenge", JOURNAL OF INVESTIGATIVE DERMATOLOGY, vol. 128, no. Suppl. 1, April 2008 (2008-04), page S192, XP008148025, & INTERNATIONAL INVESTIGATIVE DERMATOLOGY MEETING; KYOTO, JAPAN; MAY 14 17, 2008 ISSN: 0022-202X
- LUZHENG LIU ET AL: "Dynamic Programing of CD8+ T Cell Trafficking after Live Viral Immunization", IMMUNITY, vol. 25, no. 3, 1 September 2006 (2006-09-01), pages 511-520, XP55017969, ISSN: 1074-7613, DOI: 10.1016/j.immuni.2006.06.019
- TIAN, T. ET AL.: 'Overexpression of IL- alpha in Skin Differentially Modulates the Immune Response to Scarification with Vaccinia Virus.' J. INVEST. DERMATOL. vol. 129, no. 1, 10 July 2008, pages 70 - 78, XP008148223
- KANTOR, J. ET AL.: 'Immunogenicity and Safety of a Recombinant Vaccinia Virus Vaccine Expressing the Carcinoembryonic Antigen Gene in a Nonhuman Primate.' CANCER RES. vol. 52, no. 24, 15 December 1992, pages 6917 - 6925, XP002009982
- List of hits from Google search
- GARNETT CHARLIE T ET AL: "TRICOM vector based cancer vaccines", CURRENT PHARMACEUTICAL DESIGN, vol. 12, no. 3, 2006, pages 351-361, ISSN: 1381-6128
- ENNIS FRANCIS A ET AL: "Primary induction of human CD8+ cytotoxic T lymphocytes and interferon-gamma-producing T cells after smallpox vaccination", JOURNAL OF INFECTIOUS DISEASES, vol. 185, no. 11, 1 June 2002 (2002-06-01) , pages 1657-1659, ISSN: 0022-1899
- HODGE J W ET AL: "TRIAD OF COSTIMULATORY MOLECULES SYNERGIZE TO AMPLIFY T-CELL ACTIVATION", CANCER RESEARCH, AMERICAN ASSOCIATION FOR CANCER RESEARCH, US, vol. 59, 1 November 1999 (1999-11-01), pages 5800-5807, XP000882899, ISSN: 0008-5472
- PROSTVAC specifications
- IWASAKI A: "Local advantage: Skin DCs prime; skin memory T cells protect", NATURE IMMUNOLOGY 2009 NATURE PUBLISHING GROUP GBR, vol. 10, no. 5, 2009, pages 451-453, ISSN: 1529-2908
- MCCLAIN DAVID J ET AL: "Immunologic responses to vaccinia vaccines administered by different parenteral routes", JOURNAL OF INFECTIOUS DISEASES, vol. 175, no. 4, 1997, pages 756-763, ISSN: 0022-1899
- SUTTER GERD ET AL: "Nonreplicating vaccinia vector efficiently expresses recombinant genes", PROCEEDINGS OF THE NATIONAL ACADEMY OF SCIENCES OF THE UNITED STATES OF AMERICA, vol. 89, no. 22, 1992, pages 10847-10851, ISSN: 0027-8424
- LIU LUZHENG ET AL: "Epidermal injury and infection during poxvirus immunization is crucial for the generation of highly protective T cell-mediated immunity", NATURE MEDICINE, vol. 16, no. 2, February 2010 (2010-02), page 224, ISSN: 1078-8956
- FREY SHARON E ET AL: "Clinical and immunologic responses to multiple doses of IMVAMUNE (R) (Modified Vaccinia Ankara) followed by Dryvax (R) challenge", VACCINE, vol. 25, no. 51, December 2007 (2007-12), pages 8562-8573, ISSN: 0264-410X
- JENTARRA GARILYN M ET AL: "Vaccinia viruses with mutations in the E3L gene as potential replication-competent, attenuated vaccines: Scarification vaccination", VACCINE, vol. 26, no. 23, June 2008 (2008-06), pages 2860-2872, ISSN: 0264-410X
- Dr. Thomas Kupper's C.V.
- Dr. Thomas Kupper's memo "Melanoma Vaccine"
- GRANTED CLAIM 1 OF US 8691502 B2
- Granted claim 1 of JP 5 588 990

## Description

### FEDERALLY SPONSORED RESEARCH

This invention was made in part with government support under grant numbers U19 AI057330, and 5U54AI057159-05 from the National Institute of Allergy and Infectious Diseases (NIAID) of the National Institute of Health (NIH). The United States government has certain rights to this invention.

### BACKGROUND OF INVENTION

Vaccines have traditionally consisted of live attenuated pathogens, whole inactivated organisms or inactivated toxins. In many cases these approaches have been successful at inducing immune protection based on antibody mediated responses. However, certain pathogens, e.g., HIV, HCV, TB, malaria and cancer, require the induction of cell-mediated immunity (CMI). Non-live vaccines have generally proven ineffective in producing CMI. In addition, although live vaccines may induce CMI, some live attenuated vaccines may cause disease in immunosuppressed subjects.

Therefore, there is an unmet need for more effective vaccines and more effective means of delivering them to result in an enhanced therapeutic efficacy and protective immune response.

WO 2004/058278 relates to a recombinant poxvirus vector, such as a vaccinia virus vector, which comprises a nucleic acid sequence encoding IL-15, a cytokine which stimulates the proliferation and differentiation of T cells *in vitro* and can augment T cell mediated immune responses *in vivo.*

Hodge et al (1999) Cancer Research 59; 5800-5807 describes administration of a recombinant vaccinia virus expressing human carcinoembryonic antigen (CEA) with one, three or no co-stimulatory molecules to mice via tail scarification inducing a CEA-specific T cell response.

### SUMMARY OF INVENTION

The invention is defined in the accompanying claims.

Attenuated, replication-deficient vaccinia viruses, such as the modified vaccinia virus Ankara (MVA) strain, have been previously proposed as promising live viral vaccine vectors because of their impressive safety and immunogenicity profile and have been tested in both pre-clinical and clinical studies. However, viral vaccines, such as MVA, have been administered exclusively via injection routes, and until now, never via skin scarification. This may be due to the assumption that viral replication in the epidermis is required for the development of pox lesion and the subsequent strong protection against antigen challenge. Nevertheless, the inventors immunized mice with MVA via skin scarification and, to their surprise, MVA skin scarification induced characteristic pox lesions in a dose-dependent manner and generated dose-dependent cellular and humoral immune responses against vaccinia virus (VV) antigens. Without intending to be bound by any particular mechanism or theory, the inventors believe that viral replication is not required to elicit a strong immune response by immunization via skin scarification.

MVA skin scarification provided complete protection against mortality and illness in mice challenged with intranasal Western Reserve vaccinia virus (WR-VV) infection at a dose at which replicative VV immunization via the conventional injection routes failed to protect mice from the lethal challenge. At a comparable dose of MVA immunization, the conventional injection routes only elicited weakly detectable T cell and antibody responses, even after secondary viral challenge and offered poor protection against the WR-VV intranasal challenge, whereas strong immune protection was afforded by skin scarification with either MVA or VV. Thus, epidermal immunization with live viral vaccines, such as replication-deficient poxviruses (e.g., MVA), using mechanical disruption of the skin, generates a stronger immune response and stronger protection of the immunized host at a much lower dose compared to the injection routes currently used in the clinic, which require high doses and multiple injection regimes.

Disclosed herein is a novel method for immunizing a subject against infections (or infectious diseases) or cancer, using a modified replication deficient poxvirus vector containing antigens applied to mechanically disrupted epidermis of the subject. Variant vaccinia viruses that are modified so as to be replication deficient or less infectious which have been genetically modified to contain cDNA's encoding for antigen(s) may be used.

A method for stimulating an immune response is also disclosed. The method comprises administering to a subject a live, modified, non-replicating or replication-impaired poxvirus comprising an antigen in an amount sufficient to stimulate the immune response, wherein the poxvirus is administered to a mechanically disrupted epidermis of the subject. The immune response may be a humoral response and/or a cellular response. The cellular response can be elicited by CD4+ and/or CD8+ T cells and/or B cells.

One aspect of the invention provides a live, modified, non-replicating poxvirus comprising a nucleic acid encoding a peptide or polypeptide antigen that is foreign to poxvirus, wherein the poxvirus infects, but is non-replicating in human cells; for use in a method of eliciting a T cell memory immune response to the foreign antigen, by administering the poxvirus to a mechanically disrupted epidermis of a subject so that the poxvirus expressing the foreign antigen infects the disrupted epidermis and elicits a T cell memory immune response.

The poxvirus may be orthopox, suipox, avipox, capripox, leporipox, parapoxvirus, molluscpoxvirus, or yatapoxvirus. In some embodiments, the poxvirus is TRICOM^{™}.

In some embodiments, the orthopox virus is a vaccinia virus. The vaccinia virus may be modified vaccinia virus Ankara (MVA), WR strain, NYCBH strain, Wyeth strain, ACAM2000, Lister strain, LC16m8, Elstree-BNm, Copenhagen strain, or Tiantan strain.

The epidermis may be mechanically disrupted by a scarification needle, a hypodermic needle, or an abrader device. The epidermis may be mechanically disrupted essentially at the same time as the administration of the poxvirus or before the administration of the poxvirus.

In some embodiments, the subject has or is at risk of developing cancer. The cancer may be skin, breast, prostate, brain, lung, ovary, liver, pancreas, stomach, kidney, bladder, and thyroid, or colorectal cancer. In some embodiments, the cancer is prostate adenocarcinoma, prostatic intraepithelial neoplasia, squamous cell lung carcinoma, lung adenocarcinoma, small cell lung carcinoma, ovary cancer of epithelial origin, colorectal adenocarcinoma and leiomyosarcoma, stomach adenocarcinoma and leiomyosarcoma, hepatocellular carcinoma, cholangiocarcinoma, ductal adenocarcinomas of pancreas, endocrine pancreatic tumors, renal cell carcinoma, transitional cell carcinoma of kidney and bladder, bladder squamous cell carcinoma, papillary thyroid cancer, follicular thyroid cancer, astrocytoma, or glioblastoma multiforme. The skin cancer may be melanoma, cutaneous squamous cell carcinoma or basal call carcinoma.

In some embodiments, the subject has or is at risk of developing an infection. The infection may be a viral, bacterial, fungal, or protozoal infection. Examples of viral infections include, but are not limited to, HIV, influenza, dengue, Hepatitis A virus, Hepatitis B virus, Hepatitis C virus, human papilloma virus, Ebola, Marburg, Rabies, Hanta virus infection, West Nile virus, SARS-like Coronaviruses, Herpes simplex virus, Varicella-zoster virus, Epstein-Barr virus, Human herpesvirus 8, Alpha viruses, and St. Louis encephalitis.

The bacterial infection may be Mycobacterium tuberculosis, Salmonella typhi, Bacillus anthracis, Yersinia perstis, Francisella tularensis, Legionella, Chlamydia, Rickettsia typhi, or Treponema pallidum.

Examples of fungal infections include, but are not limited to, Coccidioides immitis, Blastomyces dermatitidis, Cryptococcus neoformans, Candida albicans, and Aspergillus species.

Examples of protozoal infections include, but are not limited to, Malaria (Plasmodium falciparum, Plasmodium vivax, Plasmodium ovale, Plasmodium malariae), Leishmania species, Trypanosome species (African and American), cryptosporidiums, isospora species, Naegleria fowleri, Acanthamoeba species, Balamuthia mandrillaris, Toxoplasma gondii, and Pneumocystis carinii.

In some embodiments, the antigen is a tumor-associated antigen (TAA), a tumor-specific antigen (TSA), or a tissue-specific antigen. The TAA, TSA or tissue specific antigen may be mucin 1 (MUC1), mucin 2 (MUC2), BAGE-1, GAGE-1~8; GnTV, HERV-K-MEL, KK-LC-1, KM-HN-1, LAGE-1, MAGE-A1~A4, MAGE-A6, MAGE-A9, MAGE-A10, MAGE-A12,MAGE- C3, NA88, NY-ESO-1 / LAGE-2, SAGE, Sp17, SSX-2, SSX-4, TAG-1, TAG-2, TRAG-3, TRP2-INT2, XAGE-1b, carcinoembriogenic antigen (CEA), CA-125, gp100 / Pmel17, Kallikrein 4, mammaglobin-A, Melan-A / MART-1, NY-BR-1, OA-1, prostate specific antigen (PSA), RAB38 /NY-MEL-1, TRP-1 / gp75, TRP-2, tyrosinase, adipophilin, AIM-2, ALDH1A1, BCLX (L), BING-4, CPSF, cyclin D1, DKK1, ENAH (hMena), Ep-CAM, EphA3, EZH2, FGF5, G250 / MN / CAIX, HER-2 / neu, IL13Ralpha2, Intestinal carboxyl esterase, alpha-fetoprotein (AFP), M-CSF, MCSP, mdm-2, MMP-2, MUC1, PBF, PRAME, PSMA, RAGE-1, RGS5, RNF43, RU2AS, secernin 1, SOX10, STEAP1, survivin, Telomerase, VEGF, WT1, alpha-actinin-4, ARTC1, BCR-ABL fusion protein, B-RAF, CASP-5, CASP-8, beta-catenin, Cdc27, CDK4, CDKN2A, COA-1, dek-can fusion protein, EFTUD2, Elongation factor 2, ETV6-AML1 fusion protein, FLT3-ITD, FN1, GPNMB, LDLR-fucosyltransferaseAS fusion protein, HLA-A2, HLA-A11, hsp70-2, KIAAO205, MART2, ME1, MUM-1, MUM-2, MUM-3, neo-PAP, Myosin class I, NFYC, OGT, OS-9, p53, pml-RARalpha fusion protein, PRDX5, PTPRK, K-ras, N-ras, RBAF600, SIRT2, SNRPD1, SYT-SSX1 or -SSX2 fusion protein, TGF-betaRII, or Triosephosphate Isomerase.

In some embodiments, the antigen is a viral, bacterial, fungal or protozoal antigen. The antigen may be HIV Gag, protease, reverse transcriptase, full-length envelope protein, Vpu, Tat and Rev; influenza hemagglutinin, nucleoprotein, matrix protein 1 (M1), non-structural protein 1 (NS-1); a dengue virus cross-protective antigen shared by all major serotypes of viruses (e.g. Non-structural protein 1 or NS1, envelop domain III or EDIII), Hepatitis A virus capsid protein 1 (VP-1), Hepatitis B virus surface antigen (HBsAg) and core antigen (HBcAg), Hepatitis C core antigen, E1, E2, p7, NS2, NS4 and NS4 proteins, HPV E1, E2, L1, L2, Ebola and Marburg virus glycoprotein, nucleoprotein and matrix protein, Rabies glycoprotein, Hanta virus nucleoprotein, envelope glycoprotein and G1 protein, West Nile virus premembrane (prM) and envelop (E) glycoproteins, SARS-like Coronaviruses Orf3, Spike protein, Nucleocapsid and Membrane protein, Herpes simplex virus glycoprotein B and D; Varicella-zoster virus envelope glycoprotein E and B (gE, gB), immediate early protein 63 (IE63), Epstein-Barr virus gp350, gp110, nuclear antigen 1 (EBNA-1), EBNA 2 and EBNA-3C, Human herpesvirus 8 complement control protein (KCP), glycoprotein B, ORF6, ORF61, and ORF65), M. tuberculosis antigen 85A, 85B, MPT51, PPE44, mycobacterial 65-kDa heat shock protein (DNA-hsp65), 6-kDa early secretary antigenic target (ESAT-6), Salmonella SpaO, H1a, outer membrane proteins (OMPs), P. aeruginosa OMPs, PcrV, OprF, OprI, PilA and mutated ToxA, B. anthracis protective antigen (PA), Y. pestis low calcium response protein V (LcrV), F1 and F1-V fusion protein, Legionella peptidoglycan-associated lipoprotein (PAL), mip, flagella, OmpS, hsp60, major secretory protein (MSP), Chlamydia protease-like activity factor (CPAF), major outer membrane protein (MOMP), T. pallidum outer membrane lipoproteins, Coccidioides Ag2/Pra106, Prp2, phospholipase (P1b), alpha-mannosidase (Amn1), aspartyl protease, Gel1, Blastomyces dermatitidis surface adhesin WI-1, Cryptococcus neoformans GXM, Peptide mimotopes or mannoproteins of Cryptococcus neoformans GXM, Candida albicans hsp90-CA, 65-kDa mannoprotein (MP65), Secretory aspartyl proteinase (Sap), Alslp-N, Als3p-N, Aspergillus Asp f 16 , Asp f 2, Der p 1, and Fel d 1, rodlet A, PEP2, Aspergillus HSP90, 90-kDa catalase, Plasmodium apical membrane antigen 1 (AMA1), 25-kDa sexual-stage protein (Pfs25), erythrocyte membrane protein 1 (PfEMP1) circumsporozoite protein (CSP), Merozoite Surface Protein-1 (MSP1), Leishmania. cysteine proteinase type III (CPC), ribosomal proteins (LRP), A2 antigen, nucleosomal histones, HSP20, G46/M-2/PSA-2 promastigote surface protein, *L infantum* LACK antigen, GP63, LmSTI1, TSA, P4, NH36, papLe22, Trypanosome beta-tubulin (STIB 806), microtubule-associate protein (MAP p15), cysteine proteases (CPs), Cryptosporidiums surface proteins gp15 and gp40, Cp23 antigen, p23, Toxoplasma gondii surface antigen 1 (TgSAG1), protease inhibitor-1 (TgPI-1), surface-associated proteins MIC2, MIC3, ROP2, GRA1-GRA7, Pneumocystis carinii major surface glycoprotein (MSG), p55 antigen, Schistosomiasis mansoni Sm14, 21.7 and SmFim antigen, Tegument Protein Sm29, 26kDa GST, Schistosomajaponicum, SjCTPI, SjC23, Sj22.7, or SjGST-32.

In some embodiments, the methods for stimulating an immune response described herein further comprise administering a co-stimulatory molecule, a growth factor, an adjuvant and/or a cytokine. Examples of co-stimulatory molecules, growth factors, adjuvants or cytokines include, but are not limited to, IL-1, IL-2, IL-7, IL-12, IL-15, IL-18, IL-23, IL-27, B7-1, B7-2, LFA-3, B7-H3, CD40, CD40L, ICOS-ligand, OX-40L, 4-1BBL, GM-CSF, SCF, FGF, Flt3-ligand, CCR4, QS-7, QS-17, QS-21, CpG oligonucleotides, ST-246, AS-04, LT R192G mutant, Montanide ISA 720, heat shock proteins, synthetic mycobacterial cordfactor (CAF01), Lipid A mimetics, Salmonella enterica serovar, Typhimurium flagellin (FliC), Montanide 720, Levamisole (LMS), Imiquimod, Diphtheria Toxin, IMP321, AS02A, AS01B, AS15-SB, Alhydrogel, Montanide ISA, Aluminum hydroxide, MF59, ISCOMATRIX, MLPA, MPL and other TLR-4 ligands, MDP, other TLR-2 ligands, AS02A, AS01B, Heat Liable Toxin LTK63 and LT-R192G.

The co-stimulatory molecule may be co-expressed with the antigen by the poxvirus. The co-expressed co-stimulatory molecule may be IL-1, IL-2, IL-7, IL-12, IL-15, IL-18, IL-23, IL-27, B7-2, B7-H3, CD40, CD40L, ICOS-ligand, OX-40L, 4-1BBL, GM-CSF, SCF, FGF, Flt3-ligand, or CCR4.

The co-stimulatory molecule, growth factor, adjuvant and/or cytokine may be administered essentially at the same time as the antigen, or may be administered before or after administration of the antigen. The co-stimulatory molecule, growth factor, adjuvant and/or cytokine may be administered at essentially the same site as the antigen, or is administered at a different site as the antigen.

The methods described herein may further comprise a second administration of the antigen at a time after the first administration of the antigen.

The non-replicating or replication-impaired poxvirus comprises a viral vector comprising a nucleic acid encoding the antigen. The nucleic acid encoding the antigen may be operatively linked to a promoter. The promoter may be a constitutively active promoter or an inducible promoter. The promoter may further comprise an enhancer or another transcriptional regulatory element (TRE).

In some embodiments, the subject has not been challenged with the antigen prior to administering the poxvirus comprising the antigen and the subject is at risk of being challenged with the antigen. In these embodiments, stimulating the immune response confers protection of the subject against a disease caused by an agent presenting the antigen.

In some embodiments, the subject has been challenged with the antigen prior to administering the poxvirus comprising the antigen. In these embodiments, stimulating the immune response treats a disease in the subject caused by an agent presenting the antigen.

In some embodiments, stimulating the immune response protects from or treats the disease. The disease may be a cancer or an infection. The cancer may be melanoma, cutaneous squamous cell carcinoma, basal cell carcinoma, breast cancer, prostate adenocarcinoma, prostatic intraepithelial neoplasia, squamous cell lung carcinoma, lung adenocarcinoma, small cell lung carcinoma, ovary cancer of epithelial origin, colorectal adenocarcinoma and leiomyosarcoma, stomach adenocarcinoma and leiomyosarcoma, hepatocellular carcinoma, cholangiocarcinoma, ductal adenocarcinomas of pancreas, endocrine pancreatic tumors, renal cell carcinoma, transitional cell carcinoma of kidney and bladder, bladder squamous cell carcinoma, papillary thyroid cancer, follicular thyroid cancer, astrocytoma, or glioblastoma multiforme.

The infection may be a viral, bacterial, fungal, or protozoal infection. The infection may be HIV, influenza, dengue, Hepatitis A virus, Hepatitis B virus, Hepatitis C virus, human papilloma virus, Ebola, Marburg, Rabies, Hanta virus infection, West Nile virus, SARS-like Coronaviruses, Herpes simplex virus, Varicella-zoster virus, Epstein-Barr virus, Human herpesvirus 8, Alpha viruses, St. Louis encephalitis, Mycobacterium tuberculosis, Salmonella typhi, Bacillus anthracis, Yersinia perstis, Francisella tularensis, Legionella, Chlamydia, Rickettsia typhi, Treponema pallidum, Coccidioides immitis, Blastomyces dermatitidis, Cryptococcus neoformans, Candida albicans, Aspergillus species, Malaria (Plasmodium falciparum, Plasmodium vivax, Plasmodium ovale, Plasmodium malariae), Leishmania species, Trypanosome species (African and American), cryptosporidiums, isospora species, Naegleria fowleri, Acanthamoeba species, Balamuthia mandrillaris, Toxoplasma gondii, or Pneumocystis carinii.

According to another aspect of the invention, kits are provided. The kit comprises a device for disrupting a subject's epidermis and a live, modified, non-replicating poxvirus, as defined in the claims. The device may be a scarification needle, a hypodermic needle or an abrader.

The poxvirus may be attached to the device or admixed in a solution. The solution may further comprise an agent that enhances delivery of the poxvirus to the subject via the subject's epidermis. The solution may further comprise an agent that enhances an immune response in a subject.

The kits described herein may further comprise instructions to use the kits.

Each of the limitations of the invention can encompass various embodiments of the invention. It is, therefore, anticipated that each of the limitations of the invention involving any one element or combinations of elements can be included in each aspect of the invention. This invention is not limited in its application to the details of construction and the arrangement of components set forth in the following description or illustrated in the drawings. The invention is capable of other embodiments and of being practiced or of being carried out in various ways. Also, the phraseology and terminology used herein is for the purpose of description and should not be regarded as limiting. The use of "including," "comprising," or "having," "containing", "involving", and variations thereof herein, is meant to encompass the items listed thereafter and equivalents thereof as well as additional items.

### BRIEF DESCRIPTION OF DRAWINGS

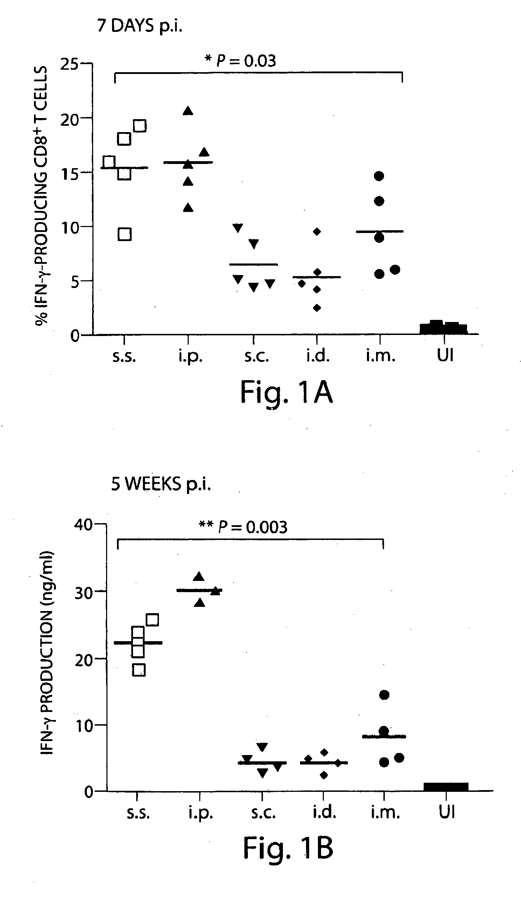

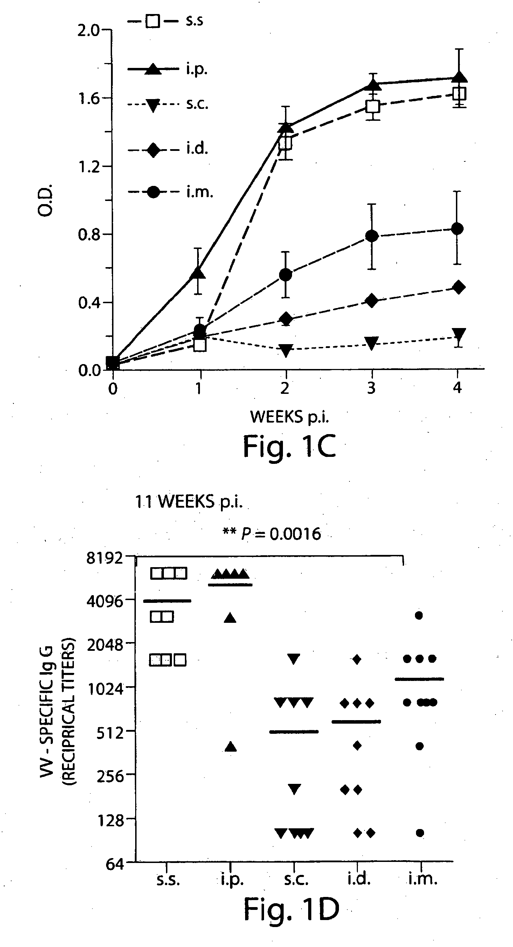

FIG. 1. Vaccinia virus inoculation via skin scarification is superiorly immunogenic compared to other immunization routes. C57BL/6 (B6) mice were immunized by VV at 2 x 10⁶ pfu dose by the indicated routes. s.s.: skin scarification; i.p.: intraperitoneal injections; s.c.: subcutaneous injections; i.d.: interdermal injection; i.m.: intramuscular injection. (a) Primary T cell response: splenocytes harvested on day 7 following immunization (p.i.) were restimulated with VV-infected target cells (splenocytes from naive mice) for 6 hours in the presence of brefelding A to measure the frequency of IFN-γ producing CD8 T cells by intracellular cytokine staining. Ul: unimmunized mice. (b)Memory VV-specific T cell activity was assessed at 5 week p.i. Splenocytes from immunized mice were restimulated with VV-infected target cells for 48 h and supernatant were harvested. IFN-g production in supernatant was measured by ELISA. (c-d). Serum VV-specific IgG level was determined at the indicated time points p.i. by ELISA.

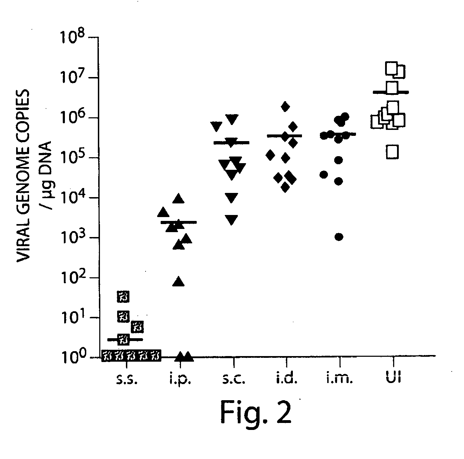

FIG. 2. VV skin scarification led to superior protection against secondary cutaneous virus challenge compared to other routes of immunization. B6 mice were immunized with VV via various routes as indicated. Eight weeks following immunization, the immune mice were challenged with secondary cutaneous VV infection. Six days after challenge, viral load at the challenged site was measured by real-time PCR. Unimmunized mice were included as control.

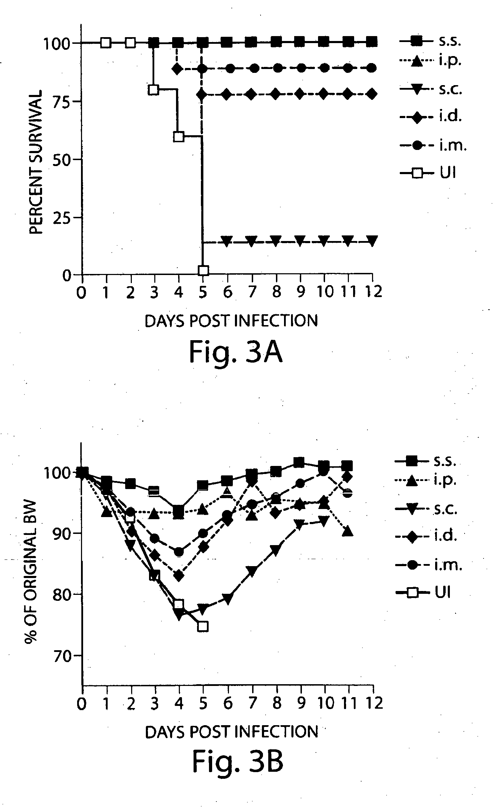

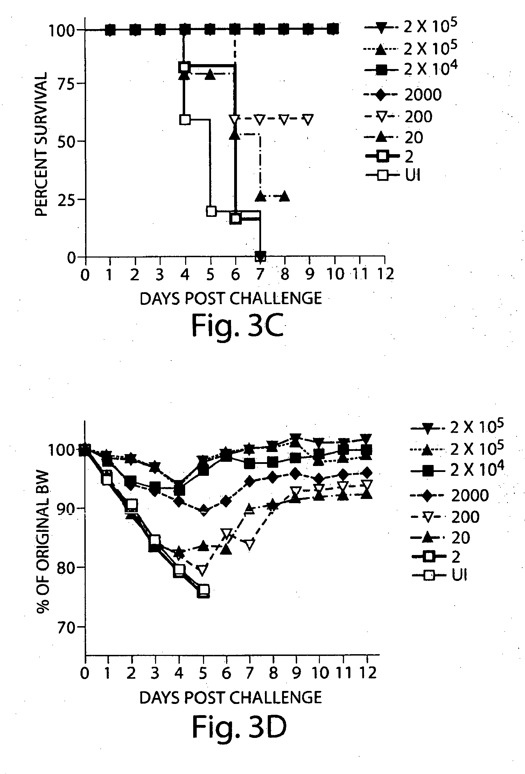

FIG. 3. Skin scarification provided superior protection against secondary intranasal viral challenge. (a-b) B6 mice were immunized by VV via various routes at 2 c 10⁶ pfu dose. Immune mice were lethally challenged with intranasal infection of WR-VV at 6 weeks p.i.. The survival (a) and change of bodyweight (BW) (b) were monitored daily after challenge, (c-d) B6 mice were immunized by. VV via skin scarification at the indicated doses, and lethally challenged with intranasal WR-VV infection at 6 weeks p.i. The survival (c) and change of BW (d) were monitored daily after challenge. Unimmunized mice were included as controls.

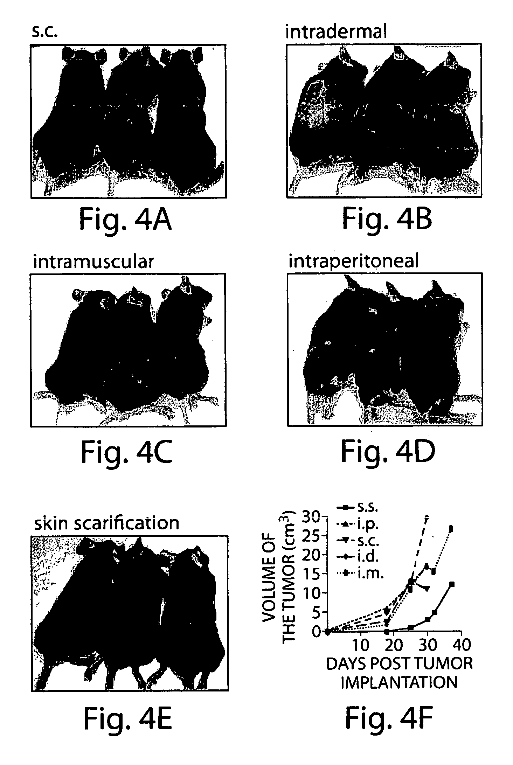

FIG. 4. Immunization via VV skin scarification provides superior protection against intradermal melanoma challenge. B6 mice were immunized with rVV-ova via the indicated routes. The immune mice were challenged 4 weeks later with intradermal injection of B16-ova melanoma cells. (a-e) Photographs of tumor-challenged mice were taken on day 18 following tumor cell implantation. (f) Tumor growth was monitored in the challenged mice for up to 40 days.

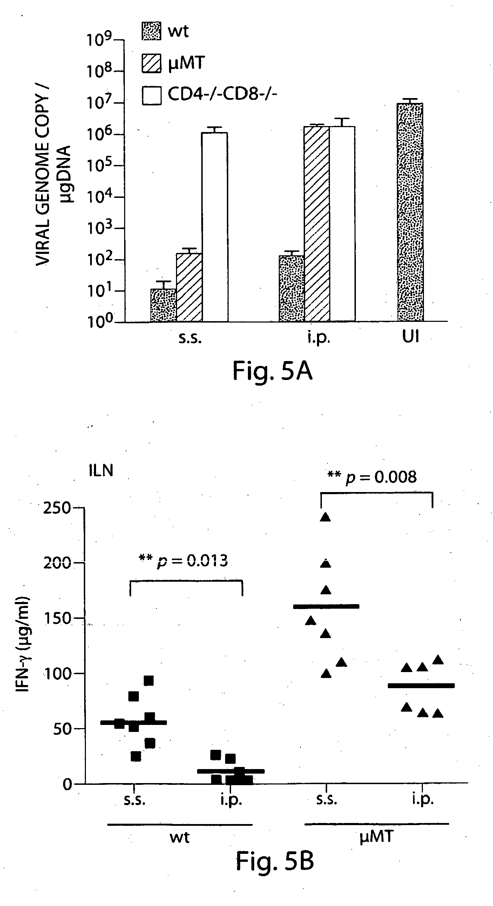

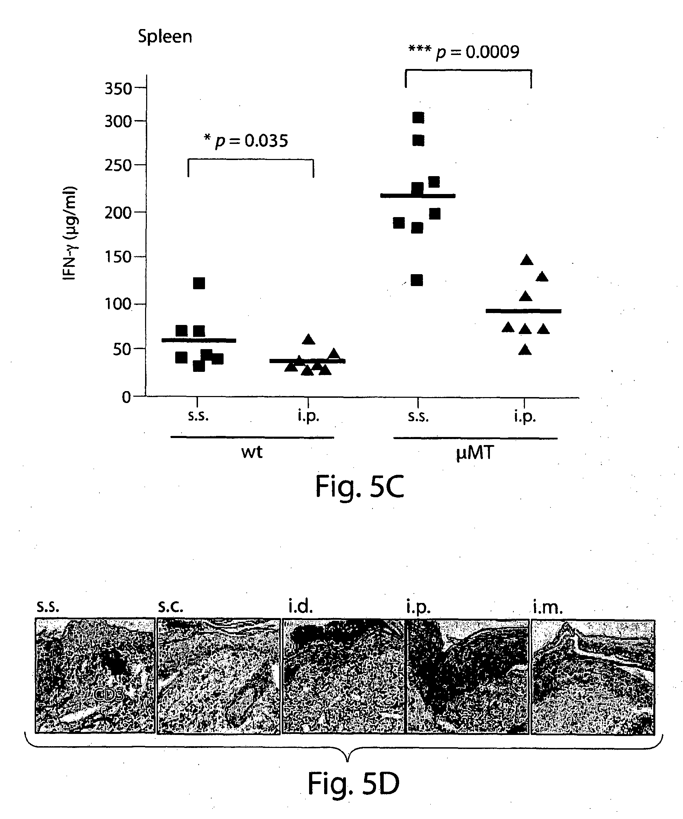

FIG. 5. T-cell mediated immune response was required for the superior protection against secondary cutaneous challenge following VV skin scarification. WT or B cell deficient mMT mice were immunized with VV via skin scarification or i.p. injection. Mice were then challenged by secondary VV cutaneous infection at 6 weeks p.i.. (a) Viral load at the challenged sites were determined on day 6 after challenge. In some groups of wt mice, both CD4⁺ and CD8⁺ T cells were depleted before the challenge. (b-c) Secondary T cell response in challenged wt and mMt immune mice was assessed in skin-draining inguinal lymph nodes (b) and spleen (c) on day 6 following challenge. (d) Skin samples were harvested from the challenged site at 4 days after challenge. Skin-infiltrating CD3⁺ T cells were identified by immunohistochemistry.

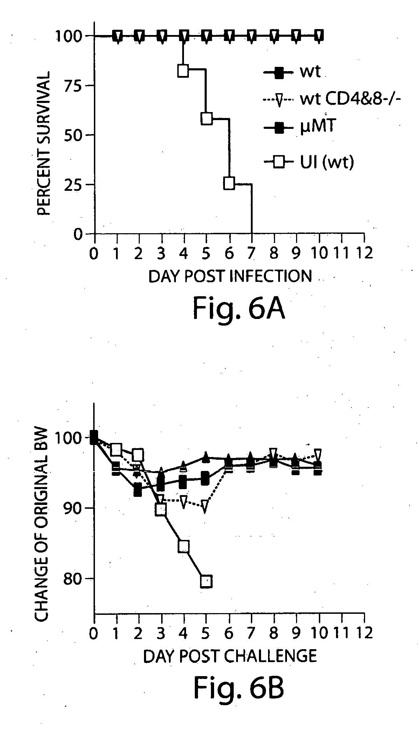

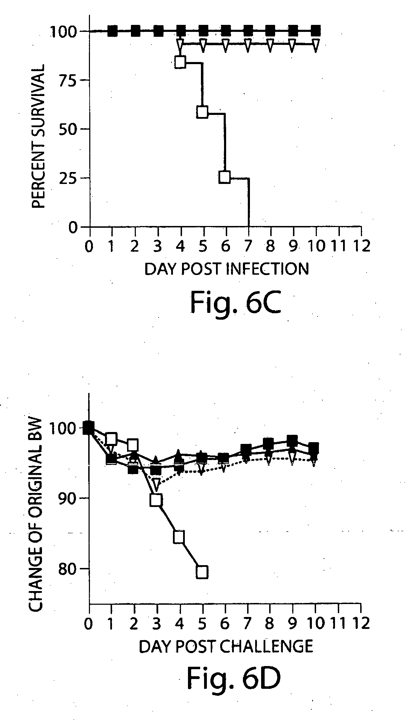

FIG. 6. T cells but no Ab were important for the prevention of illness, although neither T cells nor B cells were required for survival after lethal intranasal WR-VV challenge. Wild type (wt) or B cell deficient µMT mice were immunized with VV via skin scarification (a-b) or i.p. injection (c-d). Mice were then challenged by lethal WR-VV intranasal infection at 6 weeks p.i. In some groups of wt mice, both CD4⁺ and CD8⁺ T cells were depleted before and during challenge by large doses of anti-CD4 and anti-CD8 mAb treatment. Mice survival (a, c) and change of BW (b, d) were monitored daily after the challenge.

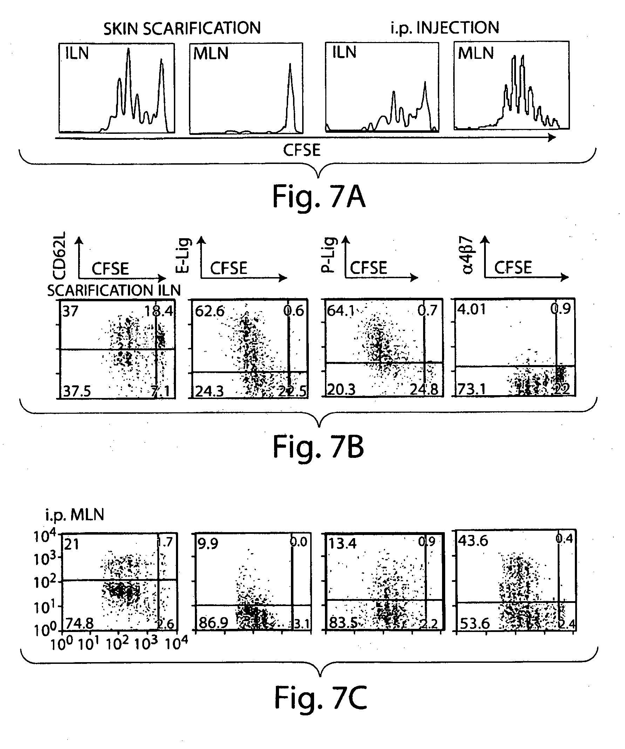

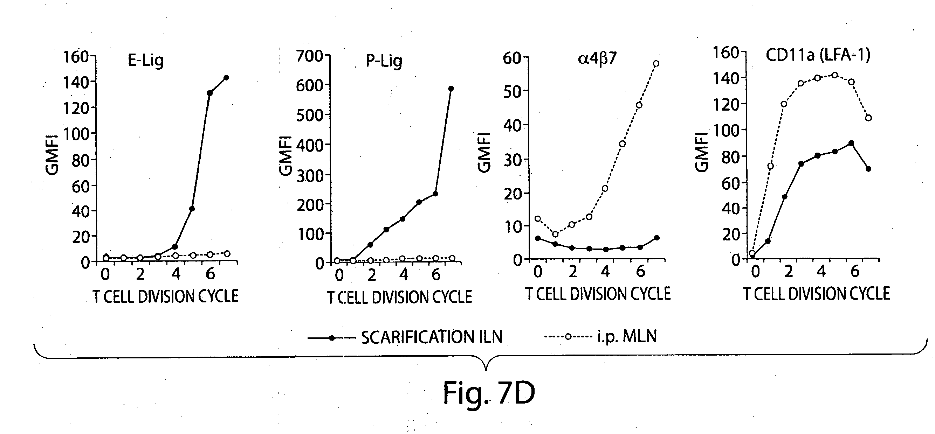

FIG. 7. The LN of T cell activation imprints differential expression of skin or gut-homing molecules on CD8⁺ T cells as early as 60 h after infection. CFSE-labeled Thy1.1⁺ OT-1 cells were adoptively transferred into Thy1.2⁺ B6 mice. Recipient mice were then infected with rVV-ova by either skin scarification or i.p. injection. (a) 60 hours after the infection, proliferation of OT-1 cells in ILN, and MLN were shown by Histograms gated on Thy1.1⁺ donor cells. (b-c) ILN of skin scarified mice (b) and MLN of i.p.-infected mice (c) were analyzed at 60 h after rVV-ova infection for OT-1 tissue-homing phenotype. Dot plots were gated on Thy1.1+ cells. The numbers in quadrant indicate the percentages in Thy1.1⁺ population. (d) The geometric mean fluorescence intensities (GMF1) of the indicated markers on OT-1 cells were plotted with the cell division cycles.

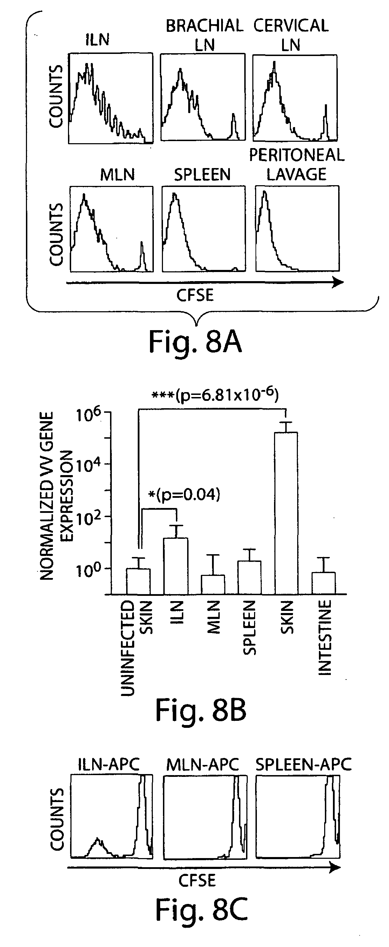

FIG. 8. Five days after VV skin scarification, T cells activated in skin-draining ILN migrated throughout secondary lymphoid tissues without virus or viral antigen dissemination. (a) Lymphocytes were prepared from the indicated tissues 5 days after rVV-ova skin scarification. The proliferation of OT-1 cells was analyzed by flow cytometry. (b) RNA samples were prepared from the indicated tissues at day 5 after VV scarification. VV infection was measured by real-time RT-PCT. Data represent the averages ± s.d. of 4 independent experiments with three mice per group. (c) Antigen presenting cells were purified with anti-MHC Class II magnetic beads from ILN, MLN and spleen of B6 mice 4 days following skin scarification with rVV-ova. The cells were co-cultured with CFSE-labeled Thy1.1⁺ OT-1 cells for 60 h. OT-1 cell activation and proliferation was monitored by flow cytometer. The histograms were gated on Thy1.1⁺ population.

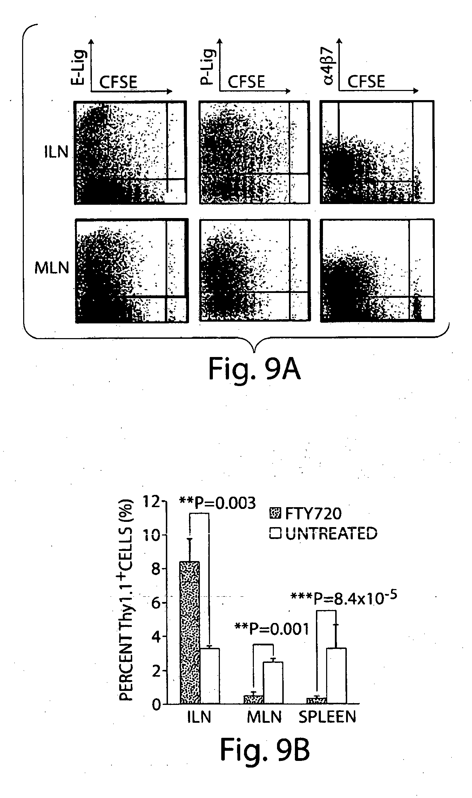

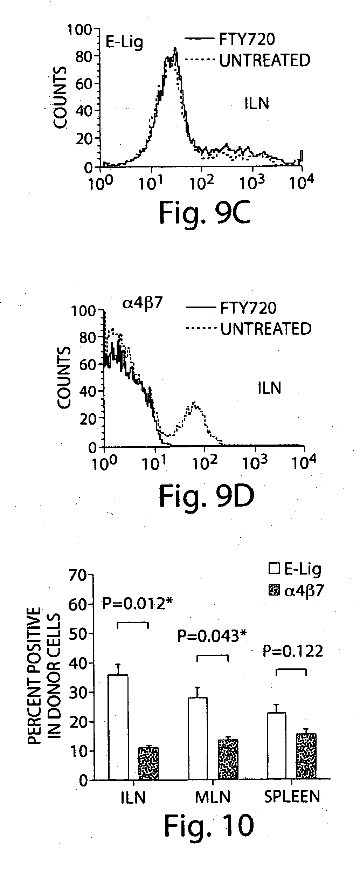

FIG. 9. At day 5 after skin scarification with rVV-ova, the gut-homing molecule α4β7 was upregulated on disseminated OT-1 CD8+ cells by secondary homing imprinting. (a) ILN and MLN were harvested at day 5 after scarification with rVV-ova. The expression of the indicated homing markers on Thy1.1⁺ OT-1 cells was determined by flow cytometry. Dot plots were gated on Thy1.1⁺ cells. (b-d) B6 mice that had received Thy1.1⁺ OT-1 cells were given daily injections of FTY720 starting 24 h before scarification with rVV-ova. On day 5 after infection, lymphocytes from the indicated tissues were harvested and analyzed by flow cytometry. (b) The percentages of Thy1.1⁺ cells in total lymphocytes. (c) the expression of E-Lig and (d) α4β7 on OT-1 cells in ILN. Histograms were gated on Thy1.1⁺ populations.
FIG. 10. The primary and secondary imprint of tissue-specific homing molecule during acute viral infection was maintained during the memory phase. Lymphocytes were harvested from the indicated tissues on day 30 after rVV-ova skin scarification. The percentages of E-Lig⁺ or α4β7⁺ cells in Thy1.1⁺ OT-1 cells were analyzed by flow cytometry. Data represent the average ± s.d. of 6 mice.

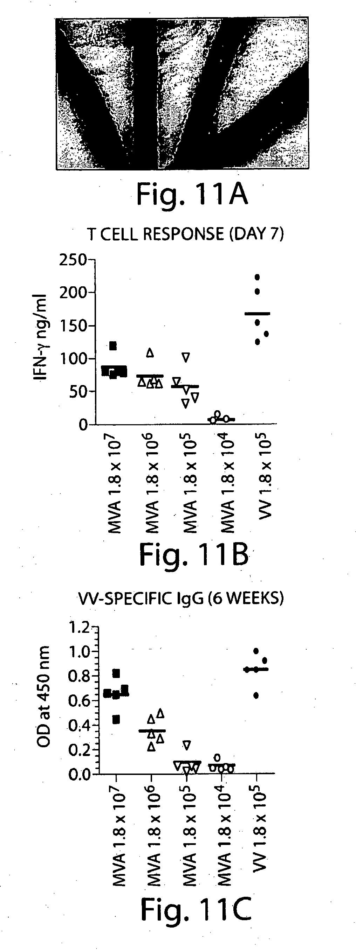

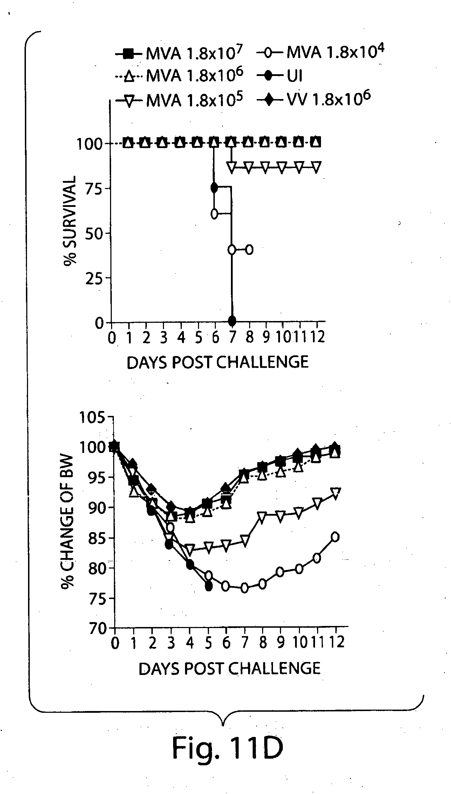

FIG. 11. MVA immunization via skin scarification (s.s.) elicits dose-dependent immune response. (a) B6 mice were immunized via s.s. with different doses of MVA (from left to right: 1.8 x 10⁷, 1.8 x 10⁶, 1.8 x 10⁵ and 1.8 x 10⁴ pfu). At day 7 post-immunization, pox lesions were observed in a dose-dependent manner. (b-c) B6 mice were immunized with MVA or VV by s.s. at the indicated doses (pfu / mouse) (b) 7 days post-immunization, primary vaccinia-specific T cell response was measured in spleens. (c) Serum vaccinia - specific IgG was measured at 6 weeks post-immunization. (d) mice were challenged with lethal i.n. infection with WR-VV at 6 weeks after immunization. Survival and change of BW were monitored daily.

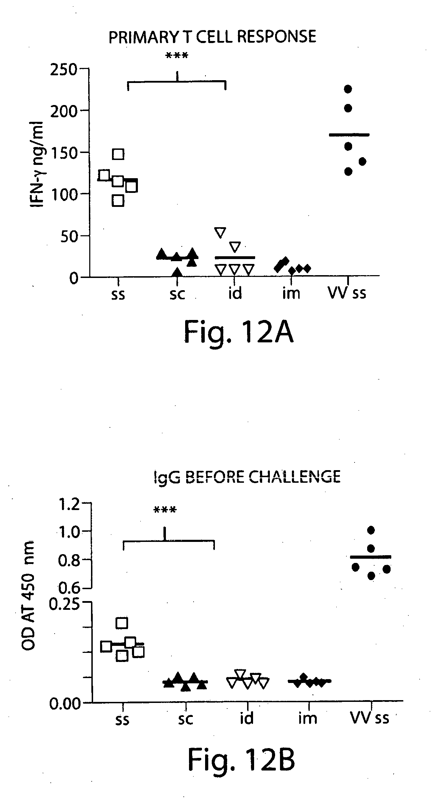

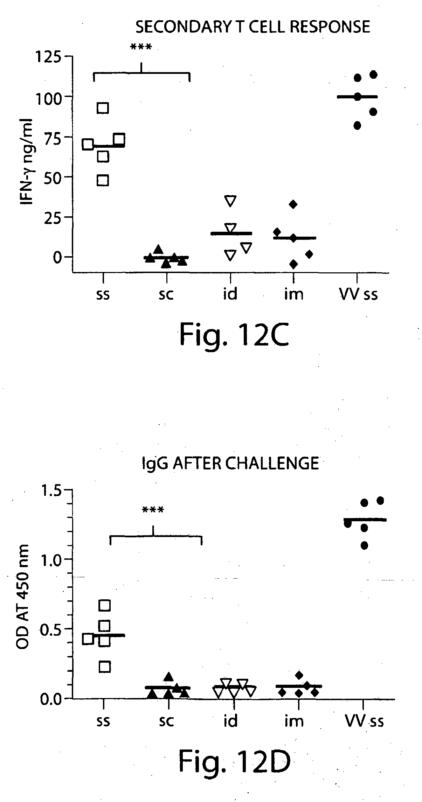

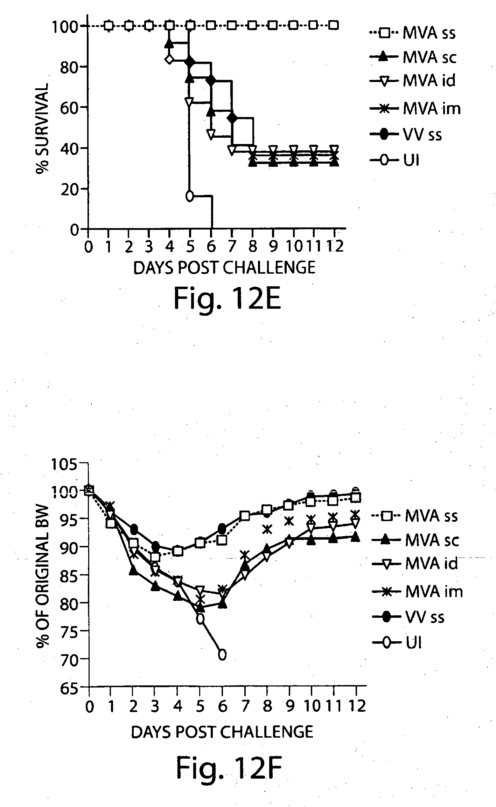

FIG. 12. Skin scarification with MVA offers superior immune response and protection efficacy compared to the injection routes. B6 mice were immunized with 2X10⁶ pfu MVA by the indicated routes. (a) primary T cell response was measured on day 7 p.i. (b) VV-specific IgG was measured at 6 weeks p.i. (c-f) Memory mice were intranasally challenged with lethal dose of WR-VV at 6 weeks p.i. secondary T cell response (c) and post-challenge VV-specific IgG were measured on day 6 challenge (d). Survival (e) and BW change (f) were monitored daily after challenge. VV skin scarified mice (2 x 10⁶ pfu) mice were included as controls.

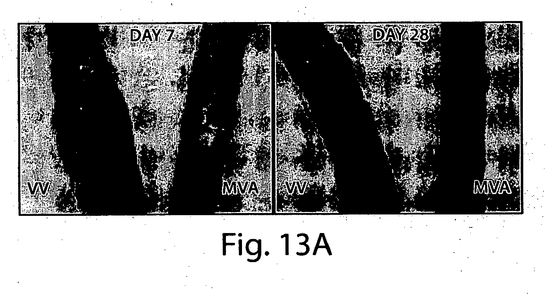

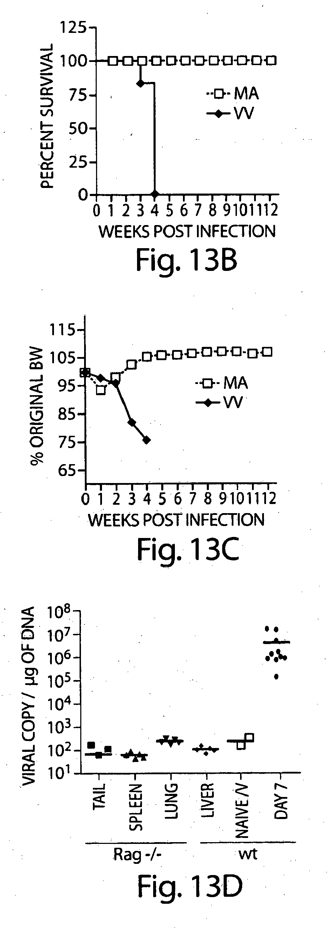

FIG. 13. MVA skin scarification is safe even for immunodeficient hosts. Rag -/mice were immunized with 2 X 10⁶ pfu MVA or VV by skin scarification. (a) Photographs of pox lesion were taken on day 7 and 28 p.i. (b) Survival and (c) BW change were monitored weekly. (d) Viral load harvested from Rag 1 -/- mice at 3 months after MVA scarification was determined by real-time PCR. Naïve skin and day 7 VV-scarified wt mice skin were used as controls.

### DETAILED DESCRIPTION

Aspects of the invention are based, in part, on the discovery that a potent and long-lasting immune response to an antigen can be achieved by administering a modified, replication-deficient or non-replicating poxvirus containing the antigen to a mechanically disrupted epidermis of the subject.

### Methods of Treatment

Disclosed herein are novel methods for stimulating an immune response and/or immunizing a subject against an infection (or infectious disease) or a cancer. The methods comprise-administering to a subject in need thereof a modified, replication-deficient or non-replicating poxvirus vectors containing antigens in an amount sufficient to stimulate the immune response against the antigen, wherein the poxvirus is administered to mechanically disrupted epidermis of the subject.

A subject shall mean a human or a vertebrate animal including but not limited to a dog, cat, horse, cow, pig, sheep, goat, turkey, chicken, primate (e.g., monkey or chimapanzee), rodent (e.g., mouse, rat or hamster) and fish. Preferably the subject is a mammal and more preferably a human.

The methods disclosed herein are useful for stimulating an immune response and/or immunizing a subject in need of such a treatment. A subject in need of treatment is a subject having or at risk of having cancer or a subject having or at risk of having an infection (e.g., a subject having or at risk of contracting a viral, bacterial, fungal or protozoal infection).

A subject having cancer is a subject that has detectable cancerous cells. "Cancer" as used herein refers to an uncontrolled growth of cells which interferes with the normal functioning of the bodily organs and systems.

A subject at risk of developing a cancer is one who has a higher than normal probability of developing cancer. These subjects include, for instance, subjects having a genetic abnormality, the presence of which has been demonstrated to have a correlative relation to a higher likelihood of developing a cancer. These subjects also include subjects exposed to cancer causing agents (i.e., carcinogens) such as tobacco, asbestos, or other chemical toxins, or subjects who have previously been treated for cancer and are in apparent remission.

A subject having an infection is a subject that has been exposed to an infectious microorganism and has acute or chronic detectable levels of the microorganism in his/her body or has signs and symptoms of the infectious microorganism. Methods of assessing and detecting infections in a subject are known by those of ordinary skill in the art.

A subject at risk of having an infection is a subject that may be expected to come in contact with an infectious microorganism. Examples of such subjects are medical workers or those traveling to parts of the world where the incidence of infection is high. In some embodiments, the subject is at an elevated risk of an infection because the subject has one or more risk factors to have an infection. Examples of risk factors to have an infection include, for example, immunosuppression, immunocompromise, age, trauma, burns (e.g., thermal burns), surgery, foreign bodies, cancer, newborns especially newborns born prematurely. The degree of risk of an infection depends on the multitude and the severity or the magnitude of the risk factors that the subject has. Risk charts and prediction algorithms are available for assessing the risk of an infection in a subject based on the presence and severity of risk factors. Other methods of assessing the risk of an infection in a subject are known by those of ordinary skill in the art. The ubject who is at an elevated risk of an infection may be an apparently healthy subject. An apparently healthy subject is a subject who has no signs or symptoms of disease. Viral vectors

Poxviruses are useful vectors for a range of uses, for example vaccines to generate immune responses, for the development of new vaccines, for delivery of desired proteins and for gene therapy. The advantages of these poxvirus vectors include: (i) ease of generation and production, (ii) the large size of the genome permitting insertion of multiple genes (i.e., as a multivalent vector), (iii) efficient delivery of genes to multiple cell types, including antigen-presenting cells, (iv) high levels of protein expression, (v) optimal presentation of antigens to the immune system, and (vi) the ability to elicit cell mediated immune responses as well as antibody responses, (vii) the ability to use combinations of poxviruses from different genera, as they are not immunologically cross-reactive and (viii) the long-term experience gained with using this vector in humans as a smallpox vaccine.

Poxviruses can be genetically engineered to contain and express foreign DNA with or without impairing the ability of the virus to replicate. Such foreign DNA can encode a wide range of proteins, such as antigens that induce protection against one or more infectious agents, immune modulating proteins such as co-stimulatory molecules, or enzymatic proteins. For example, recombinant vaccinia viruses have been engineered to express immunizing antigens of herpesvirus, hepatitis B, rabies, influenza, human immunodeficiency virus (HIV), and other viruses (Kieny et al., Nature 312:163-6 (1984); Smith et al., Nature 302: 490-5 (1983); Smith et al., Proc. Natl. Acad. Sci. USA 80:7155-9 (1983); Zagury et al., Nature 326:249-50 (1987); Cooney et al., Lancet 337:567-72 (1991); Graham et al., J. Infect. Dis. 166:244-52 (1992), and have been shown to elicit immune responses against influenza virus, dengue virus, respiratory syncytial virus, and human immunodeficiency virus (HIV). Poxviruses have also been used to generate immune reactions against tumor-associated antigens such as CEA, PSA and MUC.

Poxviruses are well known cytoplasmic viruses. The genetic material expressed by such viral vectors typically remains in the cytoplasm and does not have the potential for inadvertent integration of the genetic material into host cell genes, unless specif steps are taken. As a result of the non-integrative cytoplasmic nature of the poxvirus, the poxvirus vector system will not result in having term persistence in other cells. Thus, the vector and the transformed cells will not adversely affect cells in the host animal at locations distant from the target cell.

Compared to other systems such as retrovirus vectors (including lentiviral vectors), adenoviral vectors, and adeno-associated virus vectors, the large genome of poxviruses enables large genes to be inserted into pox-based vectors. Advantageously, because of the cytoplasmic nature of the poxvirus integration of foreign DNA into a host cell's genome will not occur.

A number of poxviruses have been developed as live viral vectors for the expression of heterologous proteins, e.g. attenuated vaccinia virus strains Modified Vaccinia Ankara (MVA) and Wyeth (Cepko et al., Cell 37:1053 1062 (1984); Morin et al., Proc. Natl. Acad. Sci. USA 84:4626 4630 (1987); Lowe et al., Proc. Natl. Acad. Sci. USA, 84:3896 3900 (1987); Panicali & Paoletti, Proc. Natl. Acad. Sci. USA, 79:4927 4931(1982); Mackett et al., Proc. Natl. Acad. Sci. USA, 79:7415 7419 (1982)). Other attenuated vaccinia virus strains include WR strain, NYCBH strain, ACAM2000, Lister strain, LC16m8, Elstree-BNm, Copenhagen strain, and Tiantan strain.

Vaccinia virus is the prototype of the genus Orthopoxvirus. It is a double-stranded DNA (deoxyribonucleic acid) virus that has a broad host range under experimental conditions (Fenner et al. Orthopoxviruses. San Diego, Calif.: Academic Press, Inc., 1989; Damaso et al., Virology 277:439-49 (2000)).

Modified vaccinia virus Ankara (MVA) or derivatives thereof have been generated by long-term serial passages of the Ankara strain of vaccinia virus (CVA) on chicken embryo fibroblasts (for review see Mayr, A., et al., Infection, 3:6-14 (1975). The MVA virus itself may be obtained from a number of public repository sources. For example, MVA was deposited in compliance with the requirements of the Budapest Treaty at CNCM (Institut Pasteur, Collection Nationale de Cultures Microorganisms, 25, rue du Docteur Roux, 75724 Paris Cedex 15) on Dec. 15, 1987 under Depositary No. I-721 (U.S. Pat. No. 5,185,146); MVA virus was deposited in compliance with the Budapest Treaty at the European Collection of Cell Cultures (ECACC) (CAMR, Porton Down, Salisbury, SP4 OJG, UK) on Jan. 27,1994, under Depository No. V94012707) (U.S. Pat. No. 6,440,422 and United States patent publication number 2003/0013190). Also, United States patent publication number 2003/0013190 further discloses particular MVA strains deposited at the ECACC under Depository No. 99101431, and ECACC provisional accession number 01021411. Commercially available are THERION-MVA, THERION PRIFREE vectors and THERION M-SERIES vectors (Therion Biologics Corporation, MA).

MVA was generated by 516 serial passages on chicken embryo fibroblasts of the Ankara strain of vaccinia virus (CVA) (for review see Mayr, A., et al. Infection 3, 6-14 [1975]). As a consequence of these long-term passages, about 31 kilobases of the genomic sequence were deleted from the virus (deletion I, II, III, IV, V, and VI) and, therefore, the resulting MVA virus was described as being highly host cell restricted to avian cells (Meyer, H. et al., J. Gen. Virol. 72, 1031-1038 [1991]). It was shown in a variety of animal models that the resulting MVA was significantly avirulent (Mayr, A. & Danner, K. [1978] Dev. Biol. Stand. 41: 225-34). Additionally, this MVA strain has been tested in clinical trials as a vaccine to immunize against the human smallpox disease (Mayr et al., Zbl. Bakt. Hyg. I, Abt. Org. B 167, 375-390 [1987], Stickl et al., Dtsch. med. Wschr. 99, 2386-2392 [1974]). These studies involved over 120,000 humans, including high-risk patients, and proved that compared to vaccinia based vaccines, MVA had diminished virulence or infectiousness while it induced a good specific immune response. Generally, a virus strain is regarded as attenuated if it has lost its capacity or only has reduced capacity to reproductively replicate in host cells.

As used herein, the term "non-replicating" or "replication-impaired" poxvirus refers to a poxvirus that is not capable of replication to any significant extent in the majority of normal mammalian cells or normal primary human cells. As used herein "significant extent" means a replication capability of 75% or less as compared to wild-type vaccinia virus in standardized assays. In some embodiments, the poxvirus has a replication capability of 65%, 55%, 45%, 35%, 25%, or 15% compared to wild-type vaccinia virus. In some embodiments, the poxvirus has a replication capability 5% or less, or 1% or less compared to wild-type vaccinia virus. Non-replicating viruses are 100% replication deficient in normal primary human cells.

Viral replication assays are known in the art, and can be performed for vaccinia viruses on e.g. primary keratinocytes, and are described for example in Liu et al. J.Virol. 2005, 79:12, 7363-70. Viruses which are non-replicating or replication-impaired may have become so naturally (i.e. they may be isolated as such from nature) or artificially e.g. by breeding in vitro or by genetic manipulation, for example deletion of a gene which is critical for replication. There will generally be one or a few cell types in which the viruses can be grown, such as CEF cells for MVA.

As used herein a "modified" poxvirus refers to a poxvirus that has been altered in some way that changes one or more characteristics of the modified virus compared to the wild-type virus. These changes may have occurred naturally or through engineering. In some embodiments, the modified poxvirus is altered to include an antigen(s) that are immunogenic (i.e., induce an immune response in a host). Antigens include, for example, cancer antigens or microbial antigens.

In some embodiments, changes in the poxvirus include, for example, alterations in the gene expression profile of the virus. The modified poxvirus expresses genes or portions of genes that encode peptides or polypeptides that are foreign to the poxvirus, i.e. would not be found in a wild-type poxvirus. These foreign, or heterologous peptides or polypeptides may comprise sequences that are immunogenic such as, for example, tumor-specific antigens (TSAs), bacterial, viral, fungal, and protozoal antigens, or antigenic sequences derived from viruses other than poxvirus. In some embodiments, the modified poxviruses described herein are capable of expressing non-poxviral polypeptides comprising antigens and are additionally attenuated, that is they have less ability to spread due to modifications affecting the rate of viral replication, such that these modified poxviruses are replication-deficient or non-replicating.

One of the advantages of poxviruses as vectors is the large size of their genomes, which permits the insertion of a wide range of genetic material including multiple genes (i.e., as a multivalent vector). The genetic material may be inserted at an appropriate site within the poxvirus genome for the recombinant virus to remain viable, i.e. the genetic material may be inserted at a site in the viral DNA (e.g., non-essential site in the viral DNA) to ensure that the recombinant virus retains the ability to infect foreign cells and to express DNA, while maintaining the desired immunogenicity and diminished virulence. For example, as described above, MVA contains 6 natural deletion sites, which have been demonstrated to serve as insertion sites. See, for example, U.S. Pat. No. 5,185,146, and U.S. Pat. No. 6,440,422. The poxviruses described herein are sometimes referred to herein as a viral vector or viral vector system.

In some embodiments, genes that code for desired antigens are inserted into the genome of a poxvirus in such a manner as to allow them to be expressed by that virus along with the expression of the normal complement of parent virus proteins. This can be accomplished by first constructing a DNA donor vector for in vivo recombination with a poxvirus.

In general, the DNA donor vector contains the following elements: (i) a prokaryotic origin of replication, so that the vector may be amplified in a prokaryotic host; (ii) a gene encoding a marker which allows selection of prokaryotic host cells that contain the vector (e.g., a gene encoding antibiotic resistance); (iii) at least one gene encoding a desired protein located adjacent to a transcriptional promoter capable of directing the expression of the gene; and (iv) DNA sequences homologous to the region of the parent virus genome where the foreign gene(s) will be inserted, flanking the construct of element (iii).

Methods for constructing donor plasmids for the introduction of multiple foreign genes into poxvirus are described, for example, in WO91/19803. In general, all DNA fragments for construction of the donor vector, including fragments containing transcriptional promoters and fragments containing sequences homologous to the region of the parent virus genome into which foreign genes are to be inserted, can be obtained from genomic DNA or cloned DNA fragments. The donor plasmids can be mono-, di-, or multivalent (i.e., can contain one or more inserted foreign gene sequences).

The donor vector may contain an additional gene which encodes a marker which will allow identification of recombinant viruses containing inserted foreign DNA. Several types of marker genes can be used to permit the identification and isolation of recombinant viruses. These include, for example, genes that encode antibiotic or chemical resistance (e.g., see Spyropoulos et al., J. Virol., 62:1046 (1988); Falkner and Moss., J. Virol., 62:1849 (1988); Franke et al., Mol. Cell. Biol., 5:1918 (1985), as well as genes such as the E. coli lacZ gene, that permit identification of recombinant viral plaques by colorimetric assay (Panicali et al., Gene, 47:193 199 (1986)).

Homologous recombination between donor plasmid DNA and viral DNA in an infected cell results in the formation of recombinant viruses that incorporate the desired elements. Appropriate host cells for in vivo recombination are generally eukaryotic cells that can be infected by the virus and transfected by the plasmid vector. Examples of such cells suitable for use with a poxvirus are chick embryo dermal (CED) cells, HuTK143 (human) cells, and CV-1 and BSC-40 (both monkey kidney) cells. Infection of cells with poxvirus and transfection of these cells with plasmid vectors is accomplished by techniques standard in the art (Panicali and Paoletti, U.S. Pat. No. 4,603,112, WO89/03429). Alternatively, the donor DNA can be directly ligated into the parental virus genome at a unique restriction site (Scheiflinger, et al. (1992) Proc. Natl. Acad. Sci. (USA) 89:9977 9981).

Following in vivo recombination or ligation, recombinant viral progeny can be identified by several techniques well known in the art. For example, if the DNA donor vector is designed to insert foreign genes into the parent virus thymidine kinase (TK) gene, viruses containing integrated DNA will be TK⁻ and can be selected on this basis (Mackett et al., Proc. Natl. Acad. Sci. USA, 79:7415 (1982)). Alternatively, co-integration of a gene encoding a marker or indicator gene with the foreign gene(s) of interest, as described above, can be used to identify recombinant progeny. One preferred indicator gene is the *E. coli* lacZ gene: recombinant viruses expressing β-galactosidase can be selected using a chromogenic substrate for the enzyme (Panicali et al., Gene, 47:193 (1986)).

Once a recombinant virus has been identified, a variety of methods well known in the art can be used to assay the expression of the polypeptide encoded by the inserted gene. These methods include, for example, black plaque assay (an in situ enzyme immunoassay performed on viral plaques), Western blot analysis, radioimmunoprecipitation (RIPA), enzyme immunoassay (EIA), or functional assay such as CTL assay.

Because poxviruses have a large genome, they can readily be used to deliver a wide range of genetic material including multiple genes (i.e., act as a multivalent vector). The sizes of the poxvirus genomes ranges between about 130-300 kbp with up to 300 genes, depending on the strain of the virus. Therefore, it is possible to insert large fragments of foreign DNA into these viruses and yet maintain stability of the viral genome.

### Antigens and Immunostimulatory Molecules Expressed by Viral Vector

At least one nucleic acid fragment encoding a gene is inserted into a poxvirus vector. In another embodiment at least two and up to about ten different nucleic acids encoding different genes are inserted into the poxvirus vector.

In some embodiments, the poxvirus has a DNA encoding a disease-related antigen of interest, such as an antigen(s) from a disease causing agent or an antigen associate with a disease state, inserted and expresses that antigen(s).

In certain embodiments, the poxvirus has the DNA encoding a co-stimulatory molecule(s) inserted and expresses the co-stimulatory molecule(s).

In certain embodiments, the poxvirus has the DNA encoding a disease-related antigen(s) of interest and a co-stimulatory molecule(s) inserted and expresses the antigen(s) and co-stimulatory molecule(s).

Any DNA of interest can be inserted into the poxvirus vector described herein. Foreign genes for insertion into the genome of a poxvirus in expressible form can be obtained by any conventional technique for isolating a desired gene.

For organisms which contain a DNA genome, the genes encoding an antigen of interest may be isolated from the genomic DNA; for organisms with RNA genomes, the desired gene may be isolated from cDNA copies of the genome. If restriction maps of the genome are available, strategies can be designed for cleaving genomic DNA by restriction endonuclease digestion to yield DNA fragments that contain the gene of interest. In some cases, desired genes may have been previously cloned and thus, the genes can be obtained from the available clones. Alternatively, if the DNA sequence of the gene is known, the gene can be synthesized by any of the conventional techniques for polymerase chain reaction or synthesis of deoxyribonucleic acids (e.g., the phosphate or phosphite triester techniques).

### Engineering of the Viral Vectors to Express Antigens

Genes encoding an antigen of interest can be amplified by cloning the gene into a bacterial host. For this purpose, various prokaryotic cloning vectors can be used. Examples are plasmids pBR322 and pEMBL.

The genes encoding the antigen of interest can be prepared for insertion into the poxvirus vectors by standard techniques. In general, the cloned genes can be excised from the prokaryotic cloning vector by restriction enzyme digestion. The DNA fragment carrying the cloned gene can be modified as needed, for example, to make the ends of the fragment compatible with the insertion sites of the poxvirus vectors, then purified prior to insertion into these vectors at restriction endonuclease cleavage sites (cloning sites). The basic techniques of inserting genes into viruses are known to the skilled artisan and involve, for example, recombination between the viral DNA sequences flanking a gene in a donor plasmid and homologous sequences present in the parental virus (Mackett, et al., Proc. Natl. Acad. Sci. USA 79:7415-7419 (1982)).

For example, the DNA gene sequence to be inserted into the virus can be placed into a plasmid, e.g., an E. coli plasmid construct, into which DNA homologous to a section of DNA such as that of the poxvirus has been inserted. Separately, the DNA gene sequence to be inserted is ligated to a promoter. The promoter-gene linkage is positioned in the plasmid construct so that the promoter-gene linkage is flanked on both ends by DNA homologous to a DNA sequence flanking a region of pox DNA which is the desired insertion region. The resulting plasmid construct is then amplified by growth within E. coli bacteria and isolated. Preferably, the plasmid also contains an origin of replication such as the E. coli origin of replication, and a marker such as an antibiotic resistance gene for selection and propagation in E. coli. The isolated plasmid containing the DNA gene sequence to be inserted is transfected into a cell culture, e.g., chick embryo fibroblasts, along with the poxvirus. Recombination between homologous pox DNA in the plasmid and the viral genome respectively results in a poxvirus modified by the presence of the promoter-gene construct in its genome, at a site which does not affect virus viability.

In certain embodiments insertion of more than one nucleic acid, for example one or more antigen(s) and co-stimulatory molecule(s).

In some embodiments, the gene(s) may be inserted into a site or region (insertion region) in the poxvirus which does not majorly affect virus viability of the resultant recombinant virus, e.g. intragenic regions between viral genes, preferably non-essential viral genes. The skilled artisan can readily identify such regions in a virus by, for example, testing segments of virus DNA for regions that allow recombinant formation without seriously affecting virus viability of the recombinant. One region that can readily be used and is present in many viruses, for example, is the thymidine kinase gene that has been found in all poxvirus genomes examined (leporipoxvirus: Upton, et al., J. Virology, 60:920 (1986) (shope fibroma virus); capripoxvirus: Gershon, et al., J. Gen. Virol., 70:525 (1989) (Kenya sheep-1); orthopoxvirus: Weir, et al., J. Virol., 46:530 (1983) (vaccinia); Esposito, et al., Virology, 135:561 (1984) (monkeypox and variola virus); Hruby, et al., PNAS, 80:3411 (1983) (vaccinia); Kilpatrick, et al., Virology, 143:399 (1985)(Yaba monkey tumor virus); avipoxvirus: Binns, et al., J. Gen. Virol. 69:1275 (1988) (fowlpox); Boyle, et al., Virology, 156:355 (1987) (fowlpox); Schnitzlein, et al., J. Virological Methods, 20:341 (1988) (fowlpox, quailpox); entomopox (Lytvyn, et al., J. Gen. Virol. 73:3235-3240 (1992)). In fowlpox, in addition to the TK region, other insertion regions include, for example, BamHI J (Jenkins, et al., AIDS Research and Human-Retroviruses 7:991-998 (1991)) the EcoRI-HindIII fragment, BamHI fragment, EcoRV-HindHIII fragment, BamHI fragment and the HindIII fragment set forth in EPO Application No. 0 308 220 A1. (Calvert, et al., J. of Virol. 67:3069-3076 (1993); Taylor, et al., Vaccine 6:497-503 (1988); Spehner, et al., (1990) and Boursnell, et al., J. of Gen. Virol. 71:621-628 (1990)).

In some embodiments, the poxviruses described herein may have optionally incorporated into their genome one or more genes or portions thereof encoding one or more immunostimulatory molecules or genes or portion thereof.

In certain embodiments the foreign DNA does not encode an entire protein but encodes antigenic fragments of proteins or epitopes. These fragments may be of any length sufficient to be immunogenic or antigenic. Fragments may be at least 4 amino acids long, preferably 5-9 amino acids, but may be longer, such as e.g. 10, 11, 12, 13, 14, 15, 16, 17, 18, 19, 20, 30, 40, 50, 60, 70, 80, 90, 100, 500 amino acids long or more, or any length in between. Preferably, epitopes that induce a protective immune response to any of a variety of pathogens, such as bacteria, viruses, fungi and protozoae such as those described herein may be expressed and may be combined with heterologous gene sequences that encode proteins with immunomodulating activities, such as cytokines, interferon type 1, gamma interferon, colony stimulating factors, interleukin-1, -2, -4, -5, - 6,-12.

In some embodiments, heterologous sequences may be derived from tumor antigens, and the resulting recombinant poxviruses may be used to generate an immune response against the tumor cells leading to tumor regression in vivo. Recombinant viruses may be engineered to express a tumor-associated antigen (TAA), a tumor-specific antigen (TSA), or a tissue-specific antigen.

In some embodiments, the inserted gene(s) encoding antigens may be operably linked to a promoter to express the inserted gene. Promoters are well known in the art and can readily be selected depending on the host and the cell type one wishes to target. For example in poxviruses, poxviral promoters may be used, such as the vaccinia 7.5K, 40K, fowlpox. In certain embodiments, enhancer elements can also be used in combination to increase the level of expression. In certain embodiments, inducible promoters, which are also well known in the art, may be used.

In some embodiments, poxvirus promoters include, e.g., an entomopox promoter, an avipox promoter, or an orthopox promoter such as a vaccinia promoter, e.g., HH, 11K or Pi. For example, the Pi promoter, from the Ava I H region of vaccinia, is described in Wachsman et al., J. of Inf. Dis. 155, 1188-1197 (1987). More particularly, this promoter is derived from the Ava I H (Xho I G) fragment of the L-variant WR vaccinia strain, in which the promoter directs transcription from right to left. The map location of the promoter is approximately 1.3 Kbp (kilobase pair) from the 5' end of Ava IH, approximately 12.5 Kbp from the 5' end of the vaccinia genome, and about 8.5 Kbp 5' of the Hind III C/N junction. The Hind III H promoter (also "HH" and "H6" herein) sequence is an up-stream of open reading frame H6 by Rosel et al., J. Virol. 60, 436-449 (1986). The 11K promoter is as described by Wittek, J. Virol. 49, 371-378 (1984) and Bertholet, C. et al., Proc. Natl. Acad. Sci. USA 82, 2096-2100 (1985). One can take advantage of whether the promoter is an early or late promoter to time expression of particular genes.

In some embodiments, the poxvirus vector comprises a promoter that is modulated by an external factor or cue, allowing control of the level of polypeptide being produced by the vectors by activating that external factor or cue. For example, heat shock proteins are proteins encoded by genes in which the promoter is regulated by temperature. The promoter of the gene which encodes the metal-containing protein metallothionine is responsive to Cd⁺ ions. Incorporation of this promoter or another promoter influenced by external cues also make it possible to regulate the production of the polypeptides comprising antigen.

In some embodiments, the poxvirus genome is modified to carry a nucleic acid encoding at least one gene of interest encoding, e.g. an antigen, which is operably linked to an "inducible" promoter. Such inducible systems allow careful regulation of gene expression. See, Miller and Whelan, Human Gene Therapy, 8:803-815 (1997). The phrase "inducible promoter" or "inducible system" as used herein includes systems wherein promoter activity can be regulated using an externally delivered agent. Such systems include, for example, systems using the lac repressor from E. coli as a transcription modulator to regulate transcription from lac operator-bearing mammalian cell promoters (Brown et al. Cell, 49:603-612, 1987); systems using the tetracycline repressor (tetR)(Gossen and Bujard, Proc. Natl. Acad. Sci. USA 89: 5547-5551, 1992; Yao et al., Human Gene Ther. 9:1939-1950, 1998; Shokelt et al., Proc. Natl. Acad. Sci. USA 92.6522-6526, 1995). Other such systems include FK506 dimer, VP16 or p65 using castradiol, RU486/mifepristone, diphenol muristerone or rapamycin (see, Miller and Whelan, supra, at FIG. 2). Yet another example is an ecdysone inducible system (see, e.g. Karns et al, MBC Biotechnology 1:11, 2001). Inducible systems are available, e.g., from Invitrogen, Clontech, and Ariad. Systems using a repressor with the operon are preferred. These promoters may be adapted by substituting portions of pox promoters for the mammalian promoter.

In some embodiments, a "transcriptional regulatory element" or "TRE" is introduced for regulation of the gene of interest. As used herein, a TRE is a polynucleotide sequence, preferably a DNA sequence, that regulates (i.e., controls) transcription of an operably-linked polynucleotide sequence by an RNA polymerase to form RNA. As used herein, a TRE increases transcription of an operably linked polynucleotide sequence in a host cell that allows the TRE to function. The TRE comprises an enhancer element and/or pox promoter element, which may or may not be derived from the same gene. The promoter and enhancer components of a TRE may be in any orientation and/or distance from the coding sequence of interest, and comprise multimers of the foregoing, as long as the desired, transcriptional activity is obtained.

In some embodiments, an "enhancer" for regulation of the gene of interest is provided. An enhancer is a term well understood in the art and is a polynucleotide sequence derived from a gene which increases transcription of a gene which is operably-linked to a promoter to an extent which is greater than the transcription activation effected by the promoter itself when operably-linked to the gene, i:e. it increases transcription from the promoter.

The activity of a regulatory element such as a TRE or an enhancer generally depends upon the presence of transcriptional regulatory factors and/or the absence of transcriptional regulatory inhibitors. Transcriptional activation can be measured in a number of ways known in the art, but is generally measured by detection and/or quantification of mRNA or the protein product of the coding sequence under control of (i.e., operatively linked to) the regulatory element. The regulatory element can be of varying lengths, and of varying sequence composition. By transcriptional activation, it is intended that transcription will be increased above basal levels in the target cell by at least about 2-fold, preferably at least about 5-fold, preferably at least about 10-fold, more preferably at least about 20-fold. More preferably at least about 50-fold, more preferably at least about 100-fold, even more preferably at least about 200-fold, even more preferably at least about 400- to about 500-fold, even more preferably, at least about 1000-fold. Basal levels are generally the level of activity, if any, in a non-target cell, or the level of activity (if any) of a reporter construct lacking the TRE or enhancer of interest as tested in a target cell type.

Certain point mutations within sequences of TREs have been shown to decrease transcription factor binding and gene activation. One of skill in the art would recognize that some alterations of bases in and around known transcription factor binding sites are more likely to negatively affect gene activation and cell-specificity, while alterations in bases which are not involved in transcription factor binding are not as likely to have such effects. Certain mutations are also capable of increasing TRE activity. Testing of the effects of altering bases may be performed in vitro or in vivo by any method known in the art, such as mobility shift assays, or transfecting vectors containing these alterations in TRE functional and TRE non-functional cells. Additionally, one of skill in the art would recognize that point mutations and deletions can be made to a TRE sequence without altering the ability of the sequence to regulate transcription.

### Disorders to be Treated or Prevented by Vaccination with Viral Vectors

As described herein, poxvirus vectors provided herein encode antigenic or immunogenic or fragments (antigens). The antigen of interest can be an antigen from a pathogenic microorganism or a tumor (or cancer) antigen. The genes or nucleic acid fragments encoding the antigen can be derived from any organism, including bacteria, viruses, fungi, protozoa, parasites, normal or transformed cells, or other microororganisms.

In some embodiments genes of interest are those which encode immunogenic proteins of a pathogenic organism. In certain embodiments, these may be protein components of surface structures such as the bacterial cell wall or viral envelope.

### Cancers

In some embodiments, genes of interest are those which encode tumor-associated antigens (TAAs), tumor specific antigens (TSAs), tissue-specific antigens, viral tumor antigens, cellular oncogene proteins, and/or tumor-associated differentiation antigens.

In some embodiments, immunogenic fragments or subunits of the proteins may be used. Multiple immunogenic fragments or subunits of various proteins may be used. For example, several different epitopes from different sites of a single protein or from different proteins of the same species, or from a protein ortholog from different species may be expressed.

The immunotherapeutic approach to the treatment of cancer is based on the observation that human tumor cells express a variety of tumor-associated antigens (TAAs) or tumor specific antigens (TSAs) that are not typically expressed in normal tissues. These antigens can serve as targets for the host immune system and elicit responses which result in tumor destruction. This immune response is mediated primarily by lymphocytes; T cells in general and class I MHC-restricted cytotoxic T lymphocytes in particular play a central role in tumor rejection. Hellstrom, K. E., et al., (1969) Adv. Cancer Res. 12:167 223; Greenberg, P. D. (1991) in Advances in Immunology, vol. 49 (Dixon, D. J., ed.), pp. 281 355, Academic Press, Inc., Orlando, FL. The cloning of TAAs for cancer immunotherapy is described e.g. in Boon, T., et al., (1994) Annu. Rev. Immunol. 12:337 365; Brithcard, V., et al., (1993) J. Exp. Med. 178:489 495; Cox, A. L., et al., (1994) Science 264:716 719; Houghton, A. N. (1994) J. Exp. Med. 180:1 4; Pardoll, D. M. (1994) Nature 369:357 358; Kawakami, Y., et al., (1994) Proc. Natl. Acad. Sci. U.S.A. 91:3515 3519; Kawakami, Y., et al., (1994) Proc. Natl. Acad. Sci. U.S.A. 91:6458 6462.

The use of vaccinia viruses for anti-tumor immunotherapy has been described e.g. in Hu, S. L., Hellstrom, I., and Hellstrom K. E. (1992) in Vaccines: New Approaches to Immunological Problems (R. W. Ellis, ed) pp. 327 343, Butterworth-Heinemann, Boston. Anti-tumor responses have been elicited using recombinant pox viruses expressing TAAs such as carcinoembryonic antigen (CEA) and prostrate specific antigen (PSA). (Muraro, R., et al., (1985) Cancer Res. 4S:5769 5780); (Kantor, 3., et al. (1992) J. Natl. Cancer Inst. 84:1084 1091); (Robbins, P. F., et al. (1991) Cancer Res. 51:3657 3662) (Kantor, 3., et al. (1992) Cancer Res. 52:6917 6925.) No toxicity with these vectors was observed.

In general, viral vaccines are believed to mediate tumor rejection by activating class I MHC-restricted T-cells, particularly cytotoxic T lymphocytes (CTLs). T-cell activation is often potentiated by providing a suitable immunomodulator, for example a T-cell co-stimulatory factor such as those of the B7 gene family. See e.g., Greenberg, P. D. (1991) in Advances in Immunology, Vol. 49 (Dixon, D. J., ed.), pp. 281 355, Academic Press, Inc., Orlando, Fla.; Fox B. A. et al. (1990) J. Biol. Response Mod. 9:499 511.

In some embodiments antigens are selected from the group consisting of tumor associated antigen (TAA), tumor specific antigens (TSA), and/or tissue-specific antigens. These antigens include, but are not limited to, melanoma TAAs which include but are not limited to MART-1 (Kawakami et al. J. Exp. Med. 180:347-352, 1994), MAGE-1, MAGE-3, GP-100, (Kawakami et al. Proc. Nat'l. Acad. Sci. U.S.A. 91:6458-6462, 1994), CEA and tyrosinase (Brichard et al. J. Exp. Med. 178:489, 1993), TAAs such as MUC-1, MUC-2, the point mutated ras oncogene and the point mutated p53 oncogenes (pancreatic cancer), CA-125 (ovarian cancer), PSA (prostate cancer), c-erb/B2 (breast cancer), KS 1/4 pan-carcinoma antigen (Perez and Walker, 1990, J. Immunol. 142:3662-3667; Bumal, 1988, Hybridoma 7(4):407-415), ovarian carcinoma antigen (CA125) (Yu et al., 1991, Cancer Res. 51(2):468-475), prostatic acid phosphate (Tailor et al., 1990, Nucl. Acids Res. 18(16):4928), prostate specific antigen (PSA) (Henttu and Vihko, 1989, Biochem. Biophys. Res. Comm. 160(2):903-910; Israeli et al., 1993, Cancer Res. 53:227-230), melanoma-associated antigen p97 (Estin et al., 1989, J. Natl. Cancer Instit. 81(6):445-446), melanoma antigen gp75 (Vijayasardahl et al., 1990, J. Exp. Med. 171(4):1375-1380), high molecular weight melanoma antigen (HMW-MAA) (Natali et al., 1987, Cancer 59:55-63; Mittelman et al., 1990, J. Clin. Invest. 86:2136-2144), prostate specific membrane antigen, carcinoembryonic antigen (CEA) (Foon et al., 1994, Proc. Am. Soc. Clin. Oncol. 13:294), polymorphic epithelial mucin antigen, human milk fat globule antigen, colorectal tumor-associated antigens such as: CEA, TAG-72 (Yokata et al., 1992, Cancer Res. 52:3402-3408), CO17-1A (Ragnhammar et al., 1993, Int. J. Cancer 53:751-758); GICA 19-9 (Herlyn et al., 1982, J. Clin. Immunol. 2:135), CTA-1 and LEA, Burkitt's lymphoma antigen-38.13, CD19 (Ghetie et al., 1994, Blood 83:1329-1336), human B-lymphoma antigen-CD20 (Reffet al., 1994, Blood 83:435-445), CD33 (Sgouros et al., 1993, J. Nucl. Med. 34:422-430), melanoma specific antigens such as ganglioside GD2 (Saleh et al., 1993, J. Immunol., 151, 3390-3398), ganglioside GD3 (Shitara et al., 1993, Cancer Immunol. Immunother. 36:373-380), ganglioside GM2 (Livingston et al., 1994, J. Clin. Oncol. 12:1036-1044), ganglioside GM3 (Hoon et al., 1993, Cancer Res. 53:5244-5250), tumor-specific transplantation type of cell-surface antigen (TSTA) such as virally-induced tumor antigens including T-antigen DNA tumor viruses and Envelope antigens of RNA tumor viruses, oncofetal antigen-alpha-fetoprotein such as CEA of colon, bladder tumor oncofetal antigen (Hellstrom et al., 1985, Cancer. Res. 45:2210-2188), differentiation antigen such as human lung carcinoma antigen L6, L20 (Hellstrom et al., 1986, Cancer Res. 46:3917-3923), antigens of fibrosarcoma, human leukemia T cell antigen-Gp37 (Bhattacharya-Chatterjee et al., 1988, J. of Immuno specifically. 141:1398-1403), neoglycoprotein, sphingolipids, breast cancer antigen such as EGFR (Epidermal growth factor receptor), HER2 antigen (p185^{HER2}), polymorphic epithelial mucin (PEM) (Hilkens et al., 1992, Trends in Bio. Chem. Sci. 17:359), malignant human lymphocyte antigen-APO-1 (Bernhard et al., 1989, Science 245:301-304), differentiation antigen (Feizi, 1985, Nature 314:53-57) such as I antigen found in fetal erythrocytes, primary endoderm, I antigen found in adult erythrocytes, preimplantation embryos, I(Ma) found in gastric adenocarcinomas, M18, M39 found in breast epithelium, SSEA-1 found in myeloid cells, VEP8, VEP9, Myl, VIM-D5, D₁₅₆₋₂₂ found in colorectal cancer, TRA-1-85 (blood group H), C14 found in colonic adenocarcinoma, F3 found in lung adenocarcinoma, AH6 found in gastric cancer, Y hapten, Le^{y} found in embryonal carcinoma cells, TL5 (blood group A), EGF receptor found in A431 cells, E₁ series (blood group B) found in pancreatic cancer, FC10.2 found in embryonal carcinoma cells, gastric adenocarcinoma antigen, CO-514 (blood group Le^{a}) found in Adenocarcinoma, NS-10 found in adenocarcinomas, CO-43 (blood group Le^{b}), G49 found in EGF receptor of A431 cells, MH2 (blood group ALe^{b}/Le^{y}) found in colonic adenocarcinoma, 19.9 found in colon cancer, gastric cancer mucins, T_{5A7} found in myeloid cells, R₂₄ found in melanoma, 4.2, G_{D3}, D1.1, OFA-1, G_{M2}, OFA-2, G_{D2}, and M1:22:25:8 found in embryonal carcinoma cells, and SSEA-3 and SSEA-4 found in 4 to 8-cell stage embryos T cell receptor derived peptides from Cutaneous T cell Lymphoma (Edelson, 1998, The Cancer Journal 4:62), IL-1, IL-2, IL-3, IL-4, IL-5, IL-6, IL-7, IL-8, IL-9, IL-10, IL-11, IL-12, IFN-α, IFN-β, IFN-β 17 mutants, IFN-65, CD2, CD3, CD4, CD5, CD8, CD11a, CD11b, CD11c, CD16, CD18, CD21, CD28, CD32, CD34, CD35, CD40, CD44, CD45, CD54, CD56, OX40L, 4-1BBL, K2, K1, Pβ, Oα, Mα, Mβ2, Mβ1, Hepsin, Pim-1, LMP1, TAP2, LMP7, TAP1, TRP, Oβ, IAβ, IAα, IEβ, IEβ2, IEα, CYP21, C4B, CYP21P, C4A, Bf, C2, HSP, G7a/b, TNF-α, TNF-β, D, L, Qa, T1α, COL11A2, DPβ2, DPα2, DPβ1, DPα1, DNα, DMα, DMβ, LMP2, TAPi1, LMP7, DOβ, DQβ2, DQα2, DQβ3, DQβ1, DQα1, DRβ, DRα, G250, HSP-70, HLA-B, HLA-C, HLA-X, HLA-E, HLA-J, HLA-A, HLA-H, HLA-G, HLA-F, nerve growth factor, somatotropin, somatomedins, parathormone, FSH, LH, EGF, TSH, THS-releasing factor, HGH, GRHR, PDGF, IGF-I, IGF-II, TGF-β, GM-CSF, M-CSF, G-CSF1, erythropoietin, β-HCG, 4-N-acetylgalactosaminyltransferase, GM2, GD2, GD3, JADE, MART, BAGE, GAGE, MAGE-1, MAGE-2, MAGE-3, XAGE, MUC-1, MUC-2, MUC-3, MUC-4, MUC-18, ICAM-1, C-CAM, V-CAM, ELAM, NM23, EGFR, E-cadherin, N-CAM, LFA-3 (CD58), EpCAM, B7.1, CEA, DCC, PSA, Her2-neu, UTAA, melanoma antigen p75, K19, HKer 8, pMel 17, TP10, tyrosinase related proteins 1 and 2, p97, p53, RB, APC, DCC, NF-1, NF-2, WT-1, MEN-I, MEN-II, BRCA1, VHL, FCC and MCC, ras, myc, neu, raf, erb, src, fms, jun, trk, ret, gsp, hst, bcl and abl, C1q, C1r, C1s, C4, C2, Factor D, Factor B, properdin, C3, C5, C6, C7, C8, C9, C1Inh, Factor H, C4b-binding protein, DAF, membrane cofactor protein, anaphylatoxin inactivator S protein, HRF, MIRL, CR1, CR2, CR3, CR4, C3a/C4a receptor, C5a receptor, Epstein-Barr Virus antigens (EBNA), BZLF-1, BXLF-1, and Nuclear Matrix Proteins, modified TAAs or TSAs, splice variants of TAAs or TSAs, functional epitopes, epitope agonists, and degenerate nucleic acid variations thereof.

In some important embodiments, antigens include: mucin 1 (MUC1), mucin 2 (MUC2), BAGE-1, GAGE-1~8; GnTV, HERV-K-MEL, KK-LC-1, KM-HN-1, LAGE-1, MAGE-A1~A4, MAGE-A6, MAGE-A9, MAGE-A10, MAGE-A12,MAGE- C3,NA88, NY-ESO-1 / LAGE-2, SAGE, Sp17, SSX-2, SSX-4, TAG-1, TAG-2, TRAG-3, TRP2-INT2, XAGE-1b, carcinoembriogenic antigen (CEA), CA-125, gp100 / Pmel17, Kallikrein 4, mammaglobin-A, Melan-A / MART-1, NY-BR-1, OA-1, prostate specific antigen (PSA), RAB38 / NY-MEL-1, TRP-1 / gp75, TRP-2, tyrosinase, adipophilin, AIM-2, ALDH1A1, BCLX (L), BING-4, CPSF, cyclin D1, DKK1, ENAH (hMena), Ep-CAM, EphA3, EZH2, FGF5, G250 / MN / CAIX, HER-2 / neu, IL13Ralpha2, Intestinal carboxyl esterase, alpha-foetoprotein(AFP), M-CSF, MCSP, mdm-2, MMP-2, MUC1, PBF, PRAME, PSMA, RAGE-1, RGS5, RNF43, RU2AS, secernin 1, SOX10, STEAP1, survivin, Telomerase, VEGF, WT1, alpha-actinin-4, ARTC1, BCR-ABL fusion protein, B-RAF, CASP-5, CASP-8, beta-catenin, Cdc27, CDK4, CDKN2A, COA-1, dek-can fusion protein, EFTUD2, Elongation factor 2, ETV6-AML1 fusion protein, FLT3-ITD, FN1, GPNMB, LDLR-fucosyltransferaseAS fusion protein, HLA-A2, HLA-A11, hsp70-2, KIAAO205, MART2, ME1, MUM-1, MUM-2, MUM-3, neo-PAP, Myosin class I, NFYC, OGT, OS-9, p53, pml-RARalpha fusion protein, PRDX5, PTPRK, K-ras, N-ras, RBAF600, SIRT2, SNRPD1, SYT-SSX1 or -SSX2 fusion protein, TGF-betaRII, and Triosephosphate Isomerase.

In some embodiments, genes of interest are those encoding an antigen of a disease causing pathogenic microorganism. These include viruses such as influenza virus hemagglutinin (Genbank accession no. JO2132; Air, 1981, Proc. Natl. Acad. Sci. USA 78:7639-7643; Newton et al., 1983, Virology 128:495-501), human respiratory syncytial virus G glycoprotein (Genbank accession no. Z33429; Garcia et al., 1994, J. Virol.; Collins et al., 1984, Proc. Natl. Acad. Sci. USA 81:7683), core protein, matrix protein or other protein of Dengue virus (Genbank accession no. M19197; Hahn et al., 1988, Virology 162:167-180), measles virus hemagglutinin (Genbank accession no. M81899; Rota et al., 1992, Virology 188:135-142), herpes simplex virus type 2 glycoprotein gB (Genbank accession no. M14923; Bzik et al., 1986, Virology 155:322-333), poliovirus I VP1 (Emini et al., 1983, Nature 304:699), envelope glycoproteins of HIV I (Putney et al., 1986, Science 234:1392-1395), hepatitis B surface antigen (Itoh et al., 1986, Nature 308:19; Neurath et al., 1986, Vaccine 4:34), diptheria toxin (Audibert et al., 1981, Nature 289:543), streptococcus 24M epitope (Beachey, 1985, Adv. Exp. Med. Biol. 185:193), gonococcal pilin (Rothbard and Schoolnik, 1985, Adv. Exp. Med. Biol. 185:247), pseudorabies virus g50 (gpD), pseudorabies virus II (gpB), pseudorabies virus gIII (gpC), pseudorabies virus glycoprotein H, pseudorabies virus glycoprotein E, transmissible gastroenteritis glycoprotein 195, transmissible gastroenteritis matrix protein, swine rotavirus glycoprotein 38, swine parvovirus capsid protein, Serpulina hydodysenteriae protective antigen, bovine viral diarrhea glycoprotein 55, Newcastle disease virus hemagglutinin-neuraminidase, swine flu hemagglutinin, swine flu neuraminidase, foot and mouth disease virus, hog colera virus, swine influenza virus, African swine fever virus, Mycoplasma hyopneumoniae, infectious bovine rhinotracheitis virus (e.g., infectious bovine rhinotracheitis virus glycoprotein E or glycoprotein G), or infectious laryngotracheitis virus (e.g., infectious laryngotracheitis virus glycoprotein G or glycoprotein 1), a glycoprotein of La Crosse virus (Gonzales-Scarano et al., 1982, Virology 120:42), neonatal calf diarrhea virus (Matsuno and Inouye, 1983, Infection and Immunity 39:155), Venezuelan equine encephalomyelitis virus (Mathews and Roehrig, 1982, J. Immunol. 129:2763), punta toro virus (Dalrymple et al., 1981, in Replication of Negative Strand Viruses, Bishop and Compans (eds.), Elsevier, N.Y., p. 167), murine leukemia virus (Steeves et al., 1974, J. Virol. 14:187), mouse mammary tumor virus (Massey and Schochetman, 1981, Virology 115:20), hepatitis B virus core protein and/or hepatitis B virus surface antigen or a fragment or derivative thereof (see, e.g., U.K. Patent Publication No. GB 2034323A published Jun. 4, 1980; Ganem and Varmus, 1987, Ann. Rev. Biochem. 56:651-693; Tiollais et al., 1985, Nature 317:489-495), antigen of equine influenza virus or equine herpesvirus (e.g., equine influenza virus type A/Alaska 91 neuraminidase, equine influenza virus type A/Miami 63 neuraminidase, equine influenza virus type A/Kentucky 81 neuraminidase equine herpesvirus type 1 glycoprotein B, and equine herpesvirus type 1 glycoprotein D, antigen of bovine respiratory syncytial virus or bovine parainfluenza virus (e.g., bovine respiratory syncytial virus attachment protein (BRSV G), bovine respiratory syncytial virus fusion protein (BRSV F), bovine respiratory syncytial virus nucleocapsid protein (BRSV N), bovine parainfluenza virus type 3 fusion protein, and the bovine parainfluenza virus type 3 hemagglutinin neuraminidase), bovine viral diarrhea virus glycoprotein 48 or glycoprotein 53, RSV-viral proteins, e.g., RSV F glycoprotein, RSV G glycoprotein, influenza viral proteins, e.g., influenza virus neuraminidase, influenza virus hemagglutinin, herpes simplex viral protein, e.g., herpes simplex virus glycoprotein including for example, gB, gC, gD, and gE, HIV (GP-120, p17, GP-160, gag, po1, qp41, gp120, vif, tat, rev, nef, vpr, vpu, vpx antigens), influenza (NP, hemagluttinin (HA antigen), neuraminidase, PB1, PB2, PA, NP, M1, M₂, NS₁, NS₂)), papillomaviruses (E1, E2, E3, E4, E5a, E5b, E6, E7, E8, L1, L2), adenovirus (E1A, E1B, E2, E3, E4, E5, L1, L2, L3, L4, L5), HSV (ribonucleotide reductase, α-TIF, ICP4, ICP8, 1CP35, LAT-related proteins, gB, gC, gD, gE, gH, gI, gJ, and dD antigens), human papilloma virus, equine encephalitis virus, hepatitis (Hep B Surface Antigen (gp27s, gp36s, gp42s, p22^{c}, pol, x)).

In some embodiments, the antigenic or immunogenic protein fragment or epitope may be derived from a pathogenic virus such as, an antigen of adenovirdiae (e.g., mastadenovirus and aviadenovirus), herpesviridae (e.g., herpes simplex virus 1, herpes simplex virus 2, herpes simplex virus 5, and herpes simplex virus 6), leviviridae (e.g., levivirus, enterobacteria phase MS2, allolevirus), poxyiridae (e.g., chordopoxyirinae, parapoxvirus, avipoxvirus, capripoxvirus, leporipoxvirus, suipoxvirus, molluscipoxvirus, and entomopoxyirinae), papovaviridae (e.g., polyomavirus and papillomavirus), paramyxoviridae (e.g., paramyxovirus, parainfluenza virus 1, mobillivirus (e.g., measles virus), rubulavirus (e.g., mumps virus), pneumonovirinae (e.g., pneumovirus, human respiratory syncytial virus), metapneumovirus (e.g., avian pneumovirus and human metapneumovirus), picornaviridae (e.g., enterovirus, rhinovirus, hepatovirus (e.g., human hepatitis A virus), cardiovirus, and apthovirus), reoviridae (e.g., orthoreovirus, orbivirus, rotavirus, cypovirus, fijivirus, phytoreovirus, and oryzavirus), retroviridae (e.g., mammalian type B retroviruses, mammalian type C retroviruses, avian type C retroviruses, type D retrovirus group, BLV-HTLV retroviruses), lentivirus (e.g. human immunodeficiency virus 1 and human immunodeficiency virus 2), spumavirus, flaviviridae (e.g., hepatitis C virus), hepadnaviridae (e.g., hepatitis B virus), togaviridae (e.g., alphavirus (e.g., sindbis virus) and rubivirus (e.g., rubella virus), rhabdoviridae (e.g., vesiculovirus, lyssavirus, ephemerovirus, cytorhabdovirus, and necleorhabdovirus), arenaviridae (e.g., arenavirus, lymphocytic choriomeningitis virus, Ippy virus, and lassa virus), and coronaviridae (e.g., coronavirus and torovirus).

In some important embodiments, the antigenic or immunogenic protein fragment or epitope may be derived from a pathogenic virus such as HIV, influenza, dengue, Hepatitis A virus, Hepatitis B virus, Hepatitis C virus, human papilloma virus, Ebola, Marburg, Rabies, Hanta virus infection, West Nile virus, SARS-like Coronaviruses, Herpes simplex virus, Varicella-zoster virus, Epstein-Barr virus, Human herpesvirus 8, Alpha viruses, or St. Louis encephalitis.

### Bacterial, Viral and Parasitic Diseases

In some embodiments, the antigenic or immunogenic protein fragment or epitope may be derived from a pathogenic bacteria such as Anthrax, Chlamydia, Mycobacteria, Legioniella. Pathogenic protozoans include but are not limited to Malaria, Babesia, Schistosomiasis. Pathogenic yeast include, for example, Aspergillus and invasive Candida.

In some important embodiments, the antigenic or immunogenic protein fragment or epitope may be derived from a pathogenic bacteria such as Mycobacterium tuberculosis, Salmonella typhi, Bacillus anthracis, Yersinia perstis, Francisella tularensis, Legionella, Chlamydia, Rickettsia typhi, or Treponema pallidum.

In some embodiments, the antigenic or immunogenic protein fragment or epitope may be derived from a pathogenic fungus, including but not limited to Coccidioides immitis, Blastomyces dermatitidis, Cryptococcus neoformans, Candida albicans, and Aspergillus species.

In some embodiments, the antigenic or immunogenic protein fragment or epitope may be derived from a pathogenic protozoan, such as, for example, Plasmodium falciparum, Plasmodium vivax, Plasmodium ovale, Plasmodium malariae, Leishmania species, Trypanosome species (African and American), cryptosporidiums, isospora species, Naegleria fowleri, Acanthamoeba species, Balamuthia mandrillaris, Toxoplasma gondii, or Pneumocystis carinii.

In some important embodiments, the antigenic or immunogenic protein fragment or epitope of the infectious agent includes HIV Gag, protease, reverse transcriptase, full-length envelope protein, Vpu, Tat and Rev; influenza hemagglutinin, nucleoprotein, matrix protein 1 (M1), non-structural protein 1 (NS-1); dengue virus cross-protective antigens shared by all major serotypes of viruses (eg. Non-structural protein 1 or NS1, envelop domain III or EDIII), Hepatitis A virus capsid protein 1 (VP-1); Hepatitis B virus surface antigen (HBsAg) and core antigen (HBcAg); Hepatitis C core antigen, E1, E2, p7, NS2, NS4 and NS4 proteins; HPV E1, E2, L1, L2; Ebola and Marburg virus glycoprotein, nucleoprotein and matrix protein; Rabies glycoprotein; Hanta virus nucleoprotein, envelope glycoprotein and G1 protein; West Nile virus premembrane (prM) and envelop (E) glycoproteins; SARS-like Coronaviruses Orf3, Spike protein, Nucleocapsid and Membrane protein; Herpes simplex virus glycoprotein B and D; Varicella-zoster virus envelope glycoprotein E and B (gE, gB), immediate early protein 63 (IE63); Epstein-Barr virus gp350, gp110, nuclear antigen 1 (EBNA-1), EBNA 2 and EBNA-3C; Human herpesvirus 8 complement control protein (KCP), glycoprotein B, ORF6, ORF61, and ORF65); M. tuberculosis antigen 85A, 85B, MPT51, PPE44, mycobacterial 65-kDa heat shock protein (DNA-hsp65), 6-kDa early secretary antigenic target (ESAT-6); Salmonella SpaO and Hla, outer membrane proteins (OMPs); P.aeruginosa OMPs, PcrV, OprF, OprI, PilA and mutated ToxA; B. anthracis protective antigen (PA); Y. pestis low calcium response protein V (LcrV), F1 and F1-V fusion protein; Legionella peptidoglycan-associated lipoprotein (PAL), mip, flagella, OmpS, hsp60, major secretory protein (MSP); Chlamydia protease-like activity factor (CPAF), major outer membrane protein (MOMP); T. pallidum outer membrane lipoproteins; Coccidioides Ag2/Pra106, Prp2, phospholipase (P1b), alpha-mannosidase (Amn1), aspartyl protease, Gel1; Blastomyces dermatitidis surface adhesin WI-1; Cryptococcus neoformans GXM and its Peptide mimotopes, and mannoproteins; Candida albicans hsp90-CA, 65-kDa mannoprotein (MP65), Secretory aspartyl proteinase (Sap), Alslp-N, Als3p-N; Aspergillus Asp f 16 , Asp f 2, Der p 1, and Fel d 1, rodlet A, PEP2, Aspergillus HSP90, 90-kDa catalase; Plasmodium apical membrane antigen 1 (AMA1), 25-kDa sexual-stage protein (Pfs25), erythrocyte membrane protein 1 (PfEMP1) circumsporozoite protein (CSP), Merozoite Surface Protein-1 (MSP1 ); Leishmania cysteine proteinase type III (CPC), ribosomal proteins (LRP), A2 antigen, nucleosomal histones, HSP20, G46/M-2/PSA-2 promastigote surface protein, L infantum LACK antigen, GP63, LmSTI1, TSA, P4, NH36, papLe22; Trypanosome beta-tubulin (STIB 806), microtubule-associate protein (MAP p15), cysteine proteases (CPs); Cryptosporidiums surface proteins gp15 and gp40, Cp23 antigen, p23; Toxoplasma gondii surface antigen 1 (TgSAG1), protease inhibitor-1 (TgPI-1), surface-associated proteins MIC2, MIC3, ROP2, GRA1-GRA7; Pneumocystis carinii major surface glycoprotein (MSG), p55 antigen; Schistosomiasis mansoni Sm14, 21.7 and SmFim antigen, Tegument Protein Sm29, 26kDa GST, Schistosoma japonicum, SjCTPI, SjC23, Sj22.7, or SjGST-32.

The recombinant poxviruses administered as described herein comprise antigens to elicit an immune response in a subject. In certain embodiments, the poxviruses may additionally comprise cytokines or co-stimulatory molecules. Cytokines, e.g., IL-2, IL-6, IL-12, IL-15, or co-stimulatory molecules, e.g., B7.1, B7.2, may be used as adjuvants. In certain embodiments, either cytokines or co-stimulatory molecules can be co-administered via co-insertion of the genes encoding the molecules into the recombinant pox vector or a second recombinant poxvirus which is admixed with the recombinant poxvirus expressing the antigen. Alternatively, the cytokines can be administered separately, systemically to the host.

### Immunostimulation

In certain embodiments the recombinant poxvirus encoding the antigen fragment is additionally modified to include an immunomodulator, such as for example, DNA encoding a T-cell co-stimulatory factor and/or a cytokine such as interleukin (IL) (e.g., IL-2, IL-4, IL-10, IL-12), an interferon (IFN) (e.g., IFN-γ), granulocyte macrophage colony stimulating factor (GM-CSF) or an accessory molecule (e.g. ICAM-1). The construction of such multivalent vectors such as pox viral vectors is within the level of skill in the art based upon the present disclosure. In some cases, co-expression of the immunomodulatory agent such as the T-cell co-stimulatory factor and the antigen by multiple vectors may be desirable. It may be desirable to administer a substantially pure preparation of, e.g., the immunomodulator to boost vaccine efficacy.

In certain embodiments, nucleic acids for insertion into the poxvirus include co-stimulatory molecules, accessory molecules, and/or genes encoding a cytokine and/or growth factor. Examples of costimulatory molecules include but are not limited to B7-1, B7-2, ICAM-1, CD40, CD40L, LFA-3, CD72, OX40L (with or without OX40).

Examples of cytokines and growth factors include but are not limited to: granulocyte macrophage-colony stimulating factor (GM-CSF), granulocyte-colony stimulating factor (G-CSF), macrophage-colony stimulating factor (M-CSF), tumor necrosis factors (TNFα and TNFβ), transforming growth factors (TGFα and TGFβ), epidermal growth factors (EGF), stem cell factor (SCF), platelet-derived growth factors (PDGF), platelet-derived endothelial cell growth factor, nerve growth factor (NGF), fibroblast growth factors (FGF), insulin-like growth factors (IGF-I and IGF-II), growth hormone, interleukins 1 to 15 (IL-1 to IL-15), interferons α, β, γ (IFN-α IFN-β and IFN-y), brain-derived neurotrophic factor, neurotrophins 3 and 4, hepatocyte growth factor, erythropoictin, EGF-like mitogens, TGF-like growth factors, PDGF-like growth factors, melanocyte growth factor, mammary-derived growth factor 1, prostate growth factors, cartilage-derived growth factor, chondrocyte growth factor, bone-derived growth factor, osteosarcoma-derived growth factor, glial growth-promoting factor, colostrum basic growth factor, endothelial cell growth factor, tumor angiogenesis factor, hematopoietic stem cell growth factor, B-cell stimulating factor 2, B-cell differentiation factor, leukemia-derived growth factor, myelomonocytic growth factor, macrophage-derived growth factor, macrophage-activating factor, erythroid-potentiating activity, keratinocyte growth factor, ciliary neurotrophic growth factor, Schwann cell-derived growth factor, vaccinia virus growth factor, bombyxin, neu differentiation factor, v-Sis, glial growth factor/acetylcholine receptor-inducing activity, transferrin, bombesin and bombesin-like peptides, angiotensin II, endothelin, atrial natriuretic factor (ANF) and ANF-like peptides, vasoactive intestinal peptide, RANTES, Bradykinin and related growth factors.

In some of the preferred embodiments, the co-stimulatory molecule, growth factor, adjuvant or cytokine is IL-1, IL-2, IL-7, IL-12, IL-15, IL-18, IL-23, IL-27, B7-1, B7-2, B7-H3, LFA-3, B7-H3, CD40, CD40L, ICOS-ligand, OX-40L, 4-1BBL, GM-CSF, SCF, FGF, Flt3-ligand, CCR4, QS-7, QS-17, QS-21, CpG oligonucleotides, ST-246, AS-04, LT R192G mutant, Montanide ISA 720, heat shock proteins, synthetic mycobacterial cordfactor (CAF01), Lipid A mimetics, Salmonella enterica serovar Typhimurium flagellin (FliC), Montanide 720, Levamisole (LMS), Imiquimod, Diphtheria Toxin, IMP321, AS02A, AS01B, AS15-SB, Alhydrogel, Montanide ISA, Aluminum hydroxide, MF59, ISCOMATRIX, MLPA, MPL and other TLR-4 ligands, MDP and other TLR-2 ligands, AS02A, AS01B, Heat Liable Toxin LTK63 and LT-R192G.

In some embodiments, nucleic acids are provided that express antigenic domains rather than the entire protein. For example, if an immune reaction is desired, only the fragment necessary to stimulate the immune reaction needs to be encoded. The co-stimulatory molecules, accessory molecules, and cytokines described herein are useful as adjuvants, which can be administered systemically to the host via inserting nucleic acids encoding such into the same or different recombinant poxvirus vectors. In one embodiment, one administers a poxvirus vector containing B7, LFA-3 and ICAM-1 in conjunction with the tumor associated antigen. In a further embodiment, the poxvirus also contains OX40L. Other useful adjuvants that can be administered separately from the poxvirus are, for example, RIBI Detox (Ribi Immunochemical), QS21 (Aquila), incomplete Freund's adjuvant.

In some embodiments, poxviruses expressing B7-1, ICAM-1, and LFA-3, also known as TRICOM, are provided that induce activation of both CD4⁺ and CD8⁺ T cells. (U.S. Pat. No. 6,045,802; Hodge et al., J. Natl. Cancer Inst. 92: 1228-39 (2000); Hodge et al., Cancer Research 59: 5800-07 (1999)). OX40 is a primary co-stimulator of T cells that have encountered antigen, rather than naive T cells, and promotes T-cell expansion after T cell tolerance is induced. (Bansal-Pakal et al., Nature Med. 7: 907-12 (2001)). OX40L plays a role during T cell activation by a) sustaining the long-term proliferation of CD4⁺ and CD8⁺ T cells, b) enhancing the production of Th1 cytokines such as IL-2, IGN-γ, and TNF-α from both CD4⁺ and CD8⁺ T cells without changing IL-4 expression, c) protecting T cells from apoptosis. In certain embodiments, the combination of B7-1, ICAM-1, LFA-3, and OX40L enhances initial activation and then further potentiates sustained activation of naive and effector T cells.

The poxviruses described herein are non-replicating in the target cell. As a result of this arid the optional non-integrative cytoplasmic nature of the poxvirus vector, the vector system will not result in replication and infection of other cells. Thus, the poxvirus-infected cells will not adversely affect cells in the host at locations distant from where the target cell is.

In some embodiments, the modified poxvirus may also have altered characteristics concerning aspects of the viral life cycle, such as target cell specificity, route of infection, rate of infection, rate of replication, rate of virion assembly and/or rate of viral spreading.

In some embodiments, the poxviruses described herein are capable of infecting host cells in a host. The host cells are any cell amenable to infection by the poxvirus and capable of expressing the poxvirus genome; including any foreign genes inserted therein, e.g. encoding an antigen, at levels sufficient to elicit a host immune response to the antigen.

The poxviruses described herein can be used for any host. In some embodiments, the host is a non-mammalian host, such as birds, fish and the like. In some embodiments, the host is a mammal. Mammals include primates such as humans and chimpanzees, domestic animals such as horses, cows, pigs, etc. and pets such as dogs and cats, as well as rodents, such as mice, rats, and hamsters.

### Culturing of Viral Vector in Host Cells

Introduction of the viral vector carrying the gene to be delivered to the target host cell may be effected by any method known to those of skill in the art.

Most viral vaccines such as attenuated or recombinant viruses are manufactured from cell culture systems. The cells used for virus/vaccine production may be cell lines, i.e. cells that grow continuously in vitro, either as single-cell suspension culture in bioreactors or as a monolayer on a cell-support surface of tissue culture flasks or roller-bottles. Not only cell lines but also primary animal cells may be used for the manufacture of vaccines. For example, chordopoxvirinae, in particular MVA are amplified in cell cultures of primary or secondary chicken embryo fibroblasts (CEF). The cells are obtained from embryos of chicken eggs that are incubated for 10 to 12 days. The cells of the embryos are then dissociated and purified. These primary CEF cells can either be used directly or after one further cell passage as secondary CEF cells. Subsequently, the primary or secondary CEF cells are infected with the MVA. For the amplification of MVA the infected cells are incubated for 2-3 days at 37°C. (see, e.g., Meyer, H. et al. 1991; J. of General Virology 72, 1031-1038; Sutter et al. 1994, Vaccine, Vol. 12, No. 11, 1032-1040). CEF cells are often used since many virus vaccines are made by attenuating the virulent disease-causing virus by serially passaging in CEF cells. Attenuated viruses, such as MVA are preferably not propagated on human cells since there is a concern that the viruses might become replication competent in cells of human origin. Viruses that have regained the ability to replicate in human cells represent a health risk if administered to humans, in particular if the individuals are immune compromised. For this reason, some attenuated viruses, such as MVA, are strictly manufactured from CEF cells, if intended for human use. Moreover, CEF cells are used for those viruses that grow only in these cells, for example avian viruses such as avipox viruses, canary pox virus, ALVAC, Fowl pox virus and NYVAC.

In certain embodiments, host cells, such as epidermal epithelial cells, fibroblasts, or dendritic cells, infected with the recombinant viruses express the antigeri(s) and mary additionally express the immunostimulatory molecule(s). In these embodiments, the antigen may be expressed at the cell surface of the infected host cell. The immunostimulatory molecule may be expressed at the cell surface or may be actively secreted by the host cell.

The expression of both the antigen and the immunostimulatory molecule may provide the necessary MHC restricted peptide to specific immunosurveilling T cells and the appropriate signal to the T cell in the skin to aid in antigen recognition and proliferation or clonal expansion of antigen specific T cells. The overall result may be an upregulation of the immune system. In certain embodiments the upregulation of the immune response is an increase in antigen specific T-helper lymphocytes and/or cytotoxic lymphocytes, including for example, Th1 or Th2 CD4⁺ T-helper cell-mediated or CD8⁺ cytotoxic T-lymphocytes, which are able to kill or inhibit the growth of a disease causing agent (such as a cancer cell) or a cell infected with a disease causing agent (such as a cell infected with a virus, a bacteria, a fungus, or a protozoa. In certain embodiments, the immune stimulation may also involve an antibody response comprising generations of one or more antibody classes, such as IgM, IgG, and/or IgA.

### Methods for Determining Immune Responses

Methods for determining immune responses are known in the art. Viral lesions can be examined to determine the occurrence of an immune response to the virus and/or the antigen. In vitro assays may be used to determine the occurrence of an immune response. Examples of such in vitro assays include ELISA assays and cytotoxic T cell (CTL) assays. The immune response can be measured by detecting and/or quantifying the relative amount of an antibody, which specifically recognizes an antigen in the sera of a subject who has been treated by administering the live, modified, non-replicating or replication-impaired poxvirus comprising the antigen, relative to the amount of the antibody in an untreated subject.

Techniques for the assaying antibodies and antibody filters in a sample are known in the art and include, for example, sandwich assays, ELISA and ELISpot. Antibodies include parts of antibodies, mammalianized (e. g. humanized) antibodies, recombinant or synthetic antibodies and hybrid and single chain antibodies.

Both polyclonal and monoclonal antibodies are obtainable by immunization with the immune effectors or antigenic fragments thereof and either type is utilizable for immunoassays. The methods of obtaining both types of sera are well known in the art.

Polyclonal sera are relatively easily prepared by injection of a suitable laboratory animal with an effective amount of the immune effector, or antigenic part thereof, collecting serum from the animal and isolating specific sera by any of the known immunoadsorbent techniques. Antibodies produced by this method are utilizable in virtually any type of immunoassay.

The use of monoclonal antibodies in an immunoassay is preferred because of the ability to produce them in large quantities and the homogeneity of the product. The preparation of hybridoma cell lines for monoclonal antibody production derived by fusing an immortal cell line and lymphocytes sensitized against the immunogenic preparation can be achieved by techniques which are well known to those who are skilled in the art.

ELISA assays may be used to determine the level of isotype specific antibodies using methods known in the art. CTL assays can be used to determine the lytic activity of CTLs, measuring specific lysis of target cells expressing a certain antigen.

Immune-assays may be used to measure the activation (e.g., degree of activation) of sample immune cells. Sample immune cells refer to immune cells contained in samples from any source, including from a human patient, human donor, animal, or tissue cultured cell line. The immune cell sample can be derived from peripheral blood, lymph nodes, bone marrow, thymus, any other tissue source including in situ or excised tumor, or from tissue or organ cultures. The sample may be fractionated or purified to generate or enrich a particular immune cell subset before analysis. The immune cells can be separated and isolated from their source by standard techniques.

Immune cells include both non-resting and resting cells, and cells of the immune system that may be assayed, including, but not limited to, B lymphocytes, T lymphocytes, natural killer (NK) cells, lymphokine-activated killer (LAK) cells, monocytes, macrophages, neutrophils, granulocytes, mast cells, platelets, Langerhans cells, stem cells, dendritic cells, and peripheral blood mononuclear cells.

Immune cell activity that may be measured include, but is not limited to (1) cell proliferation by measuring the cell or DNA replication; (2) enhanced cytokine production, including specific measurements for cytokines, such as γIFN, GM-CSF, or TNF-alpha, IFN-alpha, IL-6, IL-10, IL-12; (3) cell mediated target killing or lysis; (4) cell differentiation; (5) immunoglobulin production; (6) phenotypic changes; (7) production of chemotactic factors or chemotaxis, meaning the ability to respond to a chemotactin with chemotaxis; (8) immunosuppression, by inhibition of the activity of some other immune cell type; (9) chemokine secretion such as IP-10; (10) expression of costimulatory molecules (e.g., CD80, CD 86) and maturation molecules (e.g., CD83), (12) upregulation of class II MHC expression; and (13) apoptosis, which refers to fragmentation of activated immune cells under certain circumstances, as an indication of abnormal activation.

Reporter molecules may be used for many of the immune assays described. A reporter molecule is a molecule which, by its chemical nature, provides an analytically identifiable signal which allows the detection of antigen-bound antibody. Detection may be either qualitative or quantitative. The most commonly used reporter molecules in this type of assay are either enzymes, fluorophores or radionuclide containing molecules (i. e. radioisotopes) and chemiluminescent molecules. In the case of an enzyme immunoassay, an enzyme is conjugated to the second antibody, generally by means of glutaraldehyde or periodate. As will be readily recognized, however, a wide variety of different conjugation techniques exist, which are readily available to the skilled artisan. Commonly used enzymes include horseradish peroxidase, glucose oxidase, beta- galactosidase and alkaline phosphatase, amongst others. The substrates to be used with the specific enzymes are generally chosen for the production, upon hydrolysis by the corresponding enzyme, of a detectable colour change. Examples of suitable enzymes include alkaline phosphatase and peroxidase. It is also possible to employ fluorogenic substrates, which yield a fluorescent product rather than the chromogenic substrates noted above. In all cases, the enzyme-labeled antibody is added to the first antibody-antigen complex, allowed to bind, and then the excess reagent is washed away. A solution containing the appropriate substrate is then added to the complex of antibody-antigen- antibody. The substrate will react with the enzyme linked to the second antibody, giving a qualitative visual signal, which may be further quantitated, usually spectrophotometrically, to give an indication of the amount of antigen which was present in the sample. Alternately, fluorescent compounds, such as fluorescein and rhodamine, may be chemically coupled to antibodies without altering their binding capacity. When activated by illumination with light of a particular wavelength, the fluorochrome-labeled antibody adsorbs the light energy, inducing a state to excitability in the molecule, followed by emission of the light at a characteristic colour visually detectable with a light microscope. The fluorescent labeled antibody is allowed to bind to the first antibody-antigen complex. After washing off the unbound reagent, the remaining tertiary complex is then exposed to the light of the appropriate wavelength the fluorescence observed indicates the presence of the antigen of interest.

Examples of some common immune assays are:
Cell Proliferation Assay: Activated immune cell proliferation is intended to include increase in cell number, cell growth, cell division, or cell expansion, as measured by cell number, cell weight, or by incorporation of radiolabelled nucleic acids, amino acids, proteins, or other precursor molecules. As one example, DNA replication is measured by incorporation of radioisotope labels. In some embodiments, cultures of stimulated immune cells can be measured by DNA synthesis by pulse-labeling the cultures with tritiated thymidine (³H-Tdr), a nucleoside precursor that is incorporated into newly synthesized DNA. Thymidine incorporation provides a quantitative measure of the rate of DNA synthesis, which is usually directly proportional to the rate of cell division. The amount of ³H-labeled thymidine incorporated into the replicating DNA of cultured cells is determined by scintillation counting in a liquid scintillation spectrophotometer. Scintillation counting yields data in counts per minute (cpm) which may then be used as a standard measure of immune cell responsiveness. The cpm in resting immune cell cultures may be either subtracted from or divided into cpm of the primed immune cells, which will yield a stimulation index ratio.

Flow cytometry can also be used to measure proliferation by measuring DNA with light scatter, Coulter volume and fluorescence, all of which are techniques that are well known in the art.

Enhanced Cytokine Production Assay: A measure of immune cell stimulation is the ability of the cells to secrete cytokines, lymphokines, or other growth factors. Cytokine production, including specific measurements for cytokines, such as γIFN, GM-CSF, or TNF-alpha, may be made by radioimmunoassay (RIA), enzyme-linked immunoabsorbent assay (ELISA), bioassay, or measurement of messenger RNA levels. In general, with these immunoassays, a monoclonal antibody to the cytokine to be measured is used to specifically bind to and thus identify the cytokine. Immunoassays are well known in the art and can include both competitive assays and immunometric assays, such as forward sandwich immunoassays, reverse sandwich immunoassays and simultaneous immunoassays.

In each of the above assays, the sample-containing cytokine is incubated with the cytokine-specific monoclonal antibody under conditions and for a period of time sufficient to allow the cytokines to bind to the monoclonal antibodies. In general, it is desirable to provide incubation conditions sufficient to bind as much cytokine and antibody as possible, since this will maximize the signal. Of course, the specific concentrations of antibodies, the temperature and time of incubation, as well as other such assay conditions, can be varied, depending upon various factors including the concentration of cytokine in the sample, the nature of the sample, and the like. Those skilled in the art will be able to determine operative and optimal assay conditions for each determination by employing routine experimentation.

Cell-Mediated Target Cell Lysis Assay: Another type of indicator for degree of immune cell activation is immune cell-mediated target cell lysis, which is meant to encompass any type of cell killing, including cytotoxic T lymphocyte activity, apoptosis, and the induction of target lysis by molecules secreted from non-resting immune cells stimulated to activity. Cell-mediated lympholysis techniques typically measure the ability of the stimulated immune cells to lyse ⁵¹Cr-labeled target cells. Cytotoxicity is measured as a percentage of ⁵¹Cr released in specific target cells compared to percentage of ⁵¹Cr released from control target cells. Cell killing may also be measured by counting the number of target cells, or by quantifying an inhibition of target cell growth.

Cell Differentiation Assay: Another indicator of immune cell activity is immune cell differentiation and maturation. Cell differentiation may be assessed in several different ways. One such method is by measuring cell phenotypes. The phenotypes of immune cells and any phenotypic changes can be evaluated by flow cytometry after immunofluorescent staining using monoclonal antibodies that will bind membrane proteins characteristic of various immune cell types.

A second means of assessing cell differentiation is by measuring cell function. This may be done biochemically, by measuring the expression of enzymes, mRNA's, genes, proteins, or other metabolites within the cell, or secreted from the cell. Bioassays may also be used to measure functional cell differentiation.

Immune cells express a variety of cell surface molecules which can be detected with either monoclonal antibodies or polyclonal antisera. Immune cells that have undergone differentiation or activation can also be enumerated by staining for the presence of characteristic cell surface proteins by direct immunofluorescence in fixed smears of cultured cells.

Mature B cells can be measured in immunoassays, for example, by cell surface antigens including CD 19 and CD20 with monoclonal antibodies labeled with fluorochromes or enzymes may be used to these antigens. B cells that have differentiated into plasma cells can be enumerated by staining for intracellular immunoglobulins by direct immunofluorescence in fixed smears of cultured cells.

Immunoglobulin Production Assay: B cell activation results in small, but detectable, quantities of polyclonal immunoglobulins. Following several days of culture, these immunoglobulins may be measured by radioimmunoassay or by enzyme-linked immunosorbent assay (ELISA) methods.

B cells that produce immunoglobulins can also be quantified by the reversed hemolytic plaque assay. In this assay, erythrocytes are coated with goat or rabbit antihuman immunoglobulins. These immunoglobulins are mixed with the activated immunoglobulin-producing lymphocytes and semisolid agar, and complement is added. The presence of hemolytic plaques indicates that there are immunoglobulin-producing cells.

Chemotactic Factor Assay: Chemotactic factors are molecules which induce or inhibit immune cell migration into or out of blood vessels, tissues or organs, including cell migration factors. The chemotactic factors of immune cells can be assayed by flow cytometry using labeled monoclonal antibodies to the chemotactic factor or factors being assayed. Chemotactic factors may also be assayed by ELISA or other immunoassays, bioassays, messenger RNA levels, and by direct measurements, such as cell counting, of immune cell movements in specialized migration chambers.

Addback Assays: When added to fresh peripheral blood mononuclear cells, autologous ex vivo activated cells exhibit an enhanced response to a "recall" antigen, which is an antigen to which the peripheral blood mononuclear cells had previously been exposed. Primed or stimulated immune cells should enhance other immune cells response to a "recall" antigen when cultured together. These assays are termed "helper" or "addback" assays. In this assay, primed or stimulated immune cells are added to untreated, usually autologous immune cells to determine the response of the untreated cells. The added primed cells may be irradiated to prevent their proliferation, simplifying the measurement of the activity of the untreated cells. These assays may be particularly useful in evaluating cells for blood exposed to virus. The addback assays can measure proliferation, cytokine production, and target cell lysis as described herein.

The above-described methods and other additional methods to determine an immune response are well known in the art.

### Administration of Viral Vectors to the Skin

The skin represents an attractive target site for delivery of antigens due to the high concentration of antigen presenting cells (APC), APC precursors and immune cells found within this tissue, resulting in an enhanced protective immune response. By replicating the natural infectious process of keratinocytes by vaccinia virus, which provides uniquely strong immune responses to vaccinia genes, the methods described herein similarly enhance the immunity of a subject to heterologous antigens expressed by the poxviruses provided herein, such as other viruses, bacteria, fungi and cancer antigens. The resulting immune response may protect the immunized subject or may help to alleviate or treat the disease caused by the antigen-presenting agent if the subject has already developed the disease.

In some embodiments, the recombinant poxviruses comprising antigen described herein may be administered in form of a composition, which may further comprise one or more other pharmaceutically acceptable carriers, including any suitable diluent or excipient. Preferably, the pharmaceutically acceptable carrier does not itself induce a physiological response, e.g., an immune response. Most preferably, the pharmaceutically acceptable carrier does not result in any adverse or undesired side effects and/or does not result in undue toxicity. Pharmaceutically acceptable carriers include, but are not limited to, saline, buffered saline, dextrose, water, glycerol, sterile isotonic aqueous buffer, and combinations thereof. Additional examples of pharmaceutically acceptable carriers, diluents, and excipients are provided in Remington's Pharmaceutical Sciences (Mack Pub. Co., N.J., current edition).

Modified poxviruses are preferably administered by mechanical disruption of the epidermis (e.g., by skin scarification, scratching, abrading or superficial cutting). Methods and devices for disrupting the skin and for depositing a substance into the epidermis of the skin are known in the art. Examples of devices for disrupting the skin include a scarification needle, a hypodermic needle, or an abrader.

The poxviruses of the invention and compositions thereof may be delivered into the epidermal compartment of skin in any pharmaceutically acceptable form. In some embodiments, the poxviruses and compositions thereof are applied to the skin and a device that mechanically disrupts the epidermis (e.g., abrader) is then moved or rubbed over the skin and the poxvirus. In certain embodiments, scarification needles or hypodermic needles may be used to disrupt the epidermis. It is preferred that the minimum amount of abrasion/mechanical disruption to produce the desired result be used. Determination of the appropriate amount of abrasion/mechanical disruption for a selected poxvirus and/or composition thereof is within the ordinary skill in the art. In another embodiment the poxvirus and/or composition thereof may be applied in dry form to the abrading surface of the abrading device prior to application. In this embodiment, a reconstituting liquid is applied to the skin at the delivery site and the poxvirus (composition)-coated abrading device is applied to the skin at the site of the reconstituting liquid. It is then moved or rubbed over the skin so that the poxvirus (composition) becomes dissolved in the reconstituting liquid on the surface of the skin and is delivered simultaneously with abrasion. Alternatively, a reconstituting liquid may be contained in the device (e.g., a scarification needle, a hypodermic needle, or an abrader) and released to dissolve the poxvirus (composition) as the device is applied to the skin for mechanical disruption of the epidermis. Certain poxvirus(es) (compositions) may also be coated on the device (e.g., abarding device) in the form of a gel.

Devices (e.g., a scarification needle, a hypodermic needle, or an abrader) for accurately targeting the epidermal space may be provided. These devices may have solid or hollow microprotrusions. The microprotrusions can have a length up to about 1500 microns. The microprotrusions may have a length of about 200 to 1500 microns. The microprotrusions may have a length of about 300 to 1000 microns, or in the range of about 400 to 800 microns.

The devices (e.g., a scarification needle, a hypodermic needle, or an abrader) that may be used in the methods described herein are preferably a device capable of disrupting the skin, to penetrate the epidermis without penetrating the dermis. In some embodiments, the device penetrates the stratum corneum without penetrating the entire epidermis.

In certain embodiments, the poxviruses and compositions to be administered using the methods described herein may be applied to the skin prior to abrading, simultaneous with abrading, or post-abrading.

Methods for delivering the poxviruses and compositions thereof into the epidermis of a subject are disclosed, comprising the steps of coating a patient's outer skin layer or a device (e.g., an abrader, or a scarification needle or hypodermic needle) with the poxviruses and compositions thereof and moving the device across the subject's skin to provide mechanical disruptions leaving furrows sufficient to permit entry of the poxviruses and compositions thereof into the subject's viable epidermis.

In order to achieve the desired mechanical disruptions of the epidermis, the device (e.g., an abrader, or a scarification needle or hypodermic needle) should be moved across a subject's skin at least once. The subject's skin may be disrupted in alternating directions. The device enables the poxviruses and/or compositions thereof to be absorbed more effectively thereby allowing less of the poxviruses and compositions thereof to be applied to a subject's skin or coating the device. The surface of the device may be coated with the poxviruses and/or compositions thereof desired to be delivered to the subject. In some embodiments, the poxviruses and/or compositions thereof may be a powder disposed on the abrading surface of the device. The poxviruses and/or compositions thereof to be delivered may be applied directly to the subject's skin prior to the application and movement of the device on the subject's skin.

The device (e.g., scarification needle, hypodermic needle or abrader) and the microprotrusions can be made from a plastic material that is non-reactive with the substance being administered. Suitable plastic materials include, for example, polyethylene, polypropylene, polyamides, polystyrenes, polyesters, and polycarbonates as known in the art. Alternatively, the microprotrusions can be made from a metal such as stainless steel, tungsten steel, alloys of nickel, molybdenum, chromium, cobalt, titanium, and alloys thereof, or other materials such as silicon, ceramics and glass polymers. Metal microprotrusions can be manufactured using various techniques similar to photolithographic etching of a silicon wafer or micromachining using a diamond tipped mill as known in the art. The microprotrusioris can also be manufactured by photolithographic etching of a silicon wafer using standard techniques as are known in the art. They can also be manufactured in plastic via an injection molding process, as described for example in U.S. application Ser. No. 10/193,317.

The length and thickness of the microprotrusions are selected based on the particular substance being administered and the thickness of the epidermis in the location where the device is to be applied. The microprotrusions penetrate the epidermis without piercing or passing through the entire dermis. The protrusions penetrate the stratum corneum substantially without piercing or passing through the entire epidermis.

Methods of preparing a delivery site on the skin include placing the device (e.g., microabrader, scarification needle, or hypodermic needle) against the skin of the patient in the desired location. The device is gently pressed against the skin and then moved over or across the skin. The length of the stroke of the device can vary depending on the desired size of the delivery site, defined by the delivery area desired. The dimensions of the delivery site are selected to accomplish the intended result and can vary depending on the substance, and the form of the substance, being delivered. In some embodiments, the device is moved about 2 to 15 centimeters (cm). In some embodiments, the device is moved to produce a mechanically disrupted epidermal site having a surface area of about 4 cm² to about 300 cm².

The device can then be lifted from the skin to expose the mechanically disrupted epidermal area and the recombinant poxviruses and compositions thereof may be applied to the mechanically disrupted epidermal area. In certain embodiments, the poxviruses and/or compositions thereof to be administered may be applied to the surface of the skin either before or simultaneously with the mechanical disruption of the epidermis.

The extent of the mechanical disruption of the epidermis is dependent on the pressure applied during movement and the number of repetitions with the device (e.g., scarification needle, hypodermic needle, or abrader). The device can be lifted from the skin after making the first pass and placed back onto the starting position in substantially the same place and position. The device is then moved a second time in the same direction and for the same distance. The device can be moved repetitively across the same site in alternating direction without being lifted from the skin after making the first pass. Generally, two or more passes are made with the device. The device can be swiped back and forth, in the same direction only, in a grid-like pattern, a circular pattern, or in some other pattern for a time sufficient to disrupt the epidermis to a suitable depth to enhance the delivery of the poxviruses and/or compositions thereof.

Any device known in the art for disruption of the epidermis by mechanical disruption can be used in the methods described herein. These include for example, microelectromechanical (MEMS) devices with arrays of short microneedles or microprotrusions, sandpaper-like devices, scrapers, scarification needles, hypodermic needles and the like.

In some embodiments, an immune response to the antigen can be generated by administering between about 100-fold to about a 100-fold less pfu (plaque forming units) of the poxvirus, constructed as discussed herein to a subject; when applied by mechanical disruption of the epidermis compared to conventional injection routes. In certain embodiments, a specific immune response to the antigen can be generated by administering between about 90-fold, 80-fold, 70-fold, 60-fold, 50-fold, 40-fold, 30-fold, 20-fold, 10-fold, 5-fold less pfu of the poxvirus when applied by mechanical disruption of the epidermis compared to conventional injection routes. In some embodiments a single deposition of recombinant poxvirus is required to elicit a long-lasting, potent antigen-specific immune response in the subject.

In some embodiments, the poxviruses and/or compositions thereof provided herein may also be administered on a dosage schedule, for example, an initial administration of a poxvirus and/or compositions thereof with subsequent booster administrations. In particular embodiments, a second dose of the poxvirus and/or compositions thereof is administered anywhere from two weeks to one year, preferably from one to six months, after the initial administration. Additionally, a third dose may be administered after the second dose and from three months to two years, or even longer, preferably 4 to 6 months, or 6 months to one year after the initial administration. The boosting antigen may be administered using the same poxvirus, or as a whole protein, an immunogenic peptide fraction of the protein, another recombinant viral vector, or DNA encoding the protein or peptide. In some embodiments, different poxviruses are used. For example, vaccinia may be followed by an avipox such as fowlpox, or vice versa. In some preferred embodiments, no booster immunization is required.

### Viral Vector Kits for Administration

The invention also contemplates the use of kits. In one aspect of the invention, a pharmaceutical pack or kit comprising the poxvirus is provided. In one embodiment a kit is provided comprising, one or more containers filled with one or more of the following components: a live, modified, non-replicating poxvirus as defined in the claims comprising an antigen and optionally comprising a co-stimulatory molecule, either in dried form (e.g. lyophilized), as a salt, or in a solution, optionally a second virus comprising a co-stimulatory molecule, either in dried form (e.g. lyophilized), as a salt, or in a solution, optionally a solution or gel to dissolve or admix the virus(es), and optionally an adjuvant. The kits additionally contain a device for disrupting the epidermis. Associated with such a kit can be instructions on how to use the kit and optionally a notice in the form prescribed by a governmental agency regulating the manufacture, use or sale of pharmaceuticals or biological products, which notice reflects approval by the agency of manufacture, use or sale for human administration.

Also disclosed are methods of treatment of diseases. The method involves stimulating an immune response to an antigen in a subject. The method comprises administering to a subject in need thereof a live, modified, non-replicating or replication-impaired poxvirus comprising the antigen in an amount sufficient to stimulate the immune response in the subject, wherein the poxvirus is administered to a mechanically disrupted epidermis of the subject and wherein stimulating the immune response treats a disease in the subject caused by the antigen. The subject may have been challenged with the antigen prior to administering the poxvirus comprising the antigen.

A method for protecting a subject at risk of being challenged with an antigen or of developing a disease caused by an antigen is disclosed. The method comprises stimulating an immune response to an antigen in a subject to comprising administering to a subject in need thereof a live, modified, non-replicating or replication-impaired poxvirus comprising the antigen in an amount sufficient to stimulate the immune response, wherein the poxvirus is administered to a mechanically disrupted skin wherein stimulating the immune response confers protection of the subject against a disease caused by the antigen. The subject may not have been challenged with the antigen prior to administering the poxvirus comprising the antigen.

"To treat" or "treatment" of a disease as used herein refers to improving the condition of a subject having the disease. This may include partial or complete improvement.

In the case of cancer, "treating" the cancer refers to completely or partially inhibiting proliferation or metastasis of a cancer or tumor cell, as well as inhibiting any increase in the proliferation or metastasis of a cancer or tumor cell. Treatment may lead to stasis, partial or complete remission of a tumor or may inhibit metastatic spreading of the tumor.

In the case of an infectious disease, "treating" the infectious disease means completely or partially reducing the load of the infections agent in the subject. The load may be viral load, and reducing the viral load means, for example, reducing the number of cells infected with the virus, reducing the rate of replication of the virus, reducing the number of new virions produced, reducing the number of total viral genome copies in a cell, as compared to an untreated subject. The load may be bacterial, yeast, fungi, protozoa, helminths, or parasite load, and reducing such load means, for example, reducing the number of bacteria, fungi, protozoa, helminths, yeast or parasites in a host, reducing the rate of population growth, reducing the spread throughout the subject's body, reducing the amount of toxic products produced by the bacteria, yeast, fungi, protozoa, helminths or parasites, as compared to an untreated subject.

"To protect" or "protection of" a subject from developing a disease or from becoming susceptible to an infection as referred herein means to partially or fully protect a subject. As used herein, to "fully protect" means that a treated subject may not develop a disease or infection caused by an agent such as a virus, bacterium, fungus, protozoa, helminth, and parasites, or caused by a cancer cell. To "partially protect" as used herein means that a certain subset of subjects may be fully protected from developing a disease or infection after treatment, or that the subject may not develop a disease or infection with the same severity as an untreated subject.

Provided herein is a poxvirus to treat and/or prevent an infection (or infectious disease) in a subject preferably a human. The recombinant poxvirus comprising antigen and/or compositions thereof is administered to a mechanically disrupted epidermis of the subject. Infections or infectious diseases that can be treated or prevented by these methods are caused by infectious agents including, but not limited tobacteria, viruses, fungi, protozoa, helminths, and parasites.

Examples of viruses that can be treated by the methods described herein, or for which the methods described herein confer protection, include, but are not limited to, HIV, influenza, dengue, Hepatitis A virus, Hepatitis B virus, Hepatitis C virus, Human papilloma virus, Ebola, Marburg, Rabies, Hanta virus infection, West Nile virus, SARS-like Coronaviruses, Herpes simplex virus (HSV1 and HSV2), Varicella-zoster virus, Epstein-Barr virus, Human herpesvirus 8, Alpha viruses, St. Louis encephalitis.

Other viruses that may be treated or for which the methods described herein confer protection include, but are not limited to: enteroviruses (including, but not limited to, viruses that the family picornaviridae, such as polio virus, Coxsackie virus, echo virus), rotaviruses, adenovirus, and hepatitis virus, such as hepatitis A, B, C D and E. Specific examples of viruses that have been found in humans include but are not limited to: Retroviridae (e.g., human immunodeficiency viruses, such as HIV-1 (also referred to as HTLV-III, LAV or HTLV-III/LAV, or HIV-III; and other isolates, such as HIV-LP; Picornaviridae (e.g., polio viruses, hepatitis A virus; enteroviruses, human Coxsackie viruses, rhinoviruses, echoviruses); Calciviridae (e.g., strains that cause gastroenteritis); Togaviridae (e.g., equine encephalitis viruses, rubella viruses); Flaviviridae (e.g., encephalitis viruses, yellow fever viruses); Coronaviridae (e.g., coronaviruses); Rhabdoviridae (e.g., vesicular stomatitis viruses, rabies viruses); Filoviridae (e.g., ebola viruses); Paramyxoviridae (e.g., parainfluenza viruses, mumps virus, measles virus, respiratory syncytial virus); Orthomyxoviridae (e.g., influenza viruses); Bunyaviridae (e.g., bunya viruses, phleboviruses and Nairo viruses); Arenaviridae (hemorrhagic fever viruses); Reoviridae (e.g., reoviruses, orbiviurses and rotaviruses); Birnaviridae; Hepadnaviridae (Hepatitis B virus); Parvoviridae (parvoviruses); Papovaviridae (papillomaviruses, polyoma viruses); Adenoviridae (most adenoviruses); cytomegalovirus (CMV); Poxviridae (variola viruses, vaccinia viruses, pox viruses); Iridoviridae (e.g., African swine fever virus); and other viruses acute laryngotracheobronchitis virus, Alphavirus, Kaposi's sarcoma-associated herpesvirus, Newcastle disease virus, Nipah virus, Norwalk virus, Papillomavirus, parainfluenza virus, and avian influenza.

Bacterial infections or diseases that can be treated or prevented by the poxviruses and methods described herein are caused by bacteria including, but not limited to, Mycobacterium tuberculosis, Salmonella typhi, Bacillus anthracis, Yersinia perstis, Francisella tularensis, Legionella, Chlamydia, Rickettsia typhi, and Treponema pallidum.

Other bacteria that may be treated or for which the methods described herein confer protection include, but are not limited to: Pasteurella species, Staphylococci species, and Streptococcus species. Gram negative bacteria include, but are not limited to, Escherichia coli, Pseudomonas species, and Salmonella species. Specific examples of infectious bacteria include but are not limited to, Helicobacter pyloris, Borelia burgdorferi, Mycobacteria sps (e.g. M. avium, M. intracellulare, M. kansaii, M. gordonae), Staphylococcus aureus, Neisseria gonorrhoeae, Neisseria meningitidis, Listeria monocytogenes, Streptococcus pyogenes (Group A Streptococcus), Streptococcus agalactiae (Group B Streptococcus), Streptococcus (viridans group), Streptococcus faecalis, Streptococcus bovis, Streptococcus (anaerobic sps.), Streptococcus pneumoniae, pathogenic Campylobacter sp., Enterococcus sp., Haemophilus influenzae, corynebacterium diphtheriae, corynebacterium sp., Erysipelothrix rhusiopathiae, Clostridium perfringers, Clostridium tetani, Enterobacter aerogenes, Klebsiella pneumoniae, Pasturella multocida, Bacteroides sp., Fusobacterium nucleatum, Streptobacillus moniliformis, Treponema pertenue, Leptospira, and Actinomyces israelli.

Fungal diseases that can be treated or prevented using the poxviruses and methods described herein include, but are not limited to, Coccidioides immitis, Blastomyces dermatitidis, Cryptococcus neoformans, Candida albicans, Aspergillus species.

Other fungi that may be treated or for which the methods described herein confer protection include, but are not limited to: Histoplasma capsulatum, Coccidioides immitis, and Chlamydia trachomatis.

Protozoal diseases or infections that can be treated or prevented using the poxviruses and methods described herein include, but are not limited to, Malaria (Plasmodium falciparum, Plasmodium vivax, Plasmodium ovale, Plasmodium malariae), Leishmania species, Trypanosome species (African and American), cryptosporidiums, isospora species, Naegleria fowleri, Acanthamoeba species, Balamuthia mandrillaris, Toxoplasma gondii, and Pneumocystis carinii.

Cancers or tumors which may be treated or prevented using the poxviruses provided herein include, but are not limited to, melanoma, cutaneous squamous cell carcinoma, basal cell carcinoma, breast cancer, prostate adenocarcinoma, prostatic intraepithelial neoplasia, squamous cell lung carcinoma, lung adenocarcinoma, small cell lung carcinoma, ovary cancer of epithelial origin, colorectal adenocarcinoma and leiomyosarcoma, stomach adenocarcinoma and leiomyosarcoma, hepatocellular carcinoma, cholangiocarcinoma, ductal adenocarcinomas of pancreas, endocrine pancreatic tumors, renal cell carcinoma, transitional cell carcinoma of kidney and bladder, bladder squamous cell carcinoma, papillary thyroid cancer, follicular thyroid cancer, brain cancers (astrocytoma, glioblastoma multiforme).

### Additional Agents to Administer with Viral Vector

The methods for treating or preventing a disease described herein may comprise administering additional agents. Thus, the method(s) for treating or preventing cancer described herein may be used in combination with one or more anti-cancer agents. Examples of anti-cancer drugs that may be used in the various embodiments, including pharmaceutical compositions and dosage forms and kits described herein, include, but are not limited.to: acivicin; aclarubicin; acodazole hydrochloride; acronine; adozelesin; aldesleukin; altretamine; ambomycin; ametantrone acetate; aminoglutethimide; amsacrine; anastrozole; anthramycin; asparaginase; asperlin; azacitidine; azetepa; azotomycin; batimastat; benzodepa; bicalutamide; bisantrene hydrochloride; bisnafide dimesylate; bizelesin; bleomycin sulfate; brequinar sodium; bropirimine; busulfan; cactinomycin; calusterone; capsitabine; caracemide; carbetimer; carboplatin; carmustine; carubicin hydrochloride; carzelesin; cedefingol; chlorambucil; cirolemycin; cisplatin; cladribine; crisnatol mesylate; cyclophosphamide; cytarabine; dacarbazine; dactinomycin; daunorubicin hydrochloride; decitabine; dexormaplatin; dezaguanine; dezaguanine mesylate; diaziquone; docetaxel; doxorubicin; doxorubicin hydrochloride; droloxifene; droloxifene citrate; dromostanolone propionate; duazomycin; edatrexate; eflornithine hydrochloride; elsamitrucin; enloplatin; enpromate; epipropidine; epirubicin hydrochloride; erbulozole; esorubicin hydrochloride; estramustine; estramustine phosphate sodium; etanidazole; etoposide; etoposide phosphate; etoprine; fadrozole hydrochloride; fazarabine; fenretinide; floxuridine; fludarabine phosphate; fluorouracil; flurocitabine; fosquidone; fostriecin sodium; gemcitabine; gemcitabine hydrochloride; hydroxyurea; idarubicin hydrochloride; ifosfamide; ilmofosine; interleulin II (including recombinant interleukin II, or rIL2), interferon alfa-2a; interferon alfa-2b; interferon alfa-n1; interferon alfa-n3; interferon beta-I a; interferon gamma-I b; iproplatin; irinotecan hydrochloride; lanreotide acetate; letrozole; leuprolide acetate; liarozole hydrochloride; lometrexol sodium; lomustine; losoxantrone hydrochloride; masoprocol; maytansine; mechlorethamine, mechlorethamine oxide hydrochloride rethamine hydrochloride; megestrol acetate; melengestrol acetate; melphalan; menogaril; mercaptopurine; methotrexate; methotrexate sodium; metoprine; meturedepa; mitindomide; mitocarcin; mitocromin; mitogillin; mitomalcin; mitomycin; mitosper; mitotane; mitoxantrone hydrochloride; mycophenolic acid; nocodazole; nogalamycin; ormaplatin; oxisuran; paclitaxel; pegaspargase; peliomycin; pentamustine; peplomycin sulfate; perfosfamide; pipobroman; piposulfan; piroxantrone hydrochloride; plicamycin; plomestane; porfimer sodium; porfiromycin; prednimustine; procarbazine hydrochloride; puromycin; puromycin hydrochloride; pyrazofurin; riboprine; rogletimide; safingol; safingol hydrochloride; semustine; simtrazene; sparfosate sodium; sparsomycin; spirogermanium hydrochloride; spiromustine; spiroplatin; streptonigrin; streptozocin; sulofenur; talisomycin; tecogalan sodium; tegafur; teloxantrone hydrochloride; temoporfin; teniposide; teroxirone; testolactone; thiamiprine; thioguanine; thiotepa; tiazofurin; tirapazamine; toremifene citrate; trestolone acetate; triciribine phosphate; trimetrexate; trimetrexate glucuronate; triptorelin; tubulozole hydrochloride; uracil mustard; uredepa; vapreotide; verteporfin; vinblastine sulfate; vincristine sulfate; vindesine; vindesine sulfate; vinepidine sulfate; vinglycinate sulfate; vinleurosine sulfate; vinorelbine tartrate; vinrosidine sulfate; vinzolidine sulfate; vorozole; zeniplatin; zinostatin; zorubicin hydrochloride, improsulfan, benzodepa, carboquone, triethylenemelamrine, triethylenephosphoramide, triethylenethiophosphoramide, trimethylolomelainine, chlomaphazine, novembichin, phenesterine, trofosfamide, estermustine, chlorozotocin, gemzar, nimustine, ranimustine, dacarbazine, mannomustine, mitobronitol, aclacinomycins, actinomycin F(1), azaserine, bleomycin, carubicin, carzinophilin, chromomycin, daunorubicin, daunomycin, 6-diazo-5-oxo-1-norleucine, doxorubicin, olivomycin, plicamyciri, porfiromycin, puromycin, tubercidin, zorubicin, denopterin, pteropterin, 6-mercaptopurine, ancitabine, 6-azauridine, carmofur, cytarabine, dideoxyuridine, enocitabine, pulmozyme, aceglatone, aldophosphamide glycoside, bestrabucil, defofamide, demecolcine, elfornithine, elliptinium acetate, etoglucid, flutamide, hydroxyurea, lentinan, phenamet, podophyllinic acid, 2-ethylhydrazide, razoxane, spirogermanium, tamoxifen, taxotere, tenuazonic acid, triaziquone, 2,2',2"-trichlorotriethylamine, urethan, vinblastine, vincristine, vindesine and related agents. 20-epi-1,25 dihydroxyvitamin D3; 5-ethynyluracil; abiraterone; aclarubicin; acylfulvene; adecypenol; adozelesin; aldesleukin; ALL-TK antagonists; altretamine; ambamustine; amidox; amifostine; aminolevulinic acid; amrubicin; amsacrine; anagrelide; anastrozole; andrographolide; angiogenesis inhibitors; antagonist D; antagonist G; antarelix; anti-dorsalizing morphogenetic protein-1; antiandrogen, prostatic carcinoma; antiestrogen; antineoplaston; antisense oligonucleotides; aphidicolin glycinate; apoptosis gene modulators; apoptosis regulators; apurinic acid; ara-CDP-DL-PTBA; arginine deaminase; asulacrine; atamestane; atrimustine; axinastatin 1; axinastatin 2; axinastatin 3; azasetron; azatoxin; azatyrosine; baccatin III derivatives; balanol; batimastat; BCR/ABL antagonists; benzochlorins; benzoylstaurosporine; beta lactam derivatives; beta-alethine; betaclamycin B; betulinic acid; bFGF inhibitor; bicalutamide; bisantrene; bisaziridinylspermine; bisnafide; bistratene A; bizelesin; breflate; bropirimine; budotitane; buthionine sulfoximine; calcipotriol; calphostin C; camptothecin derivatives; canarypox IL-2; capecitabine; carboxamide-amino-triazole; carboxyamidotriazole; CaRest M3; CARN 700; cartilage derived inhibitor; carzelesin; casein kinase inhibitors (ICOS); castanospermine; cecropin B; cetrorelix; chlorlns; chloroquinoxaline sulfonamide; cicaprost; cis-porphyrin; cladribine; clomifene analogues; clotrimazole; collismycin A; collismycin B; combretastatin A4; combretastatin analogue; conagenin; crambescidin 816; crisnatol; cryptophycin 8; cryptophycin A derivatives; curacin A; cyclopentanthraquinones; cycloplatam; cypemycin; cytarabine ocfosfate; cytolytic factor; cytostatin; dacliximab; decitabine; dehydrodidemnin B; deslorelin; dexamethasone; dexifosfamide; dexrazoxane; dexverapamil; diaziquone; didemnin B; didox; diethylnorspermine; dihydro-5-azacytidine; dihydrotaxol, 9-; dioxamycin; diphenyl spiromustine; docetaxel; docosanol; dolasetron; doxifluridine; droloxifene; dronabinol; duocarmycin SA; ebselen; ecomustine, edelfosine; edrecolomab; eflornithine; elemene; emitefur; epirubicin; epristeride; estramustine analogue; estrogen agonists; estrogen antagonists; etanidazole; etoposide phosphate; exemestane; fadrozole; fazarabine; fenretinide; filgrastim; finasteride; flavopiridol; flezelastine; fluasterone; fludarabine; fluorodaunorunicin hydrochloride; forfenimex; formestane; fostriecin; fotemustine; gadolinium texaphyrin; gallium nitrate; galocitabine; ganirelix; gelatinase inhibitors; gemcitabine; glutathione inhibitors; hepsulfam; heregulin; hexamethylene bisacetamide; hypericin; ibandronic acid; idarubicin; idoxifene; idramantone; ilmofosine; ilomastat; imidazoacridones; imiquimod; immunostimulant peptides; insulin-like growth factor-1 receptor inhibitor; interferon agonists; interferons; interleukins; iobenguane; iododoxorubicin; ipomeanol, 4-; iroplact; irsogladine; isobengazole; isohomohalicondrin B; itasetron; jasplakinolide; kahalalide F; lamellarin-N triacetate; lanreotide; leinamycin; lenograstim; lentinan sulfate; leptolstatin; letrozole; leukemia inhibiting factor; leukocyte alpha interferon; leuprolide+estrogen+progesterone; leuprorelin; levamisole; liarozole; linear polyamine analogue; lipophilic disaccharide peptide; lipophilic platinum compounds; lissoclinamide 7; lobaplatin; lombricine; lometrexol; lonidamine; losoxantrone; lovastatin; loxoribine; lurtotecan; lutetium texaphyrin; lysofylline; lytic peptides; maitansine; mannostatin A; marimastat; masoprocol; maspin; matrilysin inhibitors; matrix metalloproteinase inhibitors; menogaril; merbarone; meterelin; methioninase; metoclopraminde; MIF inhibitor; mifepristone; miltefosine; nirimostim; mismatched double stranded RNA; mitoguazone; mitolactol; mitomycin analogues; mitonafide; mitotoxin fibroblast growth factor-saporin; mitoxantrone; mofarotene; molgramostim; monoclonal antibody, human chorionic gonadotrophin; monophosphoryl lipid A+myobacterium cell wall sk; mopidamol; multiple drug resistance gene inhibitor; multiple tumor suppressor 1-based therapy; mustard anticancer agent; mycaperoxide B; mycobacterial cell wall extract; myriaporone; N-acetyldinaline; N-substituted benzamides; nafarelin; nagrestip; naloxone+pentazocine; napavin; naphterpin; nartograstim; nedaplatin; nemorubicin; neridronic acid; neutral endopeptidase; nilutamide; nisamycin; nitric oxide modulators; nitroxide antioxidant; nitrullyn; 06-benzylguanine; octreotide; okicenone; oligonucleotides; onapristone; ondansetron; ondansetron; oracin; oral cytokine inducer; ormaplatin; osaterone; oxaliplatin; oxaunomycin; taxel; taxel analogues; taxel derivatives; palauamine; palmitoylrhizoxin; pamidronic acid; panaxytriol; panomifene; parabactin; pazelliptine; pegaspargase; peldesine; pentosan polysulfate sodium; pentostatin; pentrozole; perflubron; perfosfamide; perillyl alcohol; phenazinomycin; phenylacetate; phosphatase inhibitors; picibanil; pilocarpine hydrochloride; pirarubicin; piritrexim; placetin A; placetin B; plasminogen activator inhibitor; platinum complex; platinum compounds; platinum-triamine complex; porfimer sodium; porfiromycin; prednisone; propyl bis-acridone; prostaglandin J2; proteasome inhibitors; protein A-based immune modulator; protein kinase C inhibitor; protein kinase C inhibitors, microalgal; protein tyrosine phosphatase inhibitors; purine nucleoside phosphorylase inhibitors; purpurins; pyrazoloacridine; pyridoxylated hemoglobin polyoxyethylene conjugate; raf antagonists; raltitrexed; ramosetron; ras farnesyl protein transferase inhibitors; ras inhibitors; ras-GAP inhibitor; retelliptine demethylated; rheniuim Re 186 etidronate; rhizoxin; ribozymes; RII retinamide; rogletimide; rohitukine; romurtide; roquinimex; rubiginone B1; ruboxyl; safingol; saintopin; SarCNU; sarcophytol A; sargramostim; Sdi 1 mimetics; semustine; senescence derived inhibitor 1; sense oligonucleotides; signal transduction inhibitors; signal transduction modulators; single chain antigen binding protein; sizofiran; sobuzoxane; sodium borocaptate; sodium phenylacetate; solverol; somatomedin binding protein; sonermin; sparfosic acid; spicamycin D; spiromustine; splenopentin; spongistatin 1; squalamine; stem cell inhibitor; stem-cell division ibitors; stipiamide; stromelysin inhibitors; sulfinosine; superactive vasoactive intestinal peptide antagonist; suradista; suramin; swainsonine; synthetic glycosaminoglycans; tallimustine; tamoxifen methiodide; tauromustine; tazarotene; tecogalan sodium; tegafur; tellurapyrylium; telomerase inhibitors; temoporfin; temozolomide; teniposide; tetrachlorodecaoxide; tetrazomine; thaliblastine; thiocoraline; thrombopoietin; thrombopoietin mimetic; thymalfasin; thymopoietin receptor agonist; thymotrinan; thyroid stimulating hormone; tin ethyl etiopurpurin; tirapazamine; titanocene bichloride; topsentin; toremifene; totipotent stem cell factor;.translation inhibitors; tretinoin; triacetyluridine; triciribine; trimetrexate; triptorelin; tropisetron; turosteride; tyrosine kinase inhibitors; tyrphostins; UBC inhibitors; ubenimex; urogenital sinus-derived growth inhibitory factor; urokinase receptor antagonists; vapreotide; variolin B; vector system, erythrocyte gene therapy; velaresol; veramine; verdins; verteporfin; vinorelbine; vinxaltine; vitaxin; vorozole; zanoterone; zeniplatin; zilascorb; and zinostatin stimalamer.

The method(s) for treating or preventing bacterial infections (or diseases) described herein may be used in combination with one or more anti-bacterial agents. Anti-bacterial agents include, but are not limited to, aminoglycosides, β-lactam agents, cephalosporins, macrolides, penicillins, quinolones, sulfonamides, and tetracyclines. Examples of anti-bacterial agents include, but are not limited to, Acedapsone, Acetosulfone Sodium, Alamecin, Alexidine, Amdinocillin Clavulanate Potassium, Amdinocillin, Amdinocillin Pivoxil, Amicycline, Arizifloxacin, Amifloxacin Mesylate, Amikacin, Amikacin Sulfate, Aminosalicylic acid, Aminosalicylate sodium, Amoxicillin, Amphomycin, Ampicillin, Ampicillin Sodium, Apalcillin Sodium, Apramycin, Aspartocin, Astromicin Sulfate, Avilamycin, Avoparcin, Azithromycin, Azlocillin, Azlocillin Sodium, Bacampicillin Hydrochloride, Bacitracin, Bacitracin Methylene Disalicylate, Bacitracin Zinc, Bambermycins, Benzoylpas Calcium, Berythromycin, Betamicin Sulfate, Biapenem, Biniramycin, Biphenamine Hydrochloride, Bispyrithione Magsulfex, Butikacin, Butirosin Sulfate, Capreomycin Sulfate, Carbadox, Carbenicillin Disodium, Carbenicillin Indanyl Sodium, Carbenicillin Phenyl Sodium, Carbenicillin Potassium, Carumonam Sodium, Cefaclor, Cefadroxil, Cefamandole, Cefamandole Nafate, Cefamandole Sodium, Cefaparole, Cefatrizine, Cefazaflur Sodium, Cefazolin, Cefazolin Sodium, Cefbuperazone, Cefdinir, Cefditoren Pivoxil, Cefepime, Cefepime Hydrochloride, Cefetecol, Cefixime, Cefmenoxime Hydrochloride, Cefmetazole, Cefmetazole Sodium, Cefonicid Monosodium, Cefonicid Sodium, Cefoperazone Sodium, Ceforanide, Cefotaxime, Cefotaxime Sodium, Cefotetan, Cefotetan Disodium, Cefotiam Hydrochloride, Cefoxitin, Cefoxitin Sodium, Cefpimizole, Cefpimizole Sodium, Cefpiramide, Cefpiramide Sodium, Cefpirome Sulfate, Cefpodoxime Proxetil, Cefprozil, Cefroxadine, Cefsulodin Sodium, Ceftazidime, Ceftazidime Sodium, Ceftibuten, Ceftizoxime Sodium, Ceftriaxone Sodium, Cefuroxime, Cefuroxime Axetil, Cefuroxime Pivoxetil, Cefuroxime Sodium, Cephacetrile Sodium, Cephalexin, Cephalexin Hydrochloride, Cephaloglycin, Cephaloridine, Cephalothin Sodium, Cephapirin Sodium, Cephradine, Cetocycline Hydrochloride, Cetophenicol, Chloramphenicol, Chloramphenicol Palmitate, Chloramphenicol Pantothenate Complex, Chloramphenicol Sodium Succinate, Chlorhexidine Phosphanilate, Chloroxylenol, Chlortetracycline Bisulfate, Chlortetracycline Hydrochloride, Cilastatin, Cinoxacin, Ciprofloxacin, Ciprofloxacin Hydrochloride, Cirolemycin, Clarithromycin, Clavulanate Potassium, Clinafloxacin Hydrochloride, Clindamycin, Clindamycin Dextrose, Clindamycin Hydrochloride, Clindamycin Palmitate Hydrochloride, Clindamycin Phosphate, Clofazimine, Cloxacillin Benzathine, Cloxacillin Sodium, Cloxyquin, Colistimethate, Colistimethate Sodium, Colistin Sulfate, Coumermycin, Coumermycin Sodium, Cyclacillin, Cycloserine, Dalfopristin, Dapsone, Daptomycin, Demeclocycline, Demeclocycline Hydrochloride, Demecycline, Denofungin, Diaveridine, Dicloxacillin, Dicloxacillin Sodium, Dihydrostreptomycin Sulfate, Dipyrithione, Dirithromycin, Doxycycline, Doxycycline Calcium, Doxycycline Fosfatex, Doxycycline Hyclate, Doxycycline Monohydrate, Droxacin Sodium, Enoxacin, Epicillin, Epitetracycline Hydrochloride, Ertapenem, Erythromycin, Erythromycin Acistrate, Erythromycin Estolate, Erythromycin Ethylsuccinate, Erythromycin Gluceptate, Erythromycin Lactobionate, Erythromycin Propionate, Erythromycin Stearate, Ethambutol Hydrochloride, Ethionamide, Fleroxacin, Floxacillin, Fludalanine, Flumequine, Fosfomycin, Fosfomycin Tromethamine, Fumoxicillin, Furazolium Chloride, Furazolium Tartrate, Fusidate Sodium, Fusidic Acid, Gatifloxacin, Genifloxacin, Gentamicin Sulfate, Gloximonam, Gramicidin, Haloprogin, Hetacillin, Hetacillin Potassium, Hexedine, Ibafloxacin, Imipenem, Isoconazole, Isepamicin, Isoniazid, Josamycin, Kanamycin Sulfate, Kitasamycin, Levofloxacin, Levofuraltadone, Levopropylcillin Potassium, Lexithromycin, Lincomycin, Lincomycin Hydrochloride, Linezolid, Lomefloxacin, Lomefloxaciri Hydrochloride, Lomefloxacin Mesylate, Loracarbef, Mafenide, Meclocycline, Meclocycline Sulfosalicylate, Megalomicin Potassium Phosphate, Mequidox, Meropenem, Methacycline, Methacycline Hydrochloride, Methenamine, Methenamine Hippurate, Methenamine Mandelate, Methicillin Sodium, Metioprim, Metronidazole Hydrochloride, Metronidazole Phosphate, Mezlocillin, Mezlocillin Sodium, Minocycline, Minocycline Hydrochloride, Mirincamycin Hydrochloride, Monensin, Monensin Sodium, Moxifloxacin Hydrochloride, Nafcillin Sodium, Nalidixate Sodium, Nalidixic Acid, Natamycin, Nebramycin, Neomycin Palmitate, Neomycin Sulfate, Neomycin Undecylenate, Netilmicin Sulfate, Neutramycin, Nifuradene, Nifuraldezone, Nifuratel, Nifuratrone, Nifurdazil, Nifurimide, Nifurpirinol, Nifurquinazol, Nifurthiazole, Nitrocycline, Nitrofurantoin, Nitromide, Norfloxacin, Novobiocin Sodium, Ofloxacin, Ormetoprim, Oxacillin Sodium, Oximonam, Oximonam Sodium, Oxolinic Acid, Oxytetracycline, Oxytetracycline Calcium, Oxytetracycline Hydrochloride, Paldimycin, Parachlorophenol, Paulomycin, Pefloxacin, Pefloxacin Mesylate, Penamecillin, Penicillin G Benzathine, Penicillin G Potassium, Penicillin G Procaine, Penicillin G Sodium, Penicillin V, Penicillin V Benzathine, Penicillin V Hydrabamine, Penicillin V Potassium, Pentizidone Sodium, Phenyl Aminosalicylate, Piperacillin, Piperacillin Sodium, Pirbenicillin Sodium, Piridicillin Sodium, Pirlimycin Hydrochloride, Pivampicillin Hydrochloride, Pivampicillin Pamoate, Pivampicillin Probenate, Polymyxin B Sulfate, Porfiromycin, Propikacin, Pyrazinamide, Pyrithione Zinc, Quindecamine Acetate, Quinupristin, Racephenicol, Ramoplanin, Ranimycin, Relomycin, Repromicin, Rifabutin, Rifametane, Rifamexil, Rifamide, Rifarripin, Rifapentine, Rifaximin, Rolitetracycline, Rolitetracycline Nitrate, Rosaramicin, Rosaramicin Butyrate, Rosaramicin Propionate, Rosaramicin Sodium Phosphate, Rosaramicin Stearate, Rosoxacin, Roxarsone, Roxithromycin, Sancycline, Sanfetrinem Sodium, Sarmoxicillin, Sarpicillin, Scopafungin, Sisomicin, Sisomicin Sulfate, Sparfloxacin, Spectinomycin Hydrochloride, Spiramycin, Stallimycin Hydrochloride, Steffimycin, Sterile Ticarcillin Disodium, Streptomycin Sulfate, Streptonicozid, Sulbactam Sodium, Sulfabenz, Sulfabenzamide, Sulfacetamide, Sulfacetamide Sodium, Sulfacytine, Sulfadiazine, Sulfadiazine Sodium, Sulfadoxine, Sulfalene, Sulfamerazine, Sulfameter, Sulfamethazine, Sulfamethizole, Sulfamethoxazole, Sulfamonomethoxine, Sulfamoxole, Sulfanilate Zinc, Sulfanitran, Sulfasalazine, Sulfasomizole, Sulfathiazole, Sulfazamet, Sulfisoxazole, Sulfisoxazole Acetyl, Sulfisoxazole Diolamine, Sulfomyxin, Sulopenem, Sultamicillin, Suncillin Sodium, Talampicillin Hydrochloride, Tazobactam, Teicoplanin, Temafloxacin Hydrochloride, Temocillin, Tetracycline, Tetracycline Hydrochloride, Tetracycline Phosphate Complex, Tetroxoprim, Thiamphenicol, Thiphencillin Potassium, Ticarcillin Cresyl Sodium, Ticarcillin Disodium, Ticarcillin Monosodium, Ticlatone, Tiodonium Chloride, Tobramycin, Tobramycin Sulfate, Tosufloxacin, Trimethoprim, Trimethoprim Sulfate, Trisulfapyrimidines, Troleandomycin, Trospectomycin Sulfate, Trovafloxacin, Tyrothricin, Vancomycin, Vancomycin Hydrochloride, Virginiamycin, Zorbamycin.

The method(s) for treating or preventing viral infections (or diseases) described herein may be used in combination with one or more anti-viral agents. Anti-viral agents can be isolated from natural sources or synthesized and are useful for killing or inhibiting the growth or function of viruses. Examples of anti-viral agents include, but are not limited to, immunoglobulins, amantadine, interferons, nucleotide analogues, and protease inhibitors. Specific examples of anti-virals include but are not limited to Acemannan; Acyclovir; Acyclovir Sodium; Adefovir; Alovudine; Alvircept Sudotox; Amantadine Hydrochloride; Aranotin; Arildone; Atevirdine Mesylate; Avridine; Cidofovir; Cipamfylline; Cytarabine Hydrochloride; Delavirdine Mesylate; Desciclovir; Didanosine; Disoxaril; Edoxudine; Enviradene; Enviroxime; Famciclovir; Famotine Hydrochloride; Fiacitabine; Fialuridine; Fosarilate; Foscarnet Sodium; Fosfonet Sodium; Ganciclovir; Ganciclovir Sodium; Idoxuridine; Kethoxal; Lamivudine; Lobucavir; Memotine Hydrochloride; Methisazone; Nevirapine; Penciclovir; Pirodavir; Ribavirin; Rimantadine Hydrochloride; Saquinavir Mesylate; Somantadine Hydrochloride; Sorivudine; Statolon; Stavudine; Tilorone Hydrochloride; Trifluridine; Valacyclovir Hydrochloride; Vidarabine; Vidarabine Phosphate; Vidarabine Sodium Phosphate; Viroxime; Zalcitabine; Zidovudine; and Zinviroxime.

Antiviral agents also include nucleotide analogues. Examples of nucleotide analogues include, but are not limited to, acyclovir (used for the treatment of herpes simplex virus and varicella-zoster virus), gancyclovir (useful for the treatment of cytomegalovirus), idoxuridine, ribavirin (useful for the treatment of respiratory syncitial virus), dideoxyinosine, dideoxycytidine, zidovudine (azidothymidine), imiquimod, and resimiquimod.

Interferons are cytokines which are secreted by virus-infected cells as well as immune cells. The interferons function by binding to specific receptors on cells adjacent to the infected cells, causing the change in the cell which protects it from infection by the virus. α and β-interferon also induce the expression of Class I and Class II MHC molecules on the surface of infected cells, resulting in increased antigen presentation for host immune cell recognition α and β-interferons are available as recombinant forms and have been used for the treatment of chronic hepatitis B and C infection. At the dosages which are effective for anti-viral therapy, interferons have severe side effects such as fever, malaise and weight loss.

The method(s) for treating or preventing fungal infections (or diseases) described herein may be used in combination with one or more anti-fungal agents. Examples of anti-fungal agents include, but are not limited to, immidazoles, such as clotrimazole, sertaconzole, fluconazole, itraconazole, ketoconazole, miconazole, and voriconacole, as well as FK 463, amphotericin B, BAY 38-9502, MK 991, pradimicin, UK 292, butenafine, and terbinafine. Other anti-fungal agents function by breaking down chitin (e.g. chitinase) or immunosuppression (501 cream).

The method(s) for treating or preventing protozoal infections (or diseases) described herein may be used in combination with one or more anti-protozoal agents. Examples of anti-protozoal agents include, but are not limited to, albendazole, amphotericin B, benznidazole, bithionol, chloroquine HCl, chloroquine phosphate, clindamycin, dehydroemetine, diethylcarbamazine, diloxanide furoate, eflornithine, furazolidaone, glucocorticoids, halofantrine, iodoquinol, ivermectin, mebendazole, mefloquine, meglumine antimoniate, melarsoprol, metrifonate, metronidazole, niclosamide, nifurtimox, oxamniquine, paromomycin, pentamidine isethionate, piperazine, praziquantel, primaquine phosphate, proguanil, pyrantel pamoate, pyrimethanmine-sulfonamides, pyrimethanmine-sulfadoxine, quinacrine HCl, quinine sulfate, quinidine gluconate, spiramycin, stibogluconate sodium (sodium antimony gluconate), suramin, tetracycline, doxycycline, thiabendazole, tinidazole, trimethroprim-sulfamethoxazole, and tryparsamide some of which are used alone or in combination with others.

The present invention is further illustrated by the following Examples.

### EXAMPLES

### Example 1: Epidermal V-V immunization via skin scarification generates significantly stronger- cellular and humoral immunity than the conventional injection routes.

A mouse model of W skin scarification was developed. The acute epidermal pox reaction developed in scarified mice closely resembles that of human smallpox vaccines. Using this model, a rigorous comparison of the primary and memory adaptive immune response following vaccinia virus (VV) immunization via skin scarification (s.s.), subcutaneous (s.c.), intradermal (i.d.) and intramuscular (i.m.) injection was undertaken. The highly immunogenic intraperitoneal (i.p.) injection route, although not used for immunization clinically, was included as positive control for VV-specific immune responses. VV skin scarification induced significantly stronger primary and memory T cell response, as well as higher serum VV-specific IgG levels, compared to the conventional injection routes (s.c., i.d., and i.m.) (Fig. 1). Long-term T cell memory and serum IgG levels were comparable in VV scarified mice and i.p. immunized mice. Thus, localized epidermal VV immunization via skin scarification achieves comparable immunogenicity compared to i.p. infection which establishes systemic viral infection, according to IFN-γ response and serum IgG level.

### Example 2: VV skin scarification provides superior protection against secondary antigenic challenge.

It was determined whether VV skin scarification could provide better protection against secondary challenge using three different models. The first challenge model was cutaneous poxvirus infection (via skin infection). This model was chosen for two reasons. First, clinically, the protection efficacy of smallpox vaccine candidates is evaluated by challenging vaccinated individuals with Dryvax skin scarification. Second, natural poxvirus infection can be acquired via cutaneous exposure to the viruses, especially at injured skin area. Following skin challenge, viral load in skin was determined by VV-specific real-time PCR (Fig. 2). Compared to unimmunized control mice, mice immunized by s.c., i.d., and i.m. injection all demonstrated partial protection, with 15, 9.5 and 3-fold reduction in viral load, respectively. 3-log reduction in viral load was achieved in i.p. immunized mice. Strikingly, all the mice previously immunized via skin scarification completely cleared the virus by this time point. Therefore, skin scarification provided superior protection against secondary cutaneous poxvirus challenge compared to the injection routes, including the highly immunogenic i.p. route.

Natural poxvirus infection is primarily transmitted by respiratory aerosol. Therefore, the protection efficacy of various immunization routes against lethal intranasal infection by pathogenic Western Reserve vaccinia virus (WR-VV) was evaluated. As shown in Fig. 3a, b, mice immunized via s.c., i.d. and i.m. injection routes (at 2 x 10⁶ pfu dose) developed apparent clinical illness (manifested by the significant loss of body weight, BW), and were only partially protected from mortality. In contrast, mice immunized via s.s. and i.p. routes were completely protected with 100% survival and minimal BW changes. In fact, skin scarification route achieved better protection efficacy than s.c., i.d. and i.m. routes even at a 1000 x lower dose (Fig. 3c, d). Thus, VV immunization via skin scarification protected respiratory mucosa more effectively than the conventional immunization routes.

It was then investigated whether the superior protection associated with VV skin scarification can be extended to non-viral challenge model. C57B1/6 mice were immunized with recombinant vaccinia virus (rVV) expressing ovalbumine (OVA) K^{b} epitope Ova₂₅₇₋₂₆₄ via different routes, and challenged with B16 melanoma cells expressing OVA intradermally 5 weeks following immunization. By 18 days after tumor implantation, all the mice immunized via the injection routes (s.c., i.d., i.m., and i.p.) developed large cutaneous melanoma mass (Fig. 4a-d). Remarkably, no visible or palpable tumor was detected on any of the skin scarified mice (Fig. 4e). Although eventually tumor developed in all the B16-OVA challenged mice, the tumor growth was significantly delayed (Fig. 4f) and survival was greatly improved (data not shown) in the scarification group compared to other groups. Given the vaccine and tumor cells only share a single CD8 T cell epitope, and mice were only given a single dose VV skin scarification, these results are remarkably promising and of broad clinical implication. It is highly possible that over time, the OVA expression is lost from the B16 tumor cells under the immune selection, rendering Ova₂₅₇₋₂₆₄-specific CD8⁺ T cell memory response ineffective to suppress tumor growth.

### Example 3: Memory T cells but not Ab are required for VV skin scarification-associated protection against secondary challenge.

To investigate the mechanism underlying the superior protective efficacy following poxvirus skin scarification, the relative contribution of humoral and cellular response in skin scarification-associated immune protection was studied. Wild type (wt) and B cell-deficient µMT mice were immunized with VV by skin scarification or i.p. injection, the two most immunogenic routes in this study. The memory mice were then challenged by secondary cutaneous or intranasal poxvirus infection. As shown in Fig. 5a, when challenged with VV on skin, the i.p. immunized µMT mice had a viral load 4-log higher than that of the i.p. immunized wt mice. Interestingly, µMT mice immunized via s.s. route still demonstrated strong protection against cutaneous challenge, with a viral load comparable to that of the wt mice immunized via skin scarification. However, when T cells were depleted from the wt memory mice before and during challenge (by large dose treatment with anti-CD4 and anti-CD8 mAbs), the immune protection was completely abrogated in both s.s. and i.p. immunization groups. This data suggest that while both T cell and Ab are required for the protection against cutaneous challenge following i.p. immunization, T cell memory response alone following VV skin scarification is strong enough to effectively control cutaneous challenge. Indeed, secondary T cell response in both spleen and lymph node (LN) draining the challenged skin was significantly stronger in scarified immune mice than in i.p. immunized mice (Fig. 5b, c). The difference between the immunization groups was even more striking in µMT mice. Challenged skin tissues from different groups of mice were further examined microscopically for the presence of T cells. Strikingly, massive CD3⁺ T cell infiltration was observed in the basal epidermis and dermis of mice immunized via s.s. route, while only a few T cells were scattered in the skin harvested from all other immunization groups (Fig. 5d). Collectively, these data suggest that VV skin scarification is uniquely potent in generating large number of skin-homing Tₑₘ that are highly protective against secondary cutaneous antigenic challenge.

Similarly, T cell memory seemed to be more important than Ab response for the complete protection against intranasal challenge. Wt and µMT mice were immunized with VV via skin scarification and lethally challenged with WR-VV via intranasal infection. Both strains of mice were completely protected against mortality with minimal change of BW. However, depletion of T cells from wt memory mice led to more pronounced BW loss, although all the T cell-depleted immune mice survived the challenge (Fig. 6). This data suggest protective Tₑₘ either residing in or able to rapidly migrate into respiratory epithelial lining are generated by epicutaneous VV immunization via skin scarification, along with the skin-homing Tₑₘ. These cells play an important role in the immune surveillance against aerosol-transmitted pathogens.

### Example 4: VV skin scarification generates large number of skin-homing T_{eff} /Tₑₘ, as well as T cells with highly versatile homing ability by primary and secondary tissue homing imprinting programs in regional LN.

How do antigen-specific T cells develop the highly versatile homing ability to provide systemic immune protection following a local VV infection restricted to skin?

This question was investigated by adoptively transferring naive CFSE-labeled Thy1.1⁺ Ova₂₅₇₋₂₆₄-specific OT-I T cells into Thy1.2⁺ wt mice and subsequently tracking their in vivo activation, proliferation and migration following skin scarification or i.p. infection with rVV-Ova₂₅₇₋₂₆₄. OT-I cells proliferated extensively within skin-draining inguinal LN (ILN) as early as 60 h following skin scarification, while similar proliferation was seen in the gut-draining mesenteric LN (MLN) after i.p. infection (Fig. 7a). As expected, these OT-I cells significantly down-regulated LN-homing molecule CD62L, which is highly expressed on naive and T_{cm} T cells circulating among secondary lymphoid tissues (Fig. 7b). Concurrently, there was a robust up-regulation of skin-homing molecule E-Lig and P-Lig (P-selectin ligands) on OT-I cells activated within ILN, and gut-homing molecule α4β7 on OT-I cells activated within MLN. The expression of the tissue-homing molecules was upregulated after 3 cell divisions and continued to increase as a function of cell division (Fig. 7c). Thus, early upon activation, antigen-specific T cells are imprinted with tissue-specific homing phenotype within regional LN where priming occurs (primary homing imprinting). This enabled the activated T cells to migrate specifically to the infected tissues as early as day 3 after VV skin scarification (data not shown). This surprisingly rapid T cell recruitment into skin after primary VV skin scarification had not been previously appreciated.

Several additional unanticipated findings were made. In contrast to the highly specific tissue trafficking at 60 h following infection, activated OT-I cells had disseminated into non-draining LN by 5 days after rVV-ova skin scarification (Fig. 8a). This was not accompanied by systemic dissemination of VV or VV antigen-bearing antigen presenting cells (APC), since vaccinia gene expression was not detected outside skin or skin-draining LN by the highly sensitive real time RT-PCR (Fig. 8b), and APC isolated from MLN and spleen failed to activate OT-I cells in vitro (Fig. 8c). Therefore, a subset of ILN-activated OT-I cells disseminate throughout lymphoid tissues and continued to divide in the absence of continued antigen stimulation. Unexpectedly, they also expressed additional tissue-specific homing molecules. As seen in Fig. 9a, gut-homing α4β7 was upregulated on the proliferating OT-I cells. When the egress of OT-I cells from ILN after their activation was blocked using FTY720, a functional antagonist of sphingosine 1-phosphate that regulates lymphocyte egress from lymphoid tissues (Fig. 9b), the expression of E-Lig on OT-I cells was unaffected (Fig. 9c), however, α4β7 on OT-I cells was abrogated (Fig. 9d). This data strongly suggest the upregulation of gut homing molecules occurred subsequent to OT-I migration into MLN (secondary tissue homing imprinting). We believe a similar process may happen in other non-draining LN where putative lung and mucosal trafficking molecules are expressed. When T cells were examined at 30 days after infection, both primary (skin-specific E-Lig) and secondary (gut-specific α4β7) homing molecules persisted on memory OT-I cells (Fig. 10). These data collectively suggest a mechanism by which localized VV immunization via skin scarification generates protective T cell response for both immediate skin-specific immune control at the virus entry site, and a more flexible systemic protection against potential viral dissemination or secondary challenge at a distinct anatomic location, such as respiratory epithelium.

### Example 5: MVA skin scarification represents a novel immunization strategy that is superior, safe and effective.

The highly attenuated replication-defective VV strain MVA has been actively explored as a promising live viral vaccine vector because of its impressive safety and immunogenicity profile in both preclinical and clinical studies. MVA vaccines have been administered exclusively via injection routes, and never via skin scarification. This may due to the intuitive assumption that viral replication in epidermis is required for the development of pox lesion and the subsequent strong protection against challenge.

Nevertheless, mice with MVA were immunized via skin scarification. Surprisingly, MVA skin scarification induced characteristic pox lesions in a dose-dependent manner (Fig. 11a), and generated dose-dependent cellular and humoral immune responses against VV antigens (Fig. 11b, c). Importantly, when lethally challenged with intranasal WR-VV infection, MVA skin scarification provided complete protection against mortality and illness at 1.8 x 10⁶ pfu (Fig. 11d), a dose at which replicative VV immunization via the conventional injection routes failed to protect mice from the lethal challenge (Fig. 3a, b). Not surprisingly, at a comparable dose (2 x 10⁶ pfu) of MVA immunization, the conventional injection routes only elicited weakly detectable T cell and Ab responses (Fig. 12a, b), even after secondary viral challenge (Fig. 12c, d), and offered poor protection against the WR-VV i.n. challenge (Fig. 12e, f), whereas strong immune protection was afforded by skin scarification with either MVA or VV.

Therefore, the superior immunogenicity and protection efficacy associated with skin scarification is extended to the highly attenuated MVA vaccine.

The safety of MVA skin scarification for immunocompromised hosts was confirmed in Rag-/- mice lacking both T cells and B cells. MVA scarified Rag-/- mice developed small pox lesion that was confined to the site of inoculation (Fig 13a), and survived long-term without losing any BW (Fig. 13b, c) or detectable viremia (Fig. 13d). In sharp contrast, VV scarified Rag-/- mice developed deteriorating skin erosion and necrosis and succumbed around 4 weeks after infection (Fig. 13 a-c).

The observation that MVA skin scarification is highly effective in generating protective immunity indicates productive viral infection in epidermis is not required to achieve strong protection efficacy. However, infection of epidermis with metabolically active live virus seems to be necessary for rigorous immune response to develop. This was suggested by, first, the failure of heat-inactivated VV to induce strong immune response when administered via skin scarification even at a high dose; second, the inability of simultaneous skin "scarification" with saline to enhance the immune responses in mice infected with VV by injection (data not shown). Unique aspects of innate cutaneous responses to VV infection in epidermis may serve as natural adjuvant to optimize the subsequent adaptive response. In support of this idea, it was found that primary human keratinocytes, but not dermal fibroblasts or dermal microvascular endothelial cells, are able to limit VV replication in vitro in the absence of host adaptive immune responses. Furthermore, transgenic overexpression of innate cytokine IL-1α in keratinocytes led to further enhancement of in vivo adaptive immune responses following VV skin scarification.

In summary, these observations demonstrate that the route of immunization is an important consideration for the design of vaccination strategy. Epidermal immunization with live viral vaccines, including the non-replicating viruses, generates far better immune responses and stronger protection compared to the injection routes routinely used in clinic, particularly concerning MVA.

## Claims

1. A live, modified, non-replicating poxvirus comprising a nucleic acid encoding a peptide or polypeptide antigen that is foreign to poxvirus, wherein the poxvirus infects, but is non-replicating in human cells; for use in a method of eliciting a T cell memory immune response to the foreign antigen, by administering the poxvirus to a mechanically disrupted epidermis of a subject so that the poxvirus expressing the foreign antigen infects the disrupted epidermis and elicits a T cell memory immune response.

2. The poxvirus for use as in claim 1, wherein the poxvirus is selected from the group consisting of: orthopox, suipox, capripox, leporipox, parapoxvirus, molluscpoxvirus, and yatapoxvirus.

3. The poxvirus for use as in claim 2, wherein the orthopox virus is a vaccinia virus.

4. The poxvirus for use as in claim 3, wherein the vaccinia virus is modified vaccinia virus Ankara (MVA).

5. The poxvirus for use as in claim 1, wherein the epidermis is mechanically disrupted by a scarification needle, a hypodermic needle, or an abrader.

6. The poxvirus for use as in claim 1, wherein the epidermis is mechanically disrupted at the same time as or before the administration of the poxvirus.

7. The poxvirus for use as in claim 1, wherein the subject has or is at risk of developing cancer.

8. The poxvirus for use as in claim 7, wherein the cancer is skin, breast, prostate, lung, brain, flung, ovary, liver, pancreas, stomach, kidney, bladder, and thyroid, or colorectal cancer.

9. The poxvirus for use as in claim 7, wherein the cancer is selected from the group consisting of melanoma, cutaneous squamous cell carcinoma, basal call carcinoma, Merkel cell carcinoma, adnexal carcinoma, cutaneous T or B cell lymphoma, sarcomas, adenocarcinoma, prostate adenocarcinoma, prostatic intraepithelial neoplasia, squamous cell lung carcinoma, lung adenocarcinoma, small cell lung carcinoma, ovarian cancer of epithelial origin, colorectal adenocarcinoma and leiomyosarcoma, stomach adenocarcinoma and leiomyosarcoma, hepatocellular carcinoma, cholangiocarcinoma; ductal adenocarcinomas of pancreas, endocrine pancreatic tumors, renal cell carcinoma, transitional cell carcinoma of kidney and bladder and bladder squamous cell carcinoma.

10. The poxvirus for use as in claim 1, wherein the subject has or is at risk of developing a viral, bacterial, fungal, or protozoal infection.

11. The poxvirus for use as in claim 10, wherein if the infection is a viral infection, the infection is caused by: HIV, influenza, dengue, Hepatitis A virus, Hepatitis B virus, Hepatitis C virus, human papilloma virus, Ebola, Marburg, Rabies, Hanta virus infection, West Nile virus, SARS-like Coronaviruses, Herpes simplex virus, Varicella-zoster virus, Epstein-Barr virus, Human herpesvirus 8, Alpha viruses, or St. Louis encephalitis;
wherein if the infection is a bacterial infection, the infection is caused by Mycobacterium tuberculosis, Salmonella typhi, Bacillus anthracis, Yersinia pestis, Francisella tularensis, Legionella, Chlamydia, Rickettsia typhi, or Treponema pallidum;
wherein if the infection is a fungal infection, the infection is caused by Coccidioides immitis, Blastomyces dermatitidis, Cryptococcus neoformans, Candida albicans, or an Aspergillus species;
wherein if the infection is a protozoal infection, the infection is caused by Malaria (Plasmodium falciparum, Plasmodium vivax, Plasmodium ovale, Plasmodium malariae), a Leishmania species, a Trypanosome species (African and American), a cryptosporidium, an isospora species, Naegleria fowleri, an Acanthamoeba species, Balamuthia mandrillaris, Toxoplasma gondii, or Pneumocystis carinii.

12. The poxvirus for use as in claim 1, wherein the antigen is a tumor-associated antigen (TAA), a tumor-specific antigen (TSA), or a tissue-specific antigen.

13. The poxvirus for use as in claim 1, wherein the antigen is a viral, bacterial, fungal or protozoal antigen.

14. The poxvirus for use as in claim 1, wherein a co-stimulatory molecule, a growth factor, an adjuvant and/or a cytokine, is co-administered before, at the same time as, or after the antigen, and at the same or different site.

15. The poxvirus for use as in claim 14, wherein the co-stimulatory molecule or cytokine is co-expressed with the antigen by the poxvirus.

16. The poxvirus for use as in claim 14 or 15, wherein the co-expressed co-stimulatory molecule is selected from the group consisting of: IL-1, IL-2, IL-7, IL-1 2, IL-15, IL-18, IL-23, IL-27, B7-2, B7-H3, CD40, CD40L, ICOS-ligand, OX-40L, 4-1BBL, GM-CSF, SCF, FGF, Flt3-ligand and

17. The poxvirus for use as in claim 14 or 15, wherein the poxvirus. expresses B7-1, ICAM-1 and LFA-3.

18. A kit comprising a device for mechanically disrupting a subject's epidermal tissue, such as a scarification needle or an abrader, and the poxvirus for use as in any one of claims 1-17.

## Patentansprüche

1. Lebendes, modifiziertes, nicht replizierendes Pockenvirus, umfassend eine Nukleinsäure, die für ein Peptid oder ein Polypeptid-Antigen kodiert, das dem Pockenvirus fremd ist, wobei das Pockenvirus menschliche Zellen infiziert, aber sich darin nicht repliziert, zur Verwendung bei einem Verfahren zum Hervorrufen einer Immunantwort von T-Gedächtniszellen auf das fremde Antigen, indem das Pockenvirus der mechanisch durchtrennten Epidermis eines Subjekts verabreicht wird, sodass das das fremde Antigen exprimierende Pockenvirus die durchtrennte Epidermis infiziert und eine Immunantwort der T-Gedächtniszellen hervorruft.

2. Pockenvirus zur Verwendung nach Anspruch 1, wobei das Pockenvirus ausgewählt ist aus der Gruppe bestehend aus: echten Pockenviren, Schweinepockenviren, Schaf- und Ziegenpockenviren, Hasenpockenviren, Parapockenviren, Molluscum-contagiosum-viren und Yatapockenviren.

3. Pockenvirus zur Verwendung nach Anspruch 2, wobei das echte Pockenvirus ein Vaccinia-Virus ist.

4. Pockenvirus zur Verwendung nach Anspruch 3, wobei das Vaccinia-Virus das modifizierte Vaccinia-Virus Ankara (MVA) ist.

5. Pockenvirus zur Verwendung nach Anspruch 1, wobei die Epidermis mittels einer Skarifikationsnadel, einer Injektionsnadel oder einem Schleifer mechanisch durchtrennt wird.

6. Pockenvirus zur Verwendung nach Anspruch 1, wobei die Epidermis zur gleichen Zeit oder vor der Verabreichung des Pockenvirus mechanisch durchtrennt wird.

7. Pockenvirus zur Verwendung nach Anspruch 1, wobei das Subjekt Krebs hat oder Gefahr läuft, Krebs zu entwickeln.

8. Pockenvirus zur Verwendung nach Anspruch 7, wobei der Krebs Haut-, Brust-, Prostata-, Lungen-, Hirn-, Lungen-, Eierstock-, Leber-, Bauchspeicheldrüsen-, Magen-, Nieren-, Blasen-, Schilddrüsen- oder Kolorektalkrebs ist.

9. Pockenvirus zur Verwendung nach Anspruch 7, wobei der Krebs aus der Gruppe bestehend aus Melanom, kutanem Plattenepithelkarzinom, Basalzellkarzinom, Merkelzellkarzinom, adnexalem Karzinom, kutanem T- oder B-Zelllymphom, Sarkomen, Adenokarzinom, Prostata-Adenokarzinom, prostatischer intraepithelialer Neoplasie, Plattenepithel-Lungenkarzinom, Lungen-Adenokarzinom, kleinzelligem Lungenkarzinom, Eierstockkrebs epithelialen Ursprungs, kolorektalem Adenokarzinom und Leiomyosarkom, Magen-Adenokarzinom und Leiomyosarkom, hepatozellulärem Karzinom, Cholangiokarzinom, duktalen Adenokarzinomen der Bauchspeicheldrüse, endokrinen Bauchspeicheldrüsentumoren, Nierenzellkarzinom, Übergangszellkarzinom der Niere und Blase sowie Plattenepithelkarzinom der Blase ausgewählt ist.

10. Pockenvirus zur Verwendung nach Anspruch 1, wobei das Subjekt eine Virus-, Bakterien-, Pilz- oder Protozoen-Infektion hat oder Gefahr läuft, eine solche Infektion zu entwickeln.

11. Pockenvirus zur Verwendung nach Anspruch 10, wobei wenn die Infektion eine Virusinfektion ist, die Infektion verursacht wird durch: HIV-Virus, Influenza-Virus, Dengue-Virus, Hepatitis-A-Virus, Hepatitis-B-Virus, Hepatitis-C-Virus, menschliches Papilloma-Virus, Ebola-Virus, Marburg-Virus, Tollwut-Virus, Hanta-Virus-Infektion, West-Nil-Virus, SARS-ähnliche Coronaviren, Herpes-simplex-Virus, Varicella-zoster-Virus, Epstein-Barr-Virus, menschliches Herpes-Virus 8, Alpha-Viren oder St-Louis-Enzephalitis-Virus;
wobei wenn die Infektion eine Bakterieninfektion ist, die Infektion verursacht wird durch Mycobacterium tuberculosis, Salmonella typhi, Bacillus anthracis, Yersinia pestis, Francisella tularensis, Legionellen, Chlamydien, Rickettsia typhi oder Treponema pallidum;
wobei wenn die Infektion eine Pilzinfektion ist, die Infektion verursacht wird durch Coccidioides immitis, Blastomyces dermatitidis, Cryptococcus neoformans, Candida albicans oder eine Aspergillus-Art;
wobei wenn die Infektion eine Protozoeninfektion ist, die Infektion verursacht wird durch Malaria (Plasmodium falciparum, Plasmodium vivax, Plasmodium ovale, Plasmodium malariae), Leishmania-Art, einer Trypanosom-Art (afrikanisch und amerikanisch), ein Kryptosporidium, einer Isospora-Art, Naegleria fowleri, einer Acanthamöbe, Balamuthia mandrillaris, Toxoplasma gondii oder Pneumocystis carinii.

12. Pockenvirus zur Verwendung nach Anspruch 1, wobei das Antigen ein tumorassoziiertes Antigen (TAA), ein tumorspezifisches Antigen (TSA) oder ein gewebespezifisches Antigen ist.

13. Pockenvirus zur Verwendung nach Anspruch 1, wobei das Antigen ein virales, ein bakterielles, ein Pilz- oder ein Protozoen-Antigen ist.

14. Pockenvirus zur Verwendung nach Anspruch 1, wobei vor, gleichzeitig mit oder nach dem Antigen ein co-stimulierendes Molekül, ein Wachstumsfaktor, ein Adjuvans und/oder ein Zytokin an der gleichen oder einer anderen Stelle co-verabreicht wird.

15. Pockenvirus zur Verwendung nach Anspruch 14, wobei das co-stimulierende Molekül oder Zytokin durch das Pockenvirus mit dem Antigen co-exprimiert wird.

16. Pockenvirus zur Verwendung nach Anspruch 14 oder 15, wobei das co-exprimierte, co-stimulierende Molekül ausgewählt ist aus der Gruppe bestehend aus: IL-1, IL-2, IL-7, IL-12, IL-15,IL-18, IL-23, IL-27, B7-2, B7-H3, CD40, CD40L, ICOS-Ligand, OX-40L, 4-1 BBL, GM-CSF, SCF, FGF, Flt3-Ligand und CCR4.

17. Pockenvirus zur Verwendung nach Anspruch 14 oder 15, wobei das Pockenvirus B7-1, ICAM-1 und LFA-3 exprimiert.

18. Kit, umfassend eine Vorrichtung zum mechanischen Durchtrennen des Epidermisgewebes eines Subjekts, wie etwa eine Skarifikationsnadel oder einen Abschleifer, sowie das Pockenvirus zur Verwendung nach einem der Ansprüche 1-17.

## Revendications

1. Poxvirus vivant, modifié et non répliquant comprenant un acide nucléique codant pour un antigène peptidique ou polypeptidique étranger au poxvirus, dans lequel le poxvirus infecte les cellules humaines mais est non répliquant dans les cellules humaines ; pour une utilisation dans un procédé consistant à provoquer une réponse immunitaire des lymphocytes T mémoire dans l'antigène étranger, en administrant le poxvirus à un épiderme mécaniquement perturbé d'un patient de sorte que le poxvirus exprimant l'antigène étranger infecte l'épiderme dissocié et provoque une réponse immunitaire des lymphocytes T mémoire.

2. Poxvirus selon la revendication 1, lequel poxvirus est choisi dans le groupe constitué par : l'orthopoxvirus, le suipoxvirus, le capripoxvirus, le leporipoxvirus, le parapoxvirus, le molluscipoxvirus et l'yatapoxvirus.

3. Poxvirus selon la revendication 2, dans lequel l'orthopoxvirus est un virus de la vaccine.

4. Poxvirus selon la revendication 3, dans lequel le virus de la vaccine est le virus de la vaccine Ankara (MVA) modifié.

5. Poxvirus pour une utilisation selon la revendication 1, dans lequel l'épiderme est mécaniquement perturbé par une aiguille à scarification, une aiguille hypodermique ou un polissoir.

6. Poxvirus pour une utilisation selon la revendication 1, dans lequel l'épiderme est mécaniquement perturbé en même temps ou avant l'administration du poxvirus.

7. Poxvirus pour une utilisation selon la revendication 1, dans lequel le patient a ou présente un risque de développer un cancer.

8. Poxvirus pour une utilisation selon la revendication 7, dans lequel le cancer est le cancer de la peau, du sein, de la prostate, du poumon, du cerveau, du poumon, de l'ovaire, du foie, du pancréas, de l'estomac, du rein, de la vessie et de la thyroïde, ou le cancer colorectal.

9. Poxvirus pour une utilisation selon la revendication 7, dans lequel le cancer est choisi dans le groupe constitué par le mélanome, le carcinome épidermoïde cutané, le carcinome basocellulaire, le carcinome à cellules de Merkel, le carcinome annexiel, le lymphome cutané T ou B, les sarcomes, l'adénocarcinome, l'adénocarcinome de la prostate, la néoplasie intraépithéliale prostatique, le carcinome épidermoïde du poumon, l'adénocarcinome pulmonaire, le carcinome pulmonaire à petites cellules, le cancer de l'ovaire d'origine épithéliale, l'adénocarcinome et le léiomyosarcome colorectal, l'adénocarcinome et le léiomyosarcome de l'estomac, le carcinome hépatocellulaire, le cholangiocarcinome, les adénocarcinomes canalaires du pancréas, les tumeurs endocrines du pancréas, le carcinome à cellules rénales, le carcinome à cellules transitionnelles du rein et de la vessie et le carcinome épidermoïde de la vessie.

10. Poxvirus pour une utilisation selon la revendication 1, dans lequel le patient a ou présente un risque de développer une infection virale, bactérienne, fongique ou protozoaire.

11. Poxvirus pour une utilisation selon la revendication 10, dans lequel, si l'infection est une infection virale, l'infection est causée par : le VIH, le virus de la grippe, le virus de la dengue, le virus de l'hépatite A, le virus de l'hépatite B, le virus de l'hépatite C, le papillomavirus humain, le virus Ebola, le virus de Marburg, le virus rabique, le virus de Hanta, le virus de West Nile, les coronavirus de type SARS, le virus de l'herpès simplex, le virus de la varicelle et du zona, le virus d'Epstein-Barr, l'herpès-virus humain type 8, les alphavirus ou l'encéphalite de Saint-Louis ;
dans lequel, si l'infection est une infection bactérienne, l'infection est causée par *Mycobacterium tuberculosis, Salmonella typhi, Bacillus anthracis, Yersinia pestis, Francisella tularensis, Legionella, Chlamydia, Rickettsia typhi* ou *Treponema pallidum ;*
dans lequel, si l'infection est une infection fongique, l'infection est causée par *Coccidioides immitis, Blastomyces dermatitidis, Cryptococcus neoformans, Candida albicans* ou une espèce *d'Aspergillus ;*
dans lequel, si l'infection est une infection protozoaire, l'infection est causée par la malaria (*Plasmodium falciparum, Plasmodium vivax, Plasmodium ovale, Plasmodium malariae*), une espèce de *Leishmania,* une espèce de *Trypanosoma* (africaine et américaine), un cryptosporidium, une espèce d'*Isospora, Naegleria fowleri,* une espèce *d'Acanthamoeba, Balamuthia mandrillaris, Toxoplasma gondii*, ou *Pneumocystis carinii.*

12. Poxvirus pour une utilisation selon la revendication 1, dans lequel l'antigène est un antigène associé aux tumeurs (TAA), un antigène spécifique des tumeurs (TSA) ou un antigène spécifique des tissus.

13. Poxvirus pour une utilisation selon la revendication 1, dans lequel l'antigène est un antigène viral, bactérien, fongique ou protozoaire.

14. Poxvirus pour une utilisation selon la revendication 1, dans lequel une molécule de costimulation, un facteur de croissance, un adjuvant et/ou une cytokine, est administré(e) avant, en même temps ou après l'antigène, et sur un site identique ou différent.

15. Poxvirus pour une utilisation selon la revendication 14, dans lequel la molécule de costimulation ou la cytokine est coexprimée avec l'antigène par le poxvirus.

16. Poxvirus pour une utilisation selon la revendication 14 ou 15, dans lequel la molécule de costimulation coexprimée est choisie dans le groupe constitué par : IL-1, IL-2, IL-7, IL-12, IL-15, IL-18, IL-23, IL-27, B7-2, B7-H3, CD40, CD40L, le ligand ICOS, OX-40L, 4-1 BBL, GM-CSF, SCF, FGF, le ligand Flt3 et CCR4.

17. Poxvirus pour une utilisation selon la revendication 14 ou 15, lequel poxvirus exprime B7-1, ICAM-1 et LFA-3.

18. Kit comprenant un dispositif de perturbation mécanique du tissu épidermique d'un patient, notamment une aiguille à scarification ou un polissoir, et le poxvirus pour une utilisation selon l'une quelconque des revendications 1 à 17.
